# EUROPEAN PATENT SPECIFICATION

(11) **EP 1 272 517 B1**
(45) Date of publication and mention of the grant of the patent: **15.10.2008**
(21) Application number: 01917331.9
(22) Date of filing: 06.04.2001
(51) Int. Cl.: C07K 14/705, C12N 15/12, G01N 33/68, A61K 38/17, A61P 25/00, C12Q 1/68

(54) **MULTIPROTEIN-COMPLEXES COMPRISING A NMDA RECEPTOR AND USES THEREOF**
EINEN NMDA REZEPTOR ENTHALTENDE MULTIPROTEIN-KOMPLEXE UND DEREN VERWENDUNG
MATIERE BIOLOGIQUE ET UTILISATIONS DE CELLE-CI

(30) Priority: 06.04.2000 GB 0008321
(43) Date of publication of application: 08.01.2003
(73) Proprietor: The University Court of the University of Edinburgh incorporated under The Universities ( Scotland) Acts, Edinburgh EH8 9YL (GB)
(72) Inventor: GRANT, Seth Garran Niels, Dept. of Neuroscience, Edinburgh EH8 9JZ (GB); HUSI, Holger, Dept. of Neuroscience, Edinburgh EH8 9JZ (GB)
(74) Representative: Miles, John Stephen
(86) International application number: PCT/GB2001/001570
(87) International publication number: WO 2001/077170

(56) References cited:
- WO-A-97/33173
- WO-A-97/46877
- ZHENG X ET AL.: "Protein kinase C potentiation of N-methyl-D-aspartate receptor activity is not mediated by phosphorylation of N-methyl-D-aspartate receptor subunits" PROCEEDINGS OF THE NATIONAL ACADEMY OF SCIENCE USA, vol. 96, no. 26, 21 December 1999 (1999-12-21), pages 15262-15267, XP002181985
- BI R ET AL.: "The tyrosine kinase and mitogen-activated protein kinase pathways mediate multiple effects of estrogen in hippocampus" PROCEEDINGS OF THE NATIONAL ACADEMY OF SCIENCE USA, vol. 97, no. 7, 28 March 2000 (2000-03-28), XP002181986
- CAMMAROTA M ET AL.: "Learning-associated activation of nuclear MAPK,CREB and Elk-1, along with Fos production, in the rat hippocampus after a one-trial avoidance learning: abolition by NMDA receptor blockade" MOLECULAR BRAIN RESEARCH, vol. 76, 10 March 2000 (2000-03-10), pages 36-46, XP001031139
- KIM JH ET AL.: "SynGap: a Synaptic RasGAP that Associates with the PSD-95/SAP90 Protein Family" NEURON, vol. 20, April 1998 (1998-04), pages 683-691, XP002181987
- LOTTSPEICH F & ZORBAS H: "Bioanalytik" 1998 , SPEKTRUM AKADEMISCHER VERLAG , HEIDELBERG, BERLIN XP002181988 chapter 9.8: Affinitätschromatographie; page 209 -page 211
- VAN ROSSUM D. ET AL: 'Dynamic Interaction Between Soluble Tubulin and C-Terminal Domains of N-Methyl-D-Aspartate Receptor Subunits' JOURNAL OF NEUROCHEMISTRY vol. 72, 1999, pages 962 - 973
- HUSI H. ET AL: 'Proteomic analysis of NMDA receptor-adhesion protein signaling complexes' NATURE NEUROSCIENCE vol. 3, no. 7, July 2000, pages 661 - 669

## Description

The present invention relates to multi-protein complexes, in particular complexes comprising an NMDA receptor, and uses of the same. The invention further relates to methods of identifying compounds for treating disorders and conditions associated with dysfunction of NMDA receptors (and complexes comprising such receptors) in the central nervous system, and to the use of such compounds.

The amino acid L-glutamate is a major excitatory neurotransmitter, with glutamatergic neurones projecting throughout the mammalian central nervous system. Anatomical, biochemical and electrophysiological analyses suggest that glutamatergic systems are involved in a broad array of neuronal processes, including synaptic plasticity (i.e. the reorganisation of synapses and *de novo* synaptic outgrowth), regulation of neurotransmitter release, long term potentiation (LTP), learning and memory, hypoxic/ischaemic neuronal damage and cell death (Bliss and Collingridge, 1993, Nature 261:31-39; Bear and Malenka, 1994, Curr. Opin. Neurobiol. 4:389-399). In addition, glutamate has been implicated in the pathogenesis of several psychiatric and neurological disorders including schizophrenia (Carlsson et al, 1999, Br. J. Psychiatry Supple. 37:2-6; Tamminga, 1999, Br. J. Psychiatry Supple. 37:12-15), severe depressive illness (Skolnick, 1999, Eur. J. Pharmacol. 375:31-40), epilepsy (Chapman, 1998, Prog. Brain Res. 116:371-383), Parkinson's disease (Loopuijt and Schmidt, 1998, Amino Acids 14:17-23), hypoxic brain injury (Mishra and Delivoria-Papadopoulos, 1999, Brain Res. Bull. 48:233-238) learning impairment (Tang et al, 1999, Nature 40:63-69) and stroke (Sun and Chen, 1999, Clin. Neuropharmacol. 22:164-171).

Glutamate mediates its neurotransmitter functions through receptors that are categorised into two main groups; ionotropic and metabotropic receptors (for review see Hollman and Heinemann, 1994, Ann. Rev. Neurosci. 17:31-108)). Ionotropic glutamate receptors contain integral cation-specific, ligand-gated ion channels, whereas metabotropic glutamate receptors are G-protein-coupled receptors that transduce extracellular signals via activation of intracellular second messenger systems.

The ionotropic glutamate receptors can be further subdivided on the basis of their ligand preference into kainate, α-amino-3-hydroxy-5-methyl-4-isoxazole-propionate (AMPA) and N-methyl-D-aspartate (NMDA) receptors. Of these, the NMDA receptor is arguably the most extensively characterised. NMDA receptors are heteromeric proteins requiring the co-agonists L-glutamate and glycine for activation, and are subject to complex allosteric regulation by polyamines, Mg²⁺, Zn²⁺, pH and redox potential. Six distinct subunits combine to form the ion channel of the NMDA receptor, namely NR1, NR2A to NR2D and NR3 (each of which is encoded by a different gene). Of these, the NR1 subunit is believed to be critical since it is the only one which is able to form a functional ion channel on its own (see Schoepfer et al, 1994, Prog. Neurobiol. 42:353-357; Sucher et al, 1996, Trends Pharmacol. Sci. 17:348-355). The remaining subunits (i.e. NR2A-D and NR3) constitute accessory subunits which modify the kinetic and pharmacological characteristics of the resulting NMDA receptor. However, the exact stoichiometry of native NMDA receptors remains to be elucidated.

Advances in the understanding of NMDA receptor composition at the genetic level, and in particular the cloning of genes encoding the various NMDA receptor subunits, have enabled the production and study of recombinant NMDA receptors. Although such studies have furthered understanding of the function and regulation of NMDA receptors, it has been shown that the pharmacological properties of recombinant receptors differ from those of native receptors expressed *in vivo* (for example, see Sucher et al, 1996, Trends Pharmacol. Sci. 17:348-355). Such disparate effects we believe result, at least in part, from the association of native NMDA receptors with a multitude of other molecules, many of which were hitherto not recognized to be associated with the NMDA receptor, thereby forming an elaborate multi-component complex responsible for mediating the various recognised functions of NMDA receptors. The isolation and partial purification of a complex which included the NMDA receptor and certain other components was described in WO 97/46877.

Kim et al. (1998) Neuron 20:683-691 discloses the isolation of a Ras-GTPase activating protein, SynGAP, which interacts with the PDZ domains of PSD-95 and SAP 102 *in vitro* and *in vivo*.

WO 97/33173 relates to neuronal nitric oxide synthase inhibitors and the use thereof in methods of treating muscular dystrophy, stroke and other neurodegenerative disorders.

Van Rossum et al. (1999) J. Neurochem. 72:962-973 discloses a study of the interactions between tubulin and the C-terminal domains of NMDA receptor subunits NR1 and NR2B.

We now describe the purification of a large multi-protein complex (which we have termed a "Hebbosome") comprising an NMDA receptor that more closely resembles such receptors as they exist *in vivo*. This enables the development of new methods of identifying candidate drugs which interfere with NMDA receptor function and which, consequently, may have utility in the treatment of the various psychiatric and neurological disorders and conditions associated with NMDA receptor dysfunction. For example, it may be possible to identify compounds that interfere with the assembly and composition of the multi-protein complex in such a way that signalling via the complex is inhibited or enhanced.

An object of the present invention is therefore to provide a multi-protein complex comprising not only the core subunits of functional NMDA receptors but also the multitude of accessory proteins which associate with such receptors *in vivo*, and which are important in mediating at least some of the disparate effects seen *in vivo*.

A first aspect of the present invention provides a multi-protein complex comprising the polypeptide subunits NR1, NR28, PSD-95 and at least three components selected from the group of components listed in Table 1.

Preferably, the complex comprises an NMDA receptor, *i*.*e*. a functional NMDA receptor ion channel.

Thus, the invention provides an NMDA receptor complex comprising the polypeptide subunit NR1, NR28, PSD-95 and at least three components selected from the group of components listed in Table 1.

Preferably, the complex has a molecular weight greater than 1000 kDa, for example greater than 1100, 1200, 1300, 1400, 1500, 1600, 1700, 1800, 1900, 2000, 2500, 3000 or 4000 kDa. More preferably, the complex has a molecular weight between 1800 and 2200 kDa. Most preferably, the complex has a molecular weight of about 2000 kDa.

Table 1 provides a list of components of a multi-protein complex which, as far as the inventors are aware, have not previously been indicated as being physically linked, directly or indirectly, to the NR1 subunit and thus have not previously been indicated as being associated with the NMDA receptor. Estimated molecular weights (obtained using SDS-PAGE) are included for the murine homologues of each of the listed components.

**TABLE 1**

| **Protein** | **Mol. Weight** | **Accession Number (SwissProt)** | |
|---|---|---|---|
| | **(kDa)** | **Mouse** | **Human** |
| ***Glutamate receptors*** | | | |
| mGluR1α¹ | 200 | P97772 | Q13255 |
| | | | |

| ***Scaffolding and adaptors*** | | | |
|---|---|---|---|
| NSF² | 83 | P46460 | P46459 |
| Homer/Vesl-1³ | 28/45 | Q9Z216 | O95349 |
| Shank⁴ | 200 | Q9WUE8(rat) | AAF02496-8 |
| AKAP-150/79⁵ | 150 | P24587(rat) | P24588 |
| SAP97⁶ | 100 | Q62696(rat) | Q12959 |
| | | | |

| ***PKA*** | | | |
|---|---|---|---|
| PKA-R2beta(β)⁷ | 53 | P31324 | P31323 |
| | | | |

| ***PKC*** | | | |
|---|---|---|---|
| PKC-beta(β)⁸ | 80 | P04411 | P05771/P05127 |
| PKC-gamma(γ)⁹ | 80 | P05697 | P05129 |
| PKC-epsilon(ε)¹⁰ | 90 | P16054 | Q02156 |
| | | | |

| ***Phosphatases*** | | | |
|---|---|---|---|
| PP2A¹¹ | 36 | P13353 | P05323/P11082 |
| PP5¹² | 50 | Q60676 | P53041 |
| PTP1D¹³ | 72 | --- | Q06124 |
| PP1¹⁴ | 36 | P37139 | P08129 |
| | | | P37140 |
| | | | P36873 |
| PP2B¹⁵ | 61 | P20652 | Q08209 |
| | | | |

| ***Tyrosine kinases*** | | | |
|---|---|---|---|
| PYK2¹⁶ | 116 | --- | Q14289 |
| | | | |

| ***MAP kinase pathway*** | | | |
|---|---|---|---|
| ERK1¹⁷ | 42/44 | Q62844 | P27361 |
| ERK2¹⁸ | 42 | P27703 | P28482 |
| MEK1¹⁹ | 45 | P31938 | Q02750 |
| MEK2²⁰ | 46 | Q63932 | P36507 |
| MKP2²¹ | 43 | --- | Q13524 |
| Rsk-2²² | 90 | P18654 | P51812 |
| c-Raf1²³ | 74 | --- | P04049 |
| | | | |

| ***Small G-proteins and modulators*** | | | |
|---|---|---|---|
| Rac1²⁴ | 21 | Q05144 | P15154 |
| Rap2²⁵ | 21 | --- | P10114/P17964 |
| Ras²⁶ | 21 | Q61411 | P01112 |
| | | P32883 | P01116 |
| | | P08643 | P01118 |
| | | P08556 | |
| NF1GRP²⁷ | 250 | Q04690 | P21359 |
| RalA²⁸ | 24 | P05810 | P11233 |
| | | | |

| ***Other signalling molecules*** | | | |
|---|---|---|---|
| PI3 kinase²⁹ | 85 | P26450 | P27986 |
| | | O08908 | O00459 |
| | | Q64143 | Q92569 |
| PLCγ³⁰ | 150 | Q62077 | P19174 |
| cPLA2³¹ | 110 | P47713 | P47712 |
| arg3.1³² | 55 | Q9WV31 | AAF07185 |
| | | Q63053 (rat) | |
| | | | |

| ***Cell adhesion and cytoskeletal proteins*** | | | |
|---|---|---|---|
| N-Cadherin³³ | 150 | P15116 | T19022/Q14923 |
| β-catenin³⁴ | 92 | Q02248 | P35222 |
| Desmoglein³⁵ | 165 | Q61495 | |
| L1³⁶ | 200 | P11627 | P32004 |
| pp120CAS³⁷ | 120 | P30999 | --- |
| Myosin³⁸ | 205 | P46735 | P35580 |
| Dynamin³⁹ | 96 | Q61358 | Q05193/P50570 |
| Clathrin heavy chain⁴⁰ | 192 | P11442 (rat) | Q00610/P53675 |
| HSP70⁴¹ | 70 | P17879 | P08107 |
| Tubulin⁴² | 50 | --- | P05215 |
| Cortactin⁴³ | 80/85 | Q60598 | Q14247 |
| CortBP-1⁴⁴ | 180/200 | O70470 (rat) | |
| | | O88864 (rat) | |
| | | | |

| ***Other components*** ***(as determined by MS)*** | | | |
|---|---|---|---|
| Bassoon⁴⁵ | 418 | O88778 | 043161 |
| | | O88778 (rat) | |
| Myosin B heavy chain | | | |
| (non-muscle type)⁴⁶ | 229 | P46735 | P35580 |
| P53 binding protein 1⁴⁷ | 214 | P70399 | Q12888 |
| Tight junction | | | |
| protein ZO-1⁴⁸ | 195 | P39447 | Q07157 |
| Est525.7 hypothetical | | | |
| 97.8 kDa protein⁴⁹ | 98 | --- | CAB43675 |
| Sarcolemmal | | | |
| associated protein-3⁵⁰ | 74 | Q28623 (rabbit) | --- |
| HSP70-like | | | |
| HS71 protein⁵¹ | 71 | U73744/P08109 | P11142 |
| Kinesin light chain 2⁵² | 67 | O88448 | Q07866/Q12840 |
| a-Internexin⁵³ | 56 | P46660 | Q16352 |
| RNA binding | | | |
| protein FUS/TLS⁵⁴ | 53 | --- | P35637 |
| Phosphofructokinase⁵⁵ | | P12382 | P17858 |
| Est700.75⁵⁶ | | AI428173* | --- |
| Est763.26⁵⁷ | | AI047568* | --- |
| Est536.68⁵⁸ | | AU050964* | --- |
| Est621.15⁵⁹ | | AV153731* | --- |
| Est571.14⁶⁰ | | --- | --- |
| Est762.20⁶¹ | | --- | --- |

| | | | |
|---|---|---|---|
| (* Accession numbers from EMBL database) | | | |

### Key to Table 1

¹ mGluR1α is a splice variant of the R1 subunit of the metabotropic glutamate receptor
² N-ethylmaleimide-sensitive factor (NSF) is a hexameric ATPase essential for eukaryotic vesicle formation
³ Homer (VESL-1) is one of a family of homer-related proteins that interact with metabotropic glutamate receptors
⁴ Shank is member of a family of proteins involved in molecular scaffolds in the post-synaptic density
⁵ AKAP-150/79 is an A-kinase anchor protein implicated in anchoring type II PKA to the post-synaptic density
⁶ SAP97 is a synapse associated protein
⁷ PKA-R2β is one of four types of regulatory subunit that make up cAMP-dependent protein kinase A (PKA)
⁸⁻¹⁰ PKCβ,γ and ε are isozymes of protein kinase C (PKC)
¹¹ PP2A is a ubiquitously expressed serine/threonine phosphatase
¹² PP5 is a low activity serine/threonine phosphatase that can be stimulated by proteolytic cleavage at the N-terminus
¹³ PTP1D is a cytosolic protein tyrosine phosphatase, also known as SHP2
^{14,15} PP1 and PP2B (calcineurin) are cytosolic protein phosphatases involved in signal transduction
¹⁶ PYK2 is a protein tyrosine kinase activated by a variety of G-protein-coupled receptors and by extracellular signals that elevate intracellular Ca²⁺ concentrations
^{17,18} ERK1 and ERK2 are extracellular signal-regulated kinases involved in the mitogen-activated protein (MAP) kinase signalling cascade
^{19,20} MEK1 and MEK2 are MAP kinase kinases
²¹ MKP2 is a MAP kinase phosphatase isoform
²² Ribosomal S6 kinases Rsk is a substrate of ERK
²³ c-Raf1 is a kinase that phosphorylates MEK1 and is stimulated by PKC
²⁴ Rac1 is a member of the Rho family of GTPases, involved in the regulation of the actin cytoskeleton
²⁵ Rap2 is a low molecular weight GTP-binding protein associated with the cytoskeleton
²⁶ Ras is a small GTPase activated by binding of a large variety of ligands to stimulatory cell surface receptors
²⁷ Nuclear factor 1 (NF1GRP) is a nuclear transcription factor
²⁸ RalA is a Ras-related protein
²⁹ Phosphatidylinositol (PI)-3 kinase is a key enzyme within the PI signal transduction pathway
³⁰ Phospholipase C is also a key enzyme within the PI signal transduction pathway, catalysing the formation of IP₃ and DAG from PI-4,5-P₂
³¹ Cytosolic phospholipase A2 is activated by glutamate stimulation
³² Arg3.1 is an immediate early gene product
³³ N-Cadherin is a member of a family of Ca²⁺-dependent intercellular adhesion molecules
³⁴ β-catenin is a cytosolic protein involved in cell-cell interactions and signal transduction
³⁵ Desmoglein is a desmosomal-associated glycoprotein involved in cell-cell adhesion
³⁶ L1 is a neural cell adhesion molecule (N-CAM)
³⁷ pp120CAS is a cell adhesion protein known to interact with components of the cadherin-catenin complex
³⁸ Myosin is a cytoskeletal protein
³⁹ Dynamin is a regulatory protein involved in receptor endocytosis
⁴⁰ Clathrin heavy chain is a subunit of clathrin, involved in vesicle trafficking and internalisation of receptors
⁴¹ HSP70 is a heat shock-induced protein located in the major histocompatibility complex class III
⁴² Tubulin is the predominant component of the microtubules of the cytoskeleton
⁴³ Cortactin is a filamentous actin binding protein and a substrate for Src tyrosine kinases
⁴⁴ Cortactin binding protein (CortBP)-1 is a proline-rich synapse associated protein
⁴⁵ Bassoon is a component of the cytoskeleton at presynaptic neurotransmitter release sites
⁴⁶ Myosin B heavy chain is a sub-unit of myosin (see '32' above)
⁴⁷ P53 binding protein 1 is a regulator of the tumour supressor protein, P53
⁴⁸ ZO-1 is a tight junction protein involved in regulating the response of the tight junction to changes in Ca²⁺, phospholipid and cytoskeleton composition
⁴⁹ Est525.7 is a brain-derived expressed sequence tag of no known function
⁵⁰ Sarcolemmal associated protein-3 is an acidic, amphipathic membrane protein
⁵¹ HS71 is a molecular chaperone protein
⁵² Kinesin light chain 2 is a subunit of the microtubule-based motor protein kinesin
⁵³ a-Internexin a neuronal intermediate filament protein
⁵⁴ FUS/TLS is an RNA binding protein
⁵⁵ Phosphofructokinase is a glycolytic enzyme
⁵⁶⁻⁶¹ Expressed sequence tags having no known functional homologies or function

Table 2 provides a list of components previously determined to be associated with NMDA receptor complexes.

**TABLE 2**

| **Protein** | **Accession Number (SwissProt)** | |
|---|---|---|
| | **Mouse** | **Human** |
| ***Glutamate receptor subunits*** | | |
| NR1¹ | P35438 | Q05586 |
| NR2A² | P35436 | --- |
| NR2B³ | Q01097 | Q13224 |
| NR2C⁴ | Q01098 | Q14957 |
| NR2D⁵ | Q03391 | --- |
| NR3⁶ | Q9Z2H0(rat) | Q12919 |
| GluR6⁷ | P39087 | Q13002 |
| GluR7⁸ | P42264 (rat) | Q13003 |
| | | |

| ***Scaffolding and adaptors*** | | |
|---|---|---|
| PSD-95⁹ | Q62108 | P78352 |
| ChapSyn110/PSD-93¹⁰ | O88523 | Q15700 |
| Sap102¹¹ | P70175 | Q92796 |
| GKAP/SAPAP¹² | --- | P78335 |
| Yotiao¹³ | AAF27283(rat) | O14869 |

| ***PKA*** | | |
|---|---|---|
| PKA catalytic subunit¹⁴ | P05132 | P17612 |
| | | |

| ***CaM kinase*** | | |
|---|---|---|
| CaM kinase IIβ¹⁵ | P28652 | Q13554 |
| | | |

| ***Tyrosine kinases*** | | |
|---|---|---|
| Src¹⁶ | P05480 | P12931 |
| Fyn¹⁷ | P39688 | P06241 |
| | | |

| ***Small G-proteins and modulators*** | | |
|---|---|---|
| SynGAP¹⁸ | O88449 (rat) | 060889 |
| | | |

| ***Other signalling molecules*** | | |
|---|---|---|
| Calmodulin¹⁹ | --- | P02593 |
| NNOS²⁰ | Q9Z0J4 | P29475 |
| | P29476 (rat) | |
| Citron²¹ | P49025 | O14578 |

| ***Cell adhesion and cytoskeletal proteins*** | | |
|---|---|---|
| MAP2B²² | P20357 | P111637 |
| Actin²³ | 089054 | 902570 |
| α-actinin 2²⁴ | Q61063 | P35609 |
| Spectrin²⁵ | P16546 | Q13813 |
| | Q62261 | Q01082 |

### Key to Table 2

¹⁻⁶ NR1, NR2A, NR2B, NR2C, NR2D and NR3 are subunits of the NMDA receptor
^{7,8} GluR6 and 7 are subunits of metabotropic glutamate receptors
⁹⁻¹¹ Post synaptic density (PSD)-95 protein, Chapsyn110 (PSD-93) and Sap 102 are members of the membrane associated guanylate kinase (MAGUK) family of PDZ-domain proteins, and interact with NR2A and NR2B subunits
¹² Guanylate kinase associated protein (GKAP/SAPAP) modulates the ion channel properties of NMDA receptors via PSD-95
¹³ Yotiao is an NR1 binding protein implicated in the cytoskeletal attachment of NMDA receptors
¹⁴ PKA catalytic subunits, in conjunction with regulatory subunits, form active protein kinase A
¹⁵ Calcium/calmodulin-dependent protein kinase (CaM kinase) IIβ is a synapse-associated protein implicated in LTP and memory
^{16,17} Src and Fyn are members of the Src family of tyrosine kinases, which are involved in NMDA receptor-mediated signal transduction
¹⁸ SynGAP is a Ras-GTPase activating protein which interacts with the PDZ domains of PSD-95 and Sap 102
¹⁹ Calmodulin is a Ca²⁺-binding protein involved in the coupling of changes in intracellular Ca²⁺ concentration to many cellular events by binding to and regulating the activity of numerous proteins
²⁰ nNOS is an isoform of nitric oxide synthase
²¹ Citron is an effector of the Rho signalling pathway, and interacts with PSD-95 at glutamatergic synapses
²² Microtubule associated protein (MAP) 2B is an A-kinase anchor protein
²³ Actin is a cytoskeletal protein
²⁴ α-actinin 2 is a component of the dystrophin-glycoprotein complex
²⁵ Spectrin is a cytoskeletal protein

Preferably, the complex comprises four or five or six or more different components selected from the group of components listed in Table 1 and/or Table 2.

The complex comprises the polypeptide subunits NR1, NR2A, NR2B, PSD-95 and at least one component selected from the group of components listed in Table 1.

Most preferably, the complex comprises all of the components listed in Tables 1 and 2.

Thus, in a preferred embodiment, the complex comprises component proteins that can be divided into five categories; neurotransmitter receptor proteins (*e*.*g*. NR1, NR2, mGluR1), cell adhesion proteins (*e*.*g*. N-cadherin, L1), adaptor proteins (*e*.*g*. PSD-95), signalling peptides (*e*.*g*. PKC, PKA, CaM kinase, phosphatases, tyrosine kinases) and cytoskeletal proteins (*e*.*g*. actin). Advantageously, the complex comprises at least one component protein from each of these five categories.

In a preferred embodiment of the first aspect of the invention, the complex is obtainable by peptide affinity chromatographic methods comprising the steps of:
(i) collecting a sample of tissue from the central nervous system of an animal; and
isolating the complex by contacting the sample with a peptide that binds to PSD-95, wherein the peptide that binds to PSD-95 is SIESDV or KRLLDAQLGSFPPWVQQTRV.

The sample of tissue may be taken from any animal which expresses an NMDA receptor in its central nervous system (CNS). Conveniently, the sample of tissue is taken from an animal which expresses a multi-protein complex according to the first aspect of the invention.

Preferably, the sample of tissue is taken from a mammal. More preferably the sample of tissue is human in origin.

In an alternative embodiment, the sample of tissue is taken from the sea slug, *Aplysia californica*.

Advantageously, the tissue sample is taken from the brain.

Conveniently, the tissue sample is taken from an anatomically discreet region or subregion within the brain, for example a defined cortical or hippocampal region or subregion.

Suitably, the complex is isolated by peptide affinity chromatography.

Also described herein is a sub-complex of a complex according to the first aspect of the invention comprising two or more polypeptide components selected from the components identified in Tables 1 and 2, wherein at least one of the components is selected from the group listed in Table 1.

Thus, a sub-complex of an NMDA receptor complex according to the first aspect of the invention is described.

The sub-complex may comprise at least one component from Table 1 and at least one component from Table 2.

Conveniently, the sub-complex comprises three or four or five or six or more components.

It will be appreciated that the sub-complexes need not comprise an NMDA receptor.

A third aspect of the invention provides a method of producing a multi-protein complex according to the first aspect of the invention comprising the steps of:
(i) collecting a sample of tissue from the central nervous system of an animal; and
(ii) isolating the complex by contacting the sample with a peptide that binds to PSD-95, wherein the peptide that binds PSD-95 is SIESDV or KRLLDAQRGSFPPWVQRTRV.

Thus, the invention provides a method of producing an NMDA receptor complex comprising the steps of:
(i) collecting a sample of tissue from the central nervous system of an animal; and
(ii) isolating the complex by contacting the sample with a peptide that binds PSD-95 as defined above.

It will be appreciated that the complex comprises the component to which the peptide binds.

The peptide used to isolate the complex binds, via the PDZ domain of PSD-95. Exemplary components comprising a PDZ domain include PSD-95, nNOS, SHANK, SAP-102, Chapsyn-110/PSD-93, Homer/Vesl-1 and Cort-BP1, Z0-1.

By 'PDZ domain' we mean peptide domains (also known as DHR domains or GLGF repeats) comprising approximately 90-residue repeats which are found in a number of proteins. Such domains are implicated in ion-channel, receptor clustering, and the linking of receptors to effector enzymes. PDZ domains are protein-recognition modules; some recognize proteins containing the consensus carboxy-terminal tripeptide motif S/TXV with high specificity. Other PDZ domains form homotypic dimers: the PDZ domain of the neuronal enzyme nitric oxide synthase binds to the PDZ domain of PSD-95, an interaction that has been implicated in its synaptic association (see Morais Cabral et al, 1996, Nature 382(6592):649-52).

In a preferred embodiment of the third aspect of the invention, the complex is isolated using peptide affinity chromatography. In this method, the protein/complex of interest is recovered by exploiting its affinity for of PSD-95. Typically, said peptide is immobilised on a resin, which is then washed and transferred into a chromatography column. As the sample containing the protein/complex of interest is washed through the column, the protein/complex binds to the peptide and thus is isolated. Bound target proteins/complexes may then be washed off the resin, for example by eluting with free peptide, or by changing the composition of the mobile phase (e.g. by changing pH or ionic strength).

Examples of the isolation and purification of proteins from tissue samples by peptide affinity chromatography are described in Murray et al (1998), J. Pept. Res. 52, 375-83 and Necina et al (1998) J. Chromatogr. B Biomed. Sci. Appl. 715, 191-201.

The peptide that binds to PSD-95 comprises an amino acid sequence which is identical to that of a region of the C-terminus of the human NMDA-R2B subunit (see SWISS Prot entry accession number Q13224).

The sequence of the last 100 amino acids of the C-terminus of the human NMDA-R2B subunit is as follows:

NPFIPTFGDDQCLLHGSKSYFFRQPTVAGASKARPDFRALVTNKP
VVSALHGAVPARFQKDICIGNQSNPCVPNNKNPRAFNGSSNGHV
YEKLSSIESDV

Specifically, the peptide is SIESDV.

Alternatively, the peptide comprises an amino acid sequence which is identical to that of a region of the C-terminus of the SynGAP subunit (see Table 2 for sequence accession numbers). This region of SynGAP is believed to bind to the third PDZ domain of PSD-95.

Specifically, the peptide is the C-terminal sequence of the rat SynGAP subunit, namely KRLLDAQRGSFPPWVQQTRV.

Also described herein is an alternative method of producing a multi-protein complex according to the first aspect of the invention or a sub-complex thereof, said method comprising the steps of:
(i) collecting a sample of tissue from the central nervous system of an animal; and
(ii) isolating the complex by contacting the sample with a molecule that binds to a component selected from the components listed in Table 1 under conditions which retain the component in a multi-protein complex.

Thus, a method of producing an NMDA receptor complex may comprise the following steps:
(i) collecting a sample of tissue from the central nervous system of an animal; and
(ii) isolating the complex by contacting the sample with a molecule that binds to a component selected from the components listed in Table 1 under conditions which retain an NMDA receptor.

It will be appreciated that the complex comprises the component to which the molecule binds.

Also described herein is a method of producing a multi-protein complex according to the first aspect of the invention or a sub-complex thereof, said method comprising the steps of:
(i) collecting a sample of tissue from the central nervous system of an animal that expresses a multi-protein complex according to the first aspect of the invention wherein the expression of at least one of the components in Table 1 is modified compared to animals expressing a wildtype multi-protein complex; and
(ii) isolating the complex by contacting the sample with a molecule that binds to a component of the (abnormal) complex.

Thus, a method of making an NMDA receptor complex may comprise the polypeptide subunit NR1 and at least one component selected from the group of components listed in table 1, said method comprising the steps of:
(i) collecting a sample of tissue from the central nervous system of an animal that expresses an NMDA receptor complex wherein the expression of at least one of the components in Table 1 is modified; and
(ii) isolating the complex by contacting the sample with a molecule that binds to a component of the complex.

Preferably, the tissue sample may be taken from the central nervous system of:
(i) a gene 'knockout' animal which does not express one or more of the components in Table 1;
(ii) an animal which expresses a mutant form of one or more of the components in Table 1; or
(iii) an animal that overexpresses one or more of the components in Table 1 (e.g. a transgenic animal).

Thus, an abnormal form of a multi-protein complex includes a complex which, compared to a complex from a normal animal (e.g. a non-human animal which has not been genetically modified), lacks a component, contains a mutant component or contains an excess of a component.

Conveniently, the animal is a transgenic non-human animal, for example a non-human animal that has been genetically modified such that it expresses an mutant form of one or more of the components of the multi-protein complex, or overexpresses one or more of the components of the multi-protein complex. Advantageously, the transgenic animal is a rodent, e.g. a mouse or rat.

The production and use of transgenic animals is now widely known in the art (for example, see reviews by Cameron, 1997, Mol. Biotechnol. 7:253-65 and Campbell et al, 1996, Mol. Psychiatry. 1:105-20). Indeed, several groups have reported the production of transgenic mice expressing abnormal NMDA receptors comprising mutant or overexpressed NR2 subunits (Suchanek et al, 1997, Biol. Chem. 378:929-934; Okabe et al, 1998, J. Neurosci. 18:4177-88; Tang et al, 1999, Nature 401:63-60; Sprengel et al, 1998, Cell 92:279-289).

The molecule that binds to a component of the multi-protein complex may be an antibody or antigen-binding fragment thereof.

It will be appreciated that the molecule that binds to a component of the complex may be a peptide (e.g. for use in peptide affinity chromatography) or may be an antibody or antigen-binding fragment thereof (e.g. for use in immunoaffinity and/or immuno-precipitation techniques).

By "antigen-binding fragment" of antibodies we include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

Exemplary methods by which a multi-protein complex of the invention may be isolated include immunoprecipitation and immuno-affinity chromatography. Such techniques are widely known in the art (for example, see Scopes, 1994, Protein purification: principles and practice, 3rd edition, Springer-Verlag, New York; Harlow and Lane (Eds.) Antibodies: A Laboratory Manual, Cold Spring Harbor Press).

In the methods of the invention for producing a multi-protein complex, the sample of tissue from which the multi-protein complex is isolated may be from any part of the central nervous system, including the brain and spinal cord.

Preferably, the tissue is from the brain.

More preferably, the tissue comprises or is from the forebrain (e.g. from the hippocampus or cortex).

It will be appreciated by those skilled in the art that a multi-protein complex according to the first aspect of the invention or a sub-complex thereof may comprise components derived from any donor animal that expresses an NMDA receptor. Conveniently, the NMDA receptor is expressed as a component of a multi-protein complex.

Preferably the components of the complex or sub-complex are derived from humans.

Persons skilled in the art will further appreciate that the sample of tissue from which the multi-protein complex is derived may be taken from an adult, juvenile or new-born animal.

It will be further appreciated that differences may exist in the composition of multi-protein complexes derived from animals of different ages (for example, see Sans et al, 2000, J. Neuroscience 20:1260-71).

In a preferred embodiment of the invention, the method of producing a multi-protein complex further comprises the step (prior to step 1) of selecting the age of the animal from which the tissue is taken.

Also described herein is a method of making a sub-complex of a multi-protein complex of the first aspect of the invention comprising the steps of:
(i) expressing in a host cell a genetic construct encoding a first component selected from the components identified in Tables 1 and 2;
(ii) expressing in a host cell a genetic construct encoding a second component selected from the components identified in Tables 1 and 2;
(iii) isolating the components from the host cell culture(s); and
(iv) contacting the components with each other,
wherein the first and second components are different and at least one of the components is selected from the components listed in Table 1.

Thus, a method of making a sub-complex of an NMDA receptor may comprise steps (i) to (iv) above.

Such a method may further comprise the step of expressing in a host cell a genetic construct encoding at least one further component selected from the components identified in Tables 1 and 2, prior to the step of contacting the components with each other. In this way, sub-complexes comprising three or four or five or six or more different components may be produced.

It will be appreciated by those skilled in the art that a genetic construct may encode one or more components of the multi-protein complex (i.e. each component of the complex need not necessarily be encoded by a separate genetic construct).

The host cell in step (i) may be the same as the host cell in step (ii), and step (iv) may occur when the at least two components are expressed in the same host cell. Thus, in this instance, step (iv) may precede step (iii).

Methods of expressing genetic constructs in host cells are widely known in the art. Thus, the DNA encoding a component of the multi-protein complex may be used in accordance with known techniques, appropriately modified in view of the teachings contained herein, to construct an expression vector, which is then used to transform an appropriate host cell for the expression and production of the polypeptide of the invention. Such techniques include those disclosed in US Patent Nos. 4,440,859 issued 3 April 1984 to Rutter et al*,* 4,530,901 issued 23 July 1985 to Weissman, 4,582,800 issued 15 April 1986 to Crowl, 4,677,063 issued 30 June 1987 to Mark et al*,* 4,678,751 issued 7 July 1987 to Goeddel, 4,704,362 issued 3 November 1987 to Itakura et al*,* 4,710,463 issued 1 December 1987 to Murray, 4,757,006 issued 12 July 1988 to Toole, Jr. et al*,* 4,766,075 issued 23 August 1988 to Goeddel et al and 4,810,648 issued 7 March 1989 to Stalker, all of which are incorporated herein by reference.

The DNA encoding the component of the multi-protein complex may be joined to a wide variety of other DNA sequences for introduction into an appropriate host. The companion DNA will depend upon the nature of the host, the manner of the introduction of the DNA into the host, and whether episomal maintenance or integration is desired.

Generally, the DNA is inserted into an expression vector, such as a plasmid, in proper orientation and correct reading frame for expression. If necessary, the DNA may be linked to the appropriate transcriptional and translational regulatory control nucleotide sequences recognised by the desired host, although such controls are generally available in the expression vector. The vector is then introduced into the host through standard techniques. Generally, not all of the hosts will be transformed by the vector. Therefore, it will be necessary to select for transformed host cells. One selection technique involves incorporating into the expression vector a DNA sequence, with any necessary control elements, that codes for a selectable trait in the transformed cell, such as antibiotic resistance. Alternatively, the gene for such selectable trait can be on another vector, which is used to co-transform the desired host cell.

Host cells that have been transformed by the recombinant DNA encoding the component are then cultured for a sufficient time and under appropriate conditions known to those skilled in the art in view of the teachings disclosed herein to permit the expression of the polypeptide, which can then be recovered.

Many expression systems are known, including bacteria (for example *E. coli* and *Bacillus subtilis*), yeasts (for example *Saccharomyces cerevisiae*), filamentous fungi (for example *Aspergillus*), plant cells, animal cells and insect cells.

The vectors include a prokaryotic replicon, such as the ColE1 *ori,* for propagation in a prokaryote, even if the vector is to be used for expression in other, non-prokaryotic, cell types. The vectors can also include an appropriate promoter such as a prokaryotic promoter capable of directing the expression (transcription and translation) of the genes in a bacterial host cell, such as *E. coli,* transformed therewith.

A promoter is an expression control element formed by a DNA sequence that permits binding of RNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are pUC18, pUC19, pBR322 and pBR329 available from Biorad Laboratories, (Richmond, CA, USA) and pTrc99A and pKK223-3 available from Pharmacia, Piscataway, NJ, USA.

A typical mammalian cell vector plasmid is pSVL available from Pharmacia, Piscataway, NJ, USA. This vector uses the SV40 late promoter to drive expression of cloned genes, the highest level of expression being found in T antigen-producing cells, such as COS-1 cells.

An example of an inducible mammalian expression vector is pMSG, also available from Pharmacia. This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems, La Jolla, CA 92037, USA. Plasmids pRS403, pRS404, pRS405 and pRS406 are Yeast Integrating plasmids (YIps) and incorporate the yeast selectable markers *HIS3, TRP1, LEU2* and *URA3*. Plasmids pRS413-416 are Yeast Centromere plasmids (YCps)

A variety of methods have been developed to operably link DNA to vectors via complementary cohesive termini. For instance, complementary homopolymer tracts can be added to the DNA segment to be inserted to the vector DNA. The vector and DNA segment are then joined by hydrogen bonding between the complementary homopolymeric tails to form recombinant DNA molecules.

Synthetic linkers containing one or more restriction sites provide an alternative method of joining the DNA segment to vectors. The DNA segment, generated by endonuclease restriction digestion as described earlier, is treated with bacteriophage T4 DNA polymerase or *E. coli* DNA polymerase I, enzymes that remove protruding, 3'-single-stranded termini with their 3'-5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, CN, USA.

A desirable way to modify the DNA encoding the component of the multi-protein complex is to use the polymerase chain reaction as disclosed by Saiki et al (1988) Science 239, 487-491.

In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

The components of the sub-complex may be isolated from the host cell culture by methods known in the art (for example, see Scopes, 1994, Protein purification: principles and practice, 3rd edition, Springer-Verlag, New York). For example, the components of the sub-complex may be isolated from the host cell culture using ion-exchange chromatography, size exclusion chromatography, hydrophobic interaction and affinity methods.

If desired, the sub-complex may be reconstituted in a lipid bilayer using known methods.

A further aspect of the invention provides a host cell for use in a method of the invention containing one or more genetic constructs that encode a component selected from the group of components listed in Table 1.

The invention further provides a host cell comprising one or more genetic constructs that encode four or more components selected from the group of components listed in Tables 1 and 2.

Preferable, the host cells further comprises one or more genetic constructs that encode a component selected from the group of components listed in Table 2.

For example, the host cell may contain genetic constructs encoding six or more components listed in Tables 1 and 2.

Exemplary host cells include cells selected from a group consisting of mammalian cell lines, insect cell lines, yeast and other eukaryotic cell lines; in addition to prokaryotic cell lines (e.g. *E.coli* expression systems).

Preferably, the host cell is a mammalian cell or is derived from such a cell, for example a human cell.

More preferably, the host cell is a neurone or a neurone-derived cell (e.g. primary neuronal cultures, stem cell derived neurones (stem cells being both pluripotent embryonic cells as well as neuronal stem cells - such as those found in neurospheres), and commonly used cell lines such as PC12 cells. However, human embryonic cells are excluded.

A further aspect of the invention provides a sub-cellular fraction isolated from a host cell of the invention, which contains within it a multi-protein complex according to the first aspect of the invention or a sub-complex thereof.

Thus, the invention provides a sub-cellular fraction isolated from a host cell of the invention, which contains within it an NMDA receptor complex or a sub-complex thereof.

Typically, the sub-cellular fraction is or comprises a plasma membrane.

Conveniently, the sub-cellular fraction contains or comprises synaptosomes, presynaptic and/or postsynaptic terminals, or soma.

Alternatively, the sub-cellular fraction contains or comprises a cytosolic fraction.

Plasma membranes may be purified from transformed host cells by methods known in the art.

Also described herein is a method of making a sub-complex of a multi-protein complex comprising the steps of:
(i) contacting a complex according to the first aspect of the invention with an agent that interferes with protein-protein interactions ; and
(ii) isolating the sub-complexes formed thereby.

Thus, a method of making a sub-complex of an NMDA receptor complex may comprise the steps of:
(i) contacting a complex according to the first aspect of the invention with an agent that interferes with protein-protein interactions ; and
(ii) isolating the sub-complexes formed thereby.

In a preferred method, a multi-protein complex is isolated using the methods described above. A sub-complex is formed by adding to said complex a competitor peptide which competes for a defined interaction of two components of the complex, thereby releasing one or more component(s) from the original complex to form one or more subcomplexes.

A further aspect of the invention provides a method of identifying a candidate compound for treating disorders and conditions associated with dysfunction of NMDA receptors in the central nervous system comprising:
(i) selecting a compound to be tested;
(ii) contacting the test compound with a multi-protein complex according to the first aspect of the invention; and
(iii) determining whether the compound to be tested interacts with the complex;
wherein test compounds that interact with the complex or sub-complex are identified as candidate compounds for alleviating the symptoms of, preventing or slowing the onset of, and/or correcting the deficit(s) associated with dysfunction of NMDA receptors in the central nervous system.

Thus, the invention provides a method of identifying a candidate compound for treating disorders and conditions associated with dysfunction of NMDA receptor complexes in the central nervous system, said method comprising steps (i) to (iii) above, wherein test compounds that interact with the complex are identified as candidate compounds for alleviating the symptoms of, preventing or slowing the onset of, and/or correcting the deficit(s) associated with dysfunction of NMDA receptor complexes in the central nervous system..

For example, multi-protein complexes of the present invention can be adapted to render the complexes immobilized to a 96 well plate or other suitable vessel for drug screening. Thus, the peptide affinity or antibody affinity method can be adapted such that the antibody or peptide is linked (directly or indirectly) to the plate; brain extracts or other sources of complexes can then be incubated in the plate resulting in the immobilization of the multi-protein complex. Molecules which are not associated with such complexes are then removed, e.g. by washing. The presence of particular components in the multi-protein complex can be tested for by ELISA techniques (binding of antibody to target and observation of bound antibody using enzyme linked secondary antibody). Alternatively, enzyme assays may be performed using specific substrates (for example, a peptide substrate for PKA could be applied to the multi-protein complex in the 96 well plate with the appropriate reaction components, and the phosphorylation of the peptide would be monitored using standard methods (for example, colorimetric assay of hydrolysed ATP). Dissociation of specific components of the complex resulting from application of a test compound to the immobilised multi-protein complex may be monitored using the same methods.

The above methods exemplify a primary screen for high-throughput analysis. Secondary screening may involve complementary methods such as chromatography, SDS-PAGE methods (*e.g.* western blot detection) and enzyme assays.

Later stages of screening may involve the use of cells (including neurons isolated from transgenic mice) expressing a multi-protein complex or sub-complex of the invention, or components thereof.

By 'disorders and conditions associated with dysfunction of NMDA receptors in the central nervous system' we include disorders and conditions associated with dysfunction of multi-protein complexes comprising NMDA receptors. Exemplary disorders and conditions include learning impairment and numerous psychiatric and neurological disorders with which NMDA receptors have been implicated (such as schizophrenia, depression, stroke, epilepsy and neurodegenerative disorders). We further include abnormalities in learning behaviour, attention and memory acquisition associated with natural variation within a population. We also include abnormal brain function following injury, such as that resulting from direct head trauma, brain surgery, stroke etc.

By 'interacts with' a multi-protein complex or sub-complex thereof we include modulates, either positively or negatively, a property of and/or induces dissociation of the multi-protein complex or sub-complex, or component thereof.

In a preferred embodiment of the above aspect of the invention, the compound to be tested is contacted with the multi-protein complex and the activity of an enzyme component of the multi-protein complex is measured. For example, candidate compounds may be identified by determining the ability of compounds to be tested to modulate, either positively or negatively, the activity of a protein kinase or phosphatase component of a multi-protein complex. Methods for measuring the activity of such enzymes are known in the art (see above).

Alternatively, candidate compounds may be identified by determining the ability of a compound to be tested to induce dissociation of the multi-protein complex. Dissociation of the complex may be assessed using several methods. For example, antibodies to a specific component that is dissociated could be used, or radiolabelled proteins and other methods.

A further aspect of the invention provides a method of identifying a candidate compound for treating disorders and conditions associated with dysfunction of NMDA receptors in the central nervous system comprising:
(i) selecting a compound to be tested;
(ii) contacting the test compound with a component selected from the group listed in Table 1; and
(iii) determining whether the compound to be tested modulates a property of said component;
wherein test compounds that modulate a property of the component are identified as candidate compounds for alleviating the symptoms of, preventing or slowing the onset of, and/or correcting the deficit(s) associated with disorders and conditions associated with dysfunction of NMDA receptors in the central nervous system.

Thus, the invention provides a method of identifying a candidate compound for treating disorders and conditions associated with dysfunction of NMDA receptor complexes in the central nervous system, comprising steps (i) to (iii) above, wherein test compounds that modulate a property of the component are identified as candidate compounds for alleviating the symptoms of, preventing or slowing the onset of, and/or correcting the deficit(s) associated with disorders and conditions associated with dysfunction of NMDA receptor complexes in the central nervous system.

For example, the activity/function of the following components from Table 1 may be assayed using general methods for measuring activity:

| *Component* | *Phosphorylation Assay** | *Activity dependent binding assay** | *Lipid assay***^{†}** |
|---|---|---|---|
| PKA (R2/catalytic subunit) | + | | |
| PKC (beta/gamma/epsilon) | + | | |
| PP2A | + | | |
| PP5 | + | | |
| PTP1D | + | | |
| Pyk2 | + | | |
| ERK (1/2) | + | | |
| MEK (1/2) | + | | |
| MKP2 | + | | |
| RSK2 | + | | |
| PI3K | + | | |
| c-RAF1 | + | | |
| | | | |
| RAC-1 | | + | |
| RAP2 | | + | |
| RAS | | + | |
| NF1GRP | | + | |
| HSP-70 | | + | |
| | | | |
| PLC-gamma | | | + |
| cPLA2 | | | + |

| | | | |
|---|---|---|---|
| * see Gille & Downward (1999) J. Biological Chemistry 274(31):22033-22040 † see Heinz et al (1998) J. Mol. Biol. 275:635-650 and Othman et al. (1996) Biochim. Biophys. Acta. 1303:92-102. | | | |

By 'modulates a property of the component' we include modulates (either positively or negatively) the catalytic activity of enzyme components, and modulates (either positively or negatively) the ability of the component to interact with one or more other components of the multi-protein complex or sub-complex. It will be appreciated that the one or more other components may be any of the components listed in Tables 1 or 2.

In a preferred embodiment of the above aspect of the invention, step (iii) comprises determining whether the test compound modulates (either positively or negatively) the ability of the component to interact with one or more other components of the multi-protein complex or sub-complex.

Advantageously, the method of the invention for identifying a compound for treating disorders and conditions associated with dysfunction of NMDA receptors in the central nervous system further comprises the step of testing the test compound in one or more screens for efficacy in the treatment of learning impairment, psychiatric disorders and/or neurological disorders.

For example, the test compounds may be further assessed using the following types of screening assay:
1. *In vitro analysis of neuronal function using cultured neurones, tissue slices or explants.*
   Compounds may be tested using known methods (for example, electrophysiology, *in situ* hybridisation, HPLC measurement of neurotransmitters) for effects on neuronal membrane properties, gene expression and neurotransmitter release.
   The effects of compounds that modulate the multi-protein complexes of the invention in primary screening assays would be tested on neuronal cells, where the assays reflected biological mechanisms in which the NMDA receptor is involved. There would also be control assays for specificity of compound action through the monitoring of biological mechanisms that are independent of NMDA receptor function. Examples of NMDA receptor function-dependent assays would include: measurement of expression components of multi-protein complexes of the invention; measurement of NMDA receptor channel properties using patch clamping and other electrophysiological methods; measuring the response of cells and multi-protein complex components to application of compounds that bind the NMDA receptor or other neurotransmitter receptors (agonists and antagonists); these responses could include cell death induced by neurotransmitters, structural changes in synapses, axons and dendrites; measure changes in synaptic transmission. Compounds that modulate NMDA receptor dependent biology would then be used for screens in intact animals.
2. *In vivo analysis of neuronal function in animal models*
   Compounds may be tested using known methods (for example, electrophysiology, microdialysis, learning and memory in mazes) for effects on neuronal membrane properties, neurotransmitter release and behaviour. For example, the effect of candidate compounds on cognitive function could be tested in rodents and primates. In such tests, attention would be focussed on phenotypes known to involve the NMDA receptor and other components in the multi-protein complex. For example, learning tests, response to chronic pain, and drug addiction tests could be employed.
*3. Studies using mutant*/*transgenic rodents*
   Screens in animals could include screens on mutant mice. For example, if a drug was known to regulate a protein bound to PSD-95, this drug would effect PSD-95 mutant mice. Where the drug specifically bound a single protein, a valuable control for drug specificity *in vivo* would be to examine the effect the drug had on mice carrying the gene mutation for that protein. The assays would be identical to those described above, except that mutant mice would provide the source of cells. Specifically, in the example of a PSD-95 mutant mouse, we would test if compounds that interfere with PSD-95 binding proteins result in changes in physiology.
*4. In vivo analysis of recovery in animals with damage to the CNS*
   Screens could also be performed in animals with impairments or damage to the nervous system (e.g. from strokes, drug, brain tumours or surgical damage). In this type of screen, the untreated animal is being monitored for recovery of brain function (a form of plasticity of learning) and the test compounds would be assessed for their ability to enhance recovery or learning.

A further aspect of the invention provides a method of identifying a candidate compound for treating disorders and conditions associated with dysfunction of NMDA receptors in the central nervous system comprising:
(i) selecting a compound known to modulate the property of a component listed in Table 1; and
(ii) testing said compound in a screen for efficacy in the treatment of disorders and conditions associated with dysfunction of NMDA receptors in the central nervous system.

Thus, the invention provides a method of identifying a candidate compound for treating disorders and conditions associated with dysfunction of NMDA receptor complexes in the central nervous system comprising steps (i) and (ii) above.

Exemplary compounds known to modulate the property of a component listed in Table 1 are listed below:

| ***Component*** | ***Antagonist*** | ***Agonist*** |
|---|---|---|
| mGluR1 | LY367385 | L-quisqualic acid |
| PKCβ | C1 | Phorbol 12-myristate 13-acetate |
| PKCγ | C1 | Phorbol 12-myristate 13-acetate |
| PKCε | C1 | Phorbol 12-myristate 13-acetate |
| PP2A | Ocadeic acid | Ceramide C2 |
| PP5 | | Ceramide C2 |
| PP1 | Ocadeic acid | Ceramide C2 |
| PP2B | CsA | Ceramide C2 |
| Erk1 | Lavostatin | |
| Erk2 | Lavostatin | |
| Mek1 | UO126 | |
| Mek2 | UO126 | |
| PI3 kinase | Wortmannin | |
| PLCγ | U-731122 | |
| CPLA2 | OBAA | |
| Tubulin | Vinblastine | Taxol |

Another aspect of the invention provides the use of the above methods for identifying a compound that interacts with a multi-protein complex of the invention for selecting agents that may be useful in the treatment of disorders and conditions associated with dysfunction of NMDA receptors in the central nervous system.

Thus, the invention provides the use of the above methods for identifying a compound that interacts with an NMDA receptor complex of the invention for selecting agents that may be useful in the treatment of disorders and conditions associated with dysfunction of NMDA receptor complexes in the central nervous system.

It will be appreciated by persons skilled in the art that in the above-described methods of the invention for identifying a candidate compound for treating disorders and conditions associated with dysfunction of NMDA receptors in the central nervous system, the multi-protein complexes, sub-complexes thereof and components thereof used in said methods may be derived from animal tissue samples or may be expressed in host cells using recombinant techniques. Additionally, the multi-protein complexes, sub-complexes thereof and components thereof may be in an isolated form (i.e. separate from the tissue samples or host cells), or in a reconstituted form, for example in a lipid bilayer.

The invention additionally provides the use of a host cell according to the invention or a subcellular fraction according to the invention in a screening assay for identifying a candidate compound for treating disorders and conditions associated with dysfunction of NMDA receptors in the central nervous system.

Thus, a host cell according to the invention or a subcellular fraction according to the invention may be used in a screening assay for identifying a candidate compound for treating disorders and conditions associated with dysfunction of NMDA receptor complexes in the central nervous system.

Also described herein is the use of a mutant or transgenic animal as used in the method according to the invention in a screening assay for identifying a candidate compound for treating disorders and conditions associated with dysfunction of NMDA receptors in the central nervous system.

Thus, a mutant or transgenic animal may be used in a screening assay for identifying a candidate compound for treating disorders and conditions associated with dysfunction of NMDA receptor complexes in the central nervous system.

A transgenic mouse with a mutation in or deletion of a component of a multi-protein complex of the invention may have a phenotype which suggests that a drug that interfered with the biological function of the normal complex in a similar way to the mutation would be medically useful. Indeed, PSD-95 mutant mouse exhibit enhanced synaptic plasticity and drugs that produced this effect could be beneficial (See Example 4). In this example, a multi protein complex comprising an NMDA receptor was isolated from the PSD-95 mutant mice using the methods disclosed herein and the composition studied. Unexpectedly a particular component (Arg3.1/Arc) was found to be absent from the multi-protein complex, whereas most other components were still present. Thus, it would be useful to identify drugs that interfere with the Arg3.1/PSD-95 interactions. In this way, the knowledge of the composition can be exploited to identify candidate compound for treating disorders and conditions associated with dysfunction of NMDA receptors in the central nervous system.

As a separate step, the isolated multi-protein complex could be used to screen for drugs that compete for the Arg3.1 interaction in a least two ways:
(i) a multi-protein complex from normal mice could be isolated (as described herein) and incubated with the candidate drug, followed by measurement of Arg3.1 association;
(ii) a multi-protein receptor complex from PSD-95 mutant mice could also be tested for binding of this drug to ascertain if there is an altered binding.

Finally, the selected drug could be tested on PSD-95 mutant mice to see if it had any 'additional' or 'side' effects that are mediated by molecular events outside of the multi-protein complex.

It will be appreciated by persons skilled in the art that such a compound obtainable or obtained by the methods of the invention for identifying a compound may be a drug-like compound or lead compound for the development of a drug-like compound.

The term "drug-like compound" is well known to those skilled in the art, and includes a compound having characteristics that may make it suitable for use in medicine, for example as the active ingredient in a medicament.

Thus, for example, a drug-like compound may be a molecule that may be synthesised by the techniques of organic chemistry, molecular biology or biochemistry, and is preferably a small molecule, which may be of less than 5000 daltons and which may be water-soluble. A drug-like compound may additionally exhibit features of selective interaction with a particular protein or proteins and be bioavailable and/or able to penetrate target cellular membranes, but it will be appreciated that these features are not essential.

The term "lead compound" is similarly well known to those skilled in the art, and may include a compound which, whilst not itself suitable for use as a drug (for example because it is only weakly potent against its intended target, non-selective in its action, unstable, poorly soluble, difficult to synthesise or has poor bioavailability), may provide a starting-point for the design of other compounds that may have more desirable characteristics.

It will be understood that it will be desirable to identify compounds that may modulate the activity of an NMDA receptors (and multi-protein complexes of the invention comprising such receptors) *in vivo.* Thus, it will be understood that reagents and conditions used in the above methods of identifying candidate compounds may be chosen such that the interactions between the compound and the multi-protein complex (or sub-complex or component thereof) are substantially the same as *in vivo.*

Also described herein is a pharmaceutical composition comprising a compound of the invention or a salt thereof and a pharmaceutically acceptable excipient or carrier.

The pharmaceutical compositions may conveniently be presented in unit dosage form and may be prepared by any of the methods well known in the art of pharmacy. Such methods include the step of bringing into association the active ingredient (compound of the invention) with the carrier which constitutes one or more accessory ingredients. In general the pharmaceutical compositions/formulations are prepared by uniformly and intimately bringing into association the active ingredient with liquid carriers or finely divided solid carriers or both, and then, if necessary, shaping the product.

The pharmaceutical compositions may be delivered via administration routes known in the art, for example via oral or parenteral administration.

Formulations in accordance with the present invention suitable for oral administration may be presented as discrete units such as capsules, cachets or tablets; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycolate, cross-linked povidone, cross-linked sodium carboxymethyl cellulose), surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethylcellulose in varying proportions to provide desired release profile.

Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose or an appropriate fraction thereof, of an active ingredient.

The compounds described herein may be used in the therapeutic and/or prophylactic treatment of a number of disorders with which the NMDA receptor has been implicated.

Preferably, the compounds are used for treating disorders and conditions associated with dysfunction of NMDA receptors in the central nervous system.

More preferably, the compound is used in the treatment of learning impairment, psychiatric disorders and/or neurological disorders. Thus, the present invention provides a method of treating a patient with learning impairment, psychiatric disorders and/or neurological disorders comprising administering to said patient a compound of the invention.

By "learning impairment" we include disorders or conditions in which there is an abnormality in the acquisition or recall of information. The simplest case is in children who may fail to learn at the rate of normal children. Learning impairments may be specific to certain modalities, for example learning of language as distinct from learning mathematical skills. Impairments in learning to read can be described as a subset of dyslexia. Learning impairments may also comprise 'impairments in learning after brain damage'. For example, a patient with a stroke, where there has been clear destruction of brain tissue, will often show a profound impediment that over time diminishes. This recovery is because other parts of the brain 'learn' to take over the function. However, such recovery may be poor in certain people and could be considered as impaired learning. Thus, compounds of the invention may have utility in the treatment of patients with damage to their central nervous system (e.g. brain/spinal column) as a result of surgical intervention, stroke or other trauma to the CNS.

Learning impairment may also be associated with an ongoing disease process. For example, degeneration in the brain (e.g. in Alzheimer's disease) is balanced against the 'learning' of remaining brain regions as described above. Thus patients with impaired learning may show more aggressive deterioration in neurodegenerative diseases.

By "psychiatric disorders" we include schizophrenia, Alzheimer's disease, chronic pain syndromes, epilepsy, Parkinson's disease, Huntington's disease, addiction disorders (including alcoholism), major depressive disorder, anxiety disorders.

By "neurological disorders" we include ischaemic brain injury (e.g. stroke), chronic pain syndromes, epilepsy, neurodegenerative disorders (CJD), damage from brain tumour, and lesions having an immunological origin (e.g. in AIDS-related illnesses).

It will be appreciated that the patient may be from any species of animal. Preferably the patient is a mammal, such as humans, dogs, cats, horses, cattle, etc.

More preferably, the patient is human.

It will be further appreciated that the compound may be administered to the patient either as a single treatment (i.e. acutely) or as repeated treatments over a prolonged period (i.e. chronically).

Also described herein is the use of a compound as described above in the preparation of a medicament for the treatment of disorders and conditions associated with dysfunction of NMDA receptors in the central nervous system.

Additionally, the invention provides use of a compound that modulates a property (e.g. the catalytic activity) of one or more of the components listed in Table 1 in the preparation of a medicament for the treatment of disorders and conditions associated with dysfunction of NMDA receptors in the central nervous system. Thus, there is provided a method of treating disorders and conditions associated with dysfunction of NMDA receptors in the central nervous system comprising administering to a patient a compound that modulates a property of one or more of the components listed in Table 1.

Thus, the invention provides the use of a compound that modulates a property (e.g. the catalytic activity) of one or more of the components listed in Table 1 in the preparation of a medicament for the treatment of disorders and conditions associated with dysfunction of NMDA receptor complexes in the central nervous system, and a method of treating disorders and conditions associated with dysfunction of NMDA receptor complexes in the central nervous system comprising administering to a patient a compound that modulates a property of one or more of the components listed in Table 1.

Examples of compounds that modulate a property of one or more of the components listed in Table 1 are listed above.

Preferably, the medicament is for treating learning impairment.

Conveniently, the medicament is for treating psychiatric disorders. For example, compounds of the invention may be used to treat schizophrenia.

Alternatively, the medicament is for treating neurological disorders, such as stroke, epilepsy and neurodegenerative disorders.

The inventors believe that there is an association between variants of the components of multi-protein complexes comprising NMDA receptors (and hence of the nucleic acids encoding said components) and the susceptibility of a subject to learning impairment, psychiatric disorders and/or neurological disorders. Thus, detection of a particular sequence variant of a nucleic acid encoding a component of the multi-protein complex (e.g. a polymorphism in the gene carried by a subject) may be useful for diagnostic and/or prognostic purposes.

By 'sequence variant' we include one of a set of alternative forms (e.g. alleles) of a nucleic acid, such as a gene, which encodes a component of a multi-protein complex of the invention.

By 'associated with' we mean that a subject who expresses the variant of the component in question (i.e. carries the allele of the nucleic acid encoding said variant) has an increased likelihood of developing or suffering from a disorder or condition associated with dysfunction of NMDA receptors in the CNS (e.g. learning impairment, psychiatric disorders and/or neurological disorders) compared to the average likelihood for the general population.

Particular sequence variants of a nucleic acid encoding a component of a multi-protein complex comprising an NMDA receptor may be associated with particular disorders and conditions in which the function of NMDA receptors is abnormal. It will be appreciated that such disorders and conditions may be associated with particular sequence variants of more than one nucleic acid encoding a component of a multi-protein complex comprising an NMDA receptor (e.g. the disorder may be associated with multiple polymorphisms in more than one such gene).

A further aspect of the invention provides a method of diagnosing or aiding diagnosis of disorders and conditions associated with dysfunction of NMDA receptors in the central nervous system comprising the steps of:
(i) providing a sample containing nucleic acid from the subject to be diagnosed; and
(ii) contacting said nucleic acid from the subject with a second nucleic acid which hybridises selectively to a nucleic acid encoding a component selected from the group of components listed in Table 1, or a mutant allele thereof, or their complement.

Thus, the invention provides a method of diagnosing or aiding diagnosis of disorders and conditions associated with dysfunction of NMDA receptor complexes in the central nervous system comprising step (i) and (ii) above.

Preferably, the nucleic acid encoding a component listed in Table 1 is a gene (e.g. a portion of genomic DNA), but alternatively it may be RNA or cDNA that encodes such a component.

For example, the presence of a particular polymorphism in a gene encoding a component from Table 1 may be indicative of learning impairment, etc. Hence, assaying for the presence of such a polymorphism may provide a diagnostic test.

It will be appreciated by those skilled in the art that contained within the nucleic acid sample from the subject to be tested will be nucleic acids that encode the components of naturally-occurring multi-protein complexes comprising NMDA receptors. The base sequence of these nucleic acids may be analysed by probing the nucleic acid sample from the subject with a second nucleic acid which hybridises selectively to the nucleic acids that encode the components of the naturally-occurring multi-protein complex (the target nucleic acid). The target nucleic acid may include DNA, cDNA or mRNA.

It will be further appreciated that the subject to be diagnosed may be from any species of animal, such as humans, dogs, cats, horses, cattle, etc.

Preferably, the subject is human.

Hybridisation experiments may also provide information about the pattern of expression of the genes that encode the components of naturally-occurring multi-protein complexes comprising NMDA receptors

By "a nucleic acid that hybridises selectively" is meant that the nucleic acid has sufficient nucleotide sequence similarity with the said target nucleic acid that it can hybridise under moderately or highly stringent conditions. As is well known in the art, the stringency of nucleic acid hybridisation depends on factors such as length of nucleic acid over which hybridisation occurs, degree of identity of the hybridising sequences and on factors such as temperature, ionic strength and CG or AT content of the sequence. Thus, any nucleic acid which is capable of selectively hybridising is useful in the practice of the invention.

Nucleic acids which can selectively hybridise to the said target nucleic acid include nucleic acids which have >95% sequence identity, preferably those with >98%, more preferably those with >99% sequence identity, over at least a portion of the nucleic acid with the said target nucleic acid. As is well known, human genes usually contain introns such that, for example, a mRNA or cDNA derived from a gene within the said target nucleic acid would not match perfectly along its entire length with the said target nucleic acid but would nevertheless be a nucleic acid capable of selectively hybridising to the said target nucleic acid.

Typical moderately or highly stringent hybridisation conditions which lead to selective hybridisation are known in the art, for example those described in Molecular Cloning, a laboratory manual, 2nd edition, Sambrook et al (eds), Cold Spring Harbor Laboratory Press, Cold Spring Harbor, NY, USA, incorporated herein by reference.

An example of a typical hybridisation solution when a nucleic acid is immobilised on a nylon membrane and the probe nucleic acid is > 500 bases or base pairs is:
6 x SSC (saline sodium citrate)
0.5% sodium dodecyl sulphate (SDS)
100 µg/ml denatured, fragmented salmon sperm DNA

The hybridisation is performed at 68°C. The nylon membrane, with the nucleic acid immobilised, may be washed at 68 ° C in 1 x SSC or, for high stringency, 0.1 x SSC.

20 x SSC may be prepared in the following way. Dissolve 175.3 g of NaCl and 88.2 g of sodium citrate in 800 ml of H2O. Adjust the pH to 7.0 with a few drops of a 10 N solution of NaOH. Adjust the volume to 1 litre with H₂O. Dispense into aliquots. Sterilize by autoclaving.

An example of a typical hybridisation solution when a nucleic acid is immobilised on a nylon membrane and the probe is an oligonucleotide of between 15 and 50 bases is:
3.0 M trimethylammonium chloride (TMACI)
0.01 M sodium phosphate (pH 6.8)
1 mm EDTA (pH 7.6)
0.5% SDS
100 µg/ml denatured, fragmented salmon sperm DNA
0.1% nonfat dried milk

The optimal temperature for hybridisation is usually chosen to be 5°C below the Ti for the given chain length. Ti is the irreversible melting temperature of the hybrid formed between the probe and its target sequence. Jacobs et al (1988) *Nucl. Acids Res.* **16**:4637 discusses the determination of Ti's. The recommended hybridisation temperature for 17-mers in 3 M TMACI is 48-50°C; for 19-mers, it is 55-57°C; and for 20-mers, it is 58-66°C.

By "nucleic acid which selectively hybridises" is also included nucleic acids which will amplify DNA from the said region the target nucleic acid by any of the well known amplification systems such as those described in more detail below, in particular the polymerase chain reaction (PCR). Suitable conditions for PCR amplification include amplification in a suitable 1 x amplification buffer:
10 x amplification buffer is 500 mM KCl; 100 mM Tris.Cl (pH 8.3 at room temperature); 15 mM MgCl2; 0.1% gelatin.

A suitable denaturing agent or procedure (such as heating to 95°C) is used in order to separate the strands of double-stranded DNA.

Suitably, the annealing part of the amplification is between 37°C and 60°C, preferably 50°C.

Although the nucleic acid which is useful in the methods of the invention may be RNA or DNA, DNA is preferred. Although the nucleic acid which is useful in the methods of the invention may be double-stranded or single-stranded, single-stranded nucleic acid is preferred under some circumstances such as in nucleic acid amplification reactions.

The nucleic acid which is useful in the methods of the invention may be very large, such as 100 kb, if it is double stranded. For example, such large nucleic acids are useful as a template for making probes for use in FISH (fluorescence in situ hybridisation) analysis. Typically, the labelled probes used in FISH are generally made by nick-translation or random priming from a genomic clone (such as an insert in a suitable PAC clone). Once made these probes are around 50-1000 nucleotides in length. However, for certain diagnostic, probing or amplifying purposes, it is preferred if the nucleic acid has fewer than 10 000, more preferably fewer than 1000, more preferably still from 10 to 100, and in further preference from 15 to 30 base pairs (if the nucleic acid is double-stranded) or bases (if the nucleic acid is single stranded). Single-stranded DNA primers, suitable for use in a polymerase chain reaction, are particularly preferred.

It will be appreciated that said target nucleic acid may be a gene (or fragment thereof) which encodes a component of a multi-protein complex of the invention. By "gene" is included not only the introns and exons but also regulatory regions associated with, and physically close to, the introns and exons, particularly those 5' to the 5'-most exon. By "physically close" is meant within 50 kb, preferably within 10 kb, more preferably within 5 kb and still more preferably within 2 kb. Serial deletions and footprinting techniques may also be used to identify the regulatory regions.

It is particularly preferred if the nucleic acid for use in the methods of the invention is an oligonucleotide primer which can be used to amplify a portion of the target nucleic acid. Once amplified, the portion of the target nucleic acid may be detected by methods known in the art (see Molecular Cloning, a laboratory manual, 2nd edition, Sambrook et al (eds), Cold Spring Harbor Laboratory Press, Cold Spring Harbor, NY, USA), for example by gel electrophoresis, blotting and hybridisation techniques.

Also described herein is a method of diagnosing or aiding diagnosis of disorders and conditions associated with dysfunction of NMDA receptors in the central nervous system comprising the steps of:
(i) providing a sample containing protein from the subject to be diagnosed;
(ii) isolating a multi-protein complex according to the first aspect of the invention from said sample; and
(iii) determining the composition of said complex and/or the activity of one or more components of said complex.

Thus, a method of diagnosing or aiding diagnosis of disorders and conditions associated with dysfunction of NMDA receptor complexes in the central nervous system may comprise step (i) to (iii) above.

Also described herein is a method of prognosing or aiding prognosis of disorders and conditions associated with dysfunction of NMDA receptors in the central nervous system comprising the steps of:
(i) providing a sample containing nucleic acid from the subject to be prognosed; and
(ii) contacting said nucleic acid from the subject with a second nucleic acid which hybridises selectively to a nucleic acid encoding a component selected from the group of components listed in Table 1, or a mutant allele thereof, or their complement.

Thus, a method of prognosing or aiding prognosis of disorders and conditions associated with dysfunction of NMDA receptor complexes in the central nervous system may comprise step (i) and (ii) above.

Additionally, a method of determining the susceptibility of a patient to disorders and conditions associated with dysfunction of NMDA receptors in the central nervous system may comprise the steps of:
(i) providing a sample containing nucleic acid from the patient to be tested; and
(ii) contacting said nucleic acid from the patient with a second nucleic acid which hybridises selectively to a nucleic acid encoding a component selected from the group of components listed in Table 1, or a mutant allele thereof, or their complement.

Thus, a method of determining the susceptibility of a patient to disorders and conditions associated with dysfunction of NMDA receptor complexes in the central nervous system may comprise step (i) and (ii) above.

It will be appreciated by those skilled in the art that the above methods of diagnosis, prognosis and determination of susceptibility may additionally comprise the step of contacting nucleic acid from the subject with at least one additional nucleic acid which hybridises selectively to a nucleic acid encoding a component selected from the group listed in Tables 1 and 2, or a mutant allele thereof, or their complement, or a mutant allele thereof, or their complement, wherein each nucleic acid that hybridises to the nucleic acid from the subject encodes a different component.

It will be further appreciated that, in the above case, the nucleic acid from the subject used in the additional step(s) need not necessarily be from the same sample as used originally but must be taken from the same subject.

Thus, the methods of the invention allow the diagnosis of disorders that are dependent on abnormalities in more than one gene.

In a preferred embodiment, the one or more nucleic acid which selectively hybridises to the nucleic acid encoding a component of a multi-protein complex or sub-complex of the invention, or a mutant allele thereof, or their complement further comprises a detectable moiety. Suitable detectable moieties include radionuclides, fluorescent labels and gold particles.

It will be appreciated that the one or more nucleic acid which selectively hybridises to the gene or cDNA encoding a component of a multi-protein complex or sub-complex of the invention, or a mutant allele thereof, or their complement may be double- or single-stranded.

Preferably, the one or more nucleic acid which selectively hybridises to the nucleic acid encoding a component of a multi-protein complex or sub-complex of the invention, or a mutant allele thereof, or their complement is less than 1000 base pairs (if said nucleic acid is double-stranded) or bases (if said nucleic acid is single-stranded), for example less than 100 base pairs or bases.

The use of nucleic acid probes in hybridisation studies is known in the art, for example see Sambrook et al (1989) Molecular Cloning: A Laboratory Manual (2nd edition), Cold Spring Habor Laboratory Press, Cold Spring Harbor, NY, USA.

In a preferred embodiment of the above methods of diagnosing, prognosing and determining the susceptibility of a subject to disorders and conditions associated with dysfunction of NMDA receptors in the central nervous system, the method further comprises the step of identifying in the nucleic acid from the patient the presence of a sequence variant of a nucleic acid encoding a component of the multi-protein complex and comparing this sequence variant to sequences in equivalent nucleic acids in one or more test populations. For example, comparisons may be made to the sequences in equivalent nucleic acids found in:
(i) a population of individuals who do not suffer and have not suffered previously from disorders and conditions associated with dysfunction of NMDA receptors in the central nervous system;
(ii) a population of patients who suffer or have suffered previously from schizophrenia, stroke or epilepsy, etc;
(iii) a population of individuals with a family history of schizophrenia, stroke or epilepsy, etc; or
(iv) a population of individuals from the same family as that of the patient (e.g. twins)
and such like.

Exemplary multigene analysis studies of this type are described in Human Molecular Genetics (2nd edition), Strachan & Read (eds.), Bios Scientific Publishers Ltd, Oxford, UK and Principles of Medical Genetics (2nd edition), Gelehrter, Collins & Ginsburg (eds.), Williams & Wilkins, USA.

A further aspect of the invention provides a method of diagnosing, prognosing and/or determining the susceptibility of a subject to disorders and conditions associated with dysfunction of NMDA receptors in the central nervous system, comprising the steps of:
(i) providing a sample containing antibodies from the subject to be tested; and
(ii) determining whether said sample contains antibodies reactive with a multi-protein complex or sub-complex according to the present invention or a component thereof.

Thus, the invention provides a method of diagnosing, prognosing and/or determining the susceptibility of a subject to disorders and conditions associated with dysfunction of NMDA receptor complexes in the central nervous system, comprising the steps of:
(i) providing a sample containing antibodies from the subject to be tested; and
(ii) determining whether said sample contains antibodies reactive with an NMDA receptor complex or sub-complex according to the present invention or a component thereof.

Thus, the presence of antibodies reactive with a multi-protein complex or sub-complex according to the present invention or a component thereof would be indicative of the NMDA receptor -associated disorder.

Preferably the sample of antibodies comprises a sample of blood serum from the subject.

In a preferred embodiment of the above method, a multi-protein complex or sub-complex according to the present invention or component thereof is immobilised on a plastic dish (e.g. a 96-well plate) and serum is then added. Binding of antibodies in the serum to the immobilised complex, sub-complex or component is determined using standard ELISA methods (for example, see Reen, 1994, Methods Mol. Biol. 32:461-466).

A still further aspect of the invention provides a kit of parts that is useful for diagnosing, prognosing or determining the susceptibility of a patient to learning disorders and conditions associated with dysfunction of NMDA receptors in the central nervous system. Thus, the invention provides a kit of parts that is useful for diagnosing, prognosing or determining the susceptibility of a patient to learning disorders and conditions associated with dysfunction of NMDA receptor complexes in the central nervous system

Conveniently, the kit of parts comprises a nucleic acid that selectively hybridises to a nucleic acid encoding a component listed in Table 1, together with enzymes and reagents for promoting nucleic acid-nucleic acid hybridisation and for detecting hybrids formed thereby.

In a preferred embodiment, the kit of parts further comprises one or more additional nucleic acids encoding a component selected from the group listed in Tables 1 and 2, each nucleic acid encoding a different component. Such kits allow detection of multiple gene polymorphisms that may be associated with disorders and conditions associated with dysfunction of NMDA receptors in the central nervous system.

The invention is now described in more detail with reference to the following Figures and Examples:

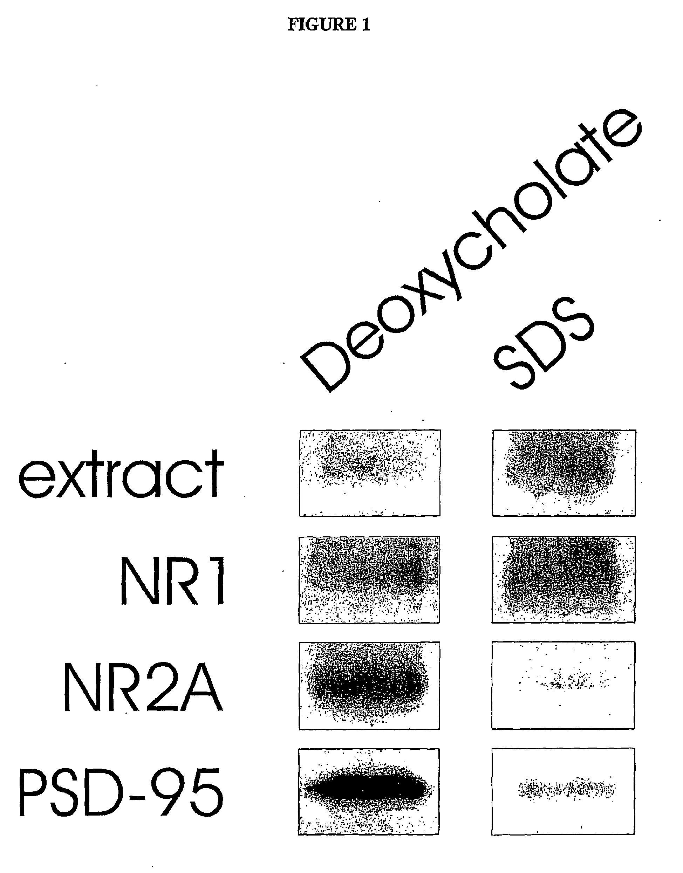

**Figure 1****. Effect of detergent extraction on the composition of a multi-protein complex extracted from mouse brain.**
   Mouse brain multi-protein complexes comprising an NMDA receptor were isolated using small scale immunoprecipitation with the MAP-NR1 sheep serum (4µl) followed by analysis by Western blotting with various antibodies as indicated on the left. The top two panels labelled 'extract' are Western blotting controls of whole brain extracts probed for NR1 with the antibody 54.1. Using 1% deoxycholate (DOC) as the only detergent resulted in the multi-protein complex composition as shown on the left side panels whereas the results depicted on the right were obtained by using a buffer system containing SDS. The exposure time of the blots to X-ray film was 10 seconds.

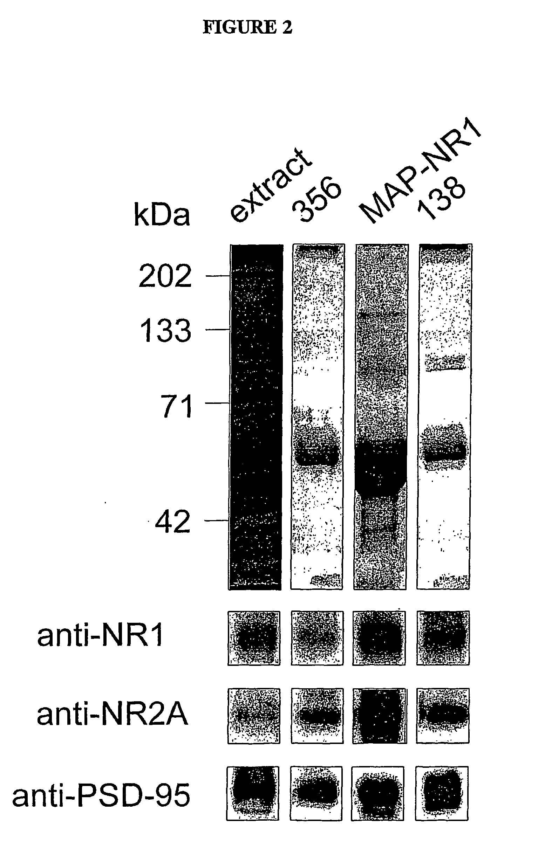

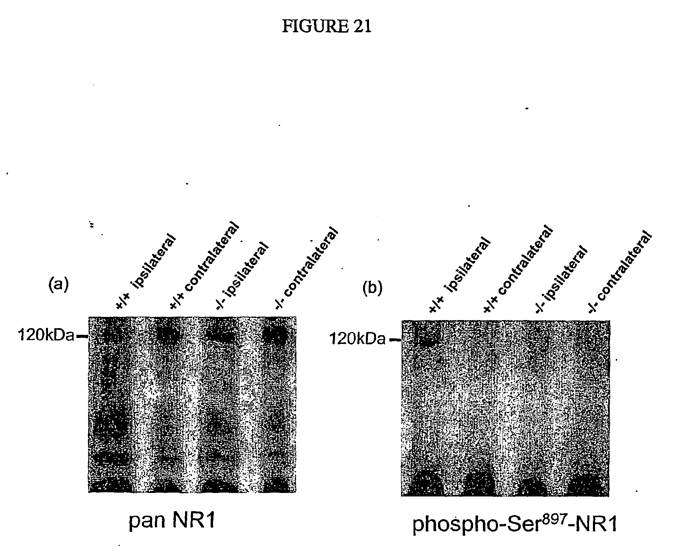

**Figure 2****. Small scale immunoprecipitation with three different IgGs.**
   Mouse brain extracts (0.2 ml) were diluted with equal amount of DOC buffer and incubated with whole serum IgG against NR1 from rabbit (356, 1µl) or sheep (MAP-NR1, 4µl) or whole serum against PSD-95 (138, 1µl) for 1 hour at 4°C. This was followed by an addition of 15 µl Protein G-Sepharose and a further incubation for 1 hour at 4°C. The resins were washed four times with 0.5 ml DOC buffer and analysed by 8% SDS-PAGE and Coomassie staining (top panels). The molecular weight markers are indicated on the left. Western blotting of these samples using the monoclonal antibody 54.1 against NR1 and anti-PSD-95, and the polyclonal IgG anti-NR2A are shown on the lower panels.

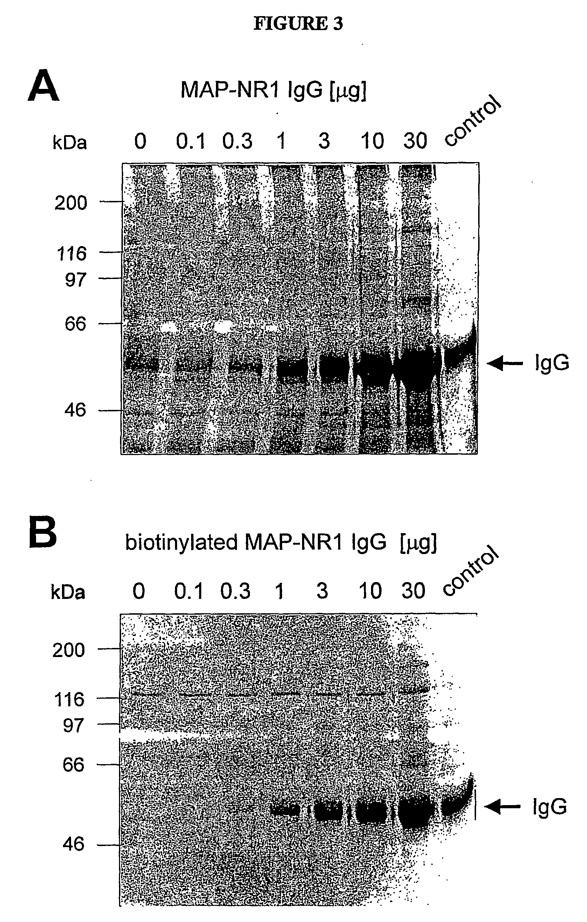

**Figure 3****. Titration of MAP-NR1 antibody.**
   Small scale experiments were done using 0 - 30 µg of purified (panel A) or biotinylated (panel B) MAP-NR1 IgGs mixed with 0.2 ml mouse brain DOC extract and 0.2 ml DOC buffer. 20 µl Protein G-Sepharose was added after 2 hours incubation at 4°C followed by a further incubation overnight at 4°C. The samples were washed 4 times with 0.5 ml DOC buffer and analysed on 7.5% SDS-PAGE gels followed by Coomassie staining. The controls were 3 µg of antibody in 0.4 ml DOC buffer processed as the samples described above. The arrows indicate the position of the IgG heavy chain and molecular weights in kDa are indicated on the left of each panel.

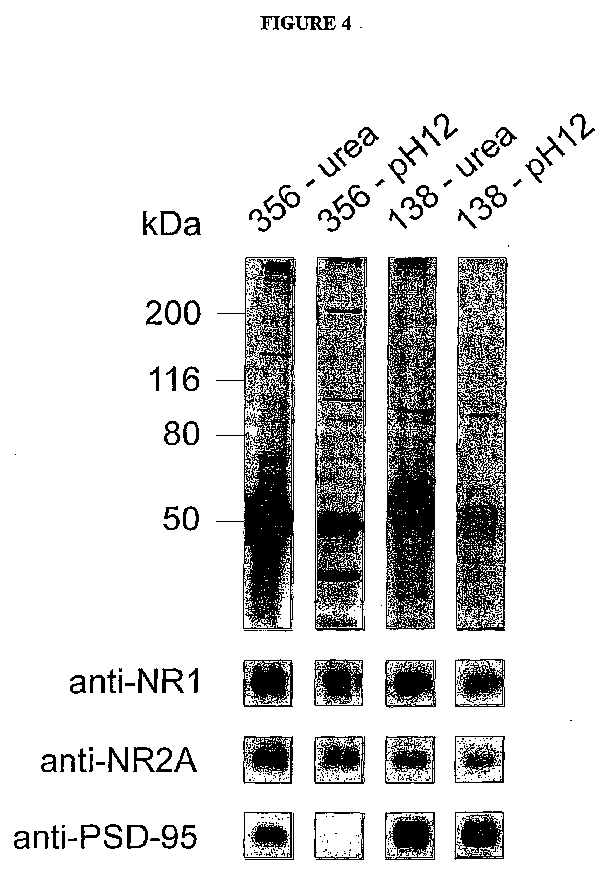

**Figure 4****. Affinity chromatography using immobilised antibodies.**
   The multi-protein complex was isolated using immobilised antibodies against NR1 (356) or PSD-95 (138) and eluted from the resins either with 6M urea or a pH shift to pH12. The samples were analysed by 7.5% SDS-PAGE and Coomassie staining (top panels) and by Western blotting using antibodies against NR1, NR2A and PSD-95 (lower panels). Molecular weight markers in kDa of the SDS-PAGE are indicated on the left.

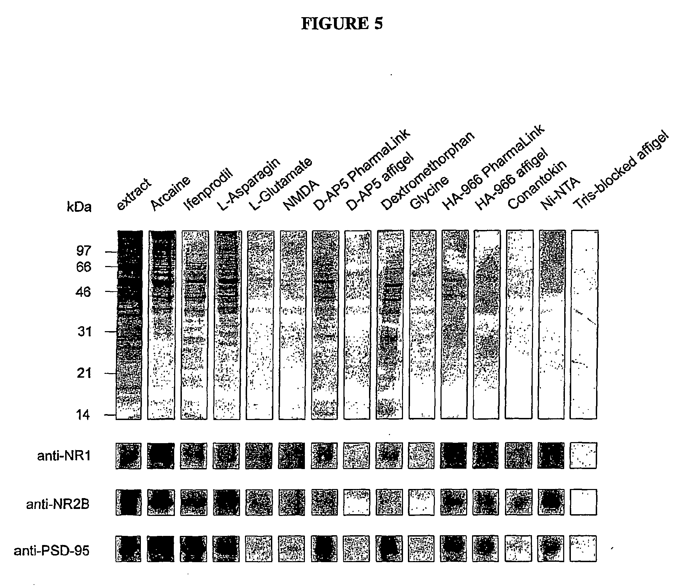

**Figure 5****. Ligand affinity purification of mouse brain extracts.**
   Brain DOC extracts were incubated with affigel-10- or PharmaLink-coupled ligands and retained proteins were resolved by 12% SDS-PAGE and Coomassie staining (top panels). The molecular weight markers in kDa are indicated on the left. The lower panels show the Western blotting analysis of the various samples using antibodies against NR1, NR2B and PSD-95. Whole extract (10 µl) was used as a control and is shown on the leftmost panels.

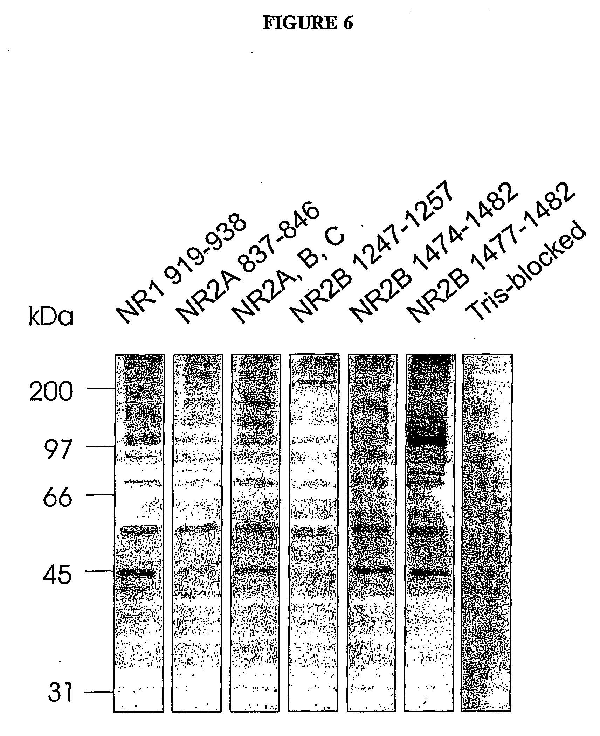

**Figure 6****. Peptide affinity purification of mouse brain extracts.**
   Small-scale experiments were performed using various peptides covalently linked to affigel-10, or a negative control of Tris-blocked affigel-10, and 0.5 ml mouse brain DOC extracts and analysed by 10% SDS-PAGE followed by Coomassie staining. The molecular weight markers in kDa are indicated on the left.

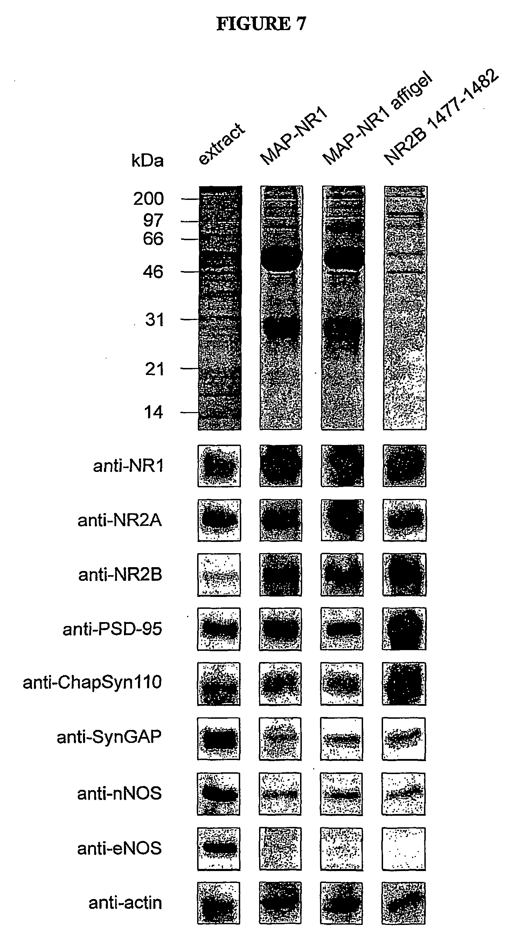

**Figure 7****. Comparative analysis of the purified multi-protein complex using three different strategies.**
   Mouse brain extracts (50 ml) were used each for large scale immunoprecipitation with 0.5 mg affinity-purified MAP-NR1 IgG (lanes MAP-NR1), upscaled immunoaffinity chromatography with 0.5 ml MAP-NR1 affigel (lanes MAP-NR1 affigel), and 0.5 ml NR2B C-terminal peptide resin (lanes NR2B 1477-1482). 10 µl of extract was used per lane as a control (lanes extract). The samples were analysed by 12 % SDS-PAGE and Coomassie stained (upper panels) or used for Western blotting probed with the antibodies as depicted (lower panels). Molecular weight markers in kDa of the SDS-PAGE gel are marked on the left.

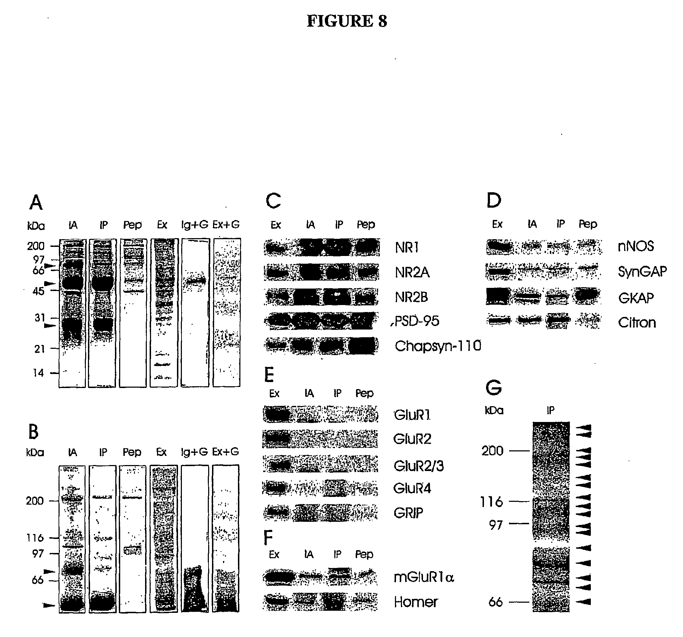

**Figure 8****. Basic composition of multi-protein complexes isolated from mouse brain.**
   **A, B:** Protein staining of SDS-PAGE (A, 12%, B, 6%) from immunoaffinity (lane IA), immunoprecipitation (lane IP) and NR2B peptide affinity (lane Pep) isolation of a multi-protein complex from mouse brain extracts (lane Ex). Negative controls shown are MAP-NR1 Ig bound to Protein G-Sepharose (lane Ig+G) and extracts bound to Protein G-Sepharose (lane Ex+G). Arrowheads indicate position of Ig species. A complex mixture of specific binding proteins were observed mainly in the range above 70kDa, and were subject to Mass Spectrometric analysis (Figure 1G, Table 2).
   **C.** Immunoblot analysis of NMDA receptor subunits and MAGUK proteins in the multi-protein complex. NR1, NR2A, NR2B, PSD-95 and Chapsyn-110 were detected.
   **D.** Immunoblot analysis of PSD-95 binding proteins in the multi-protein complex. nNOS, SynGAP, GKAP and Citron were detected.
   **E.** Immunoblot analysis of AMPA receptor subunits and GRIP in the multi-protein complex. GluR1, GluR2, GluR2/3, GluR4 and GRIP were readily detected in the starting material (Ex) but not detected in the three multi-protein complex preparations.
   **F.** Immunoblot analysis of mGluR1 receptor and Homer proteins in the multi-protein complex. mGluR1 and Homer were detected indicating the multi-protein complex includes metabotropic glutamate receptors but not AMPA receptors.
   **G.** Protein staining of the multi-protein complex (6% SDS-PAGE) showing position (arrowheads) of bands excised for mass spectrometry.

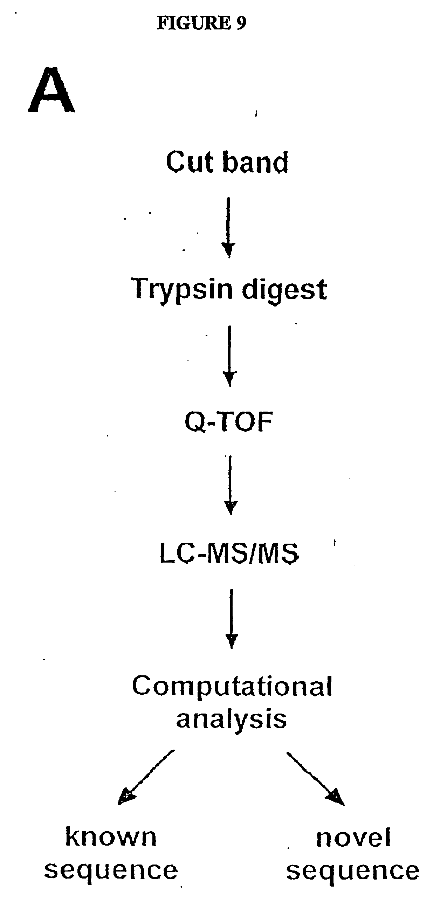

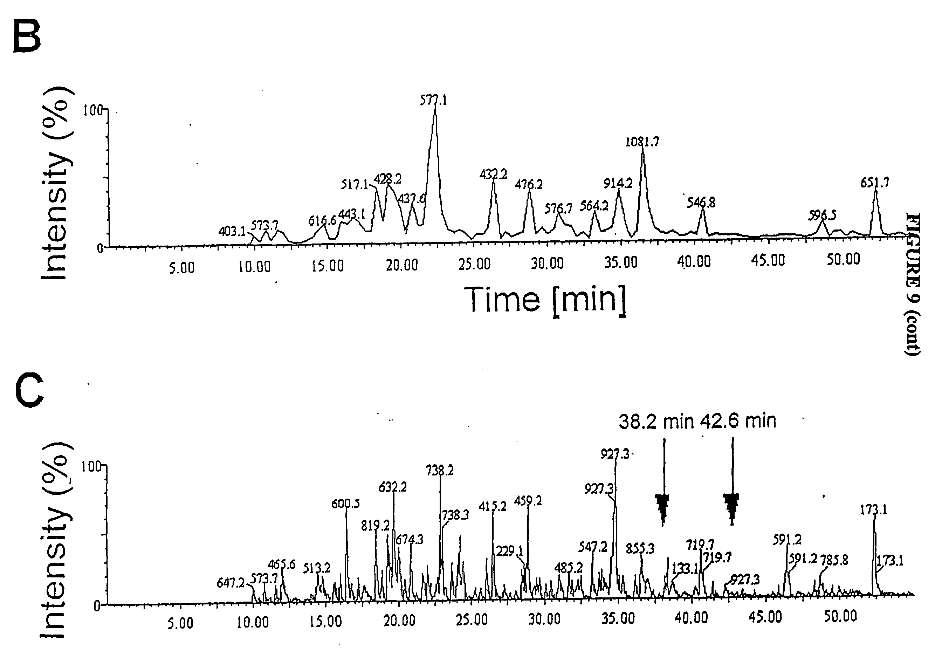

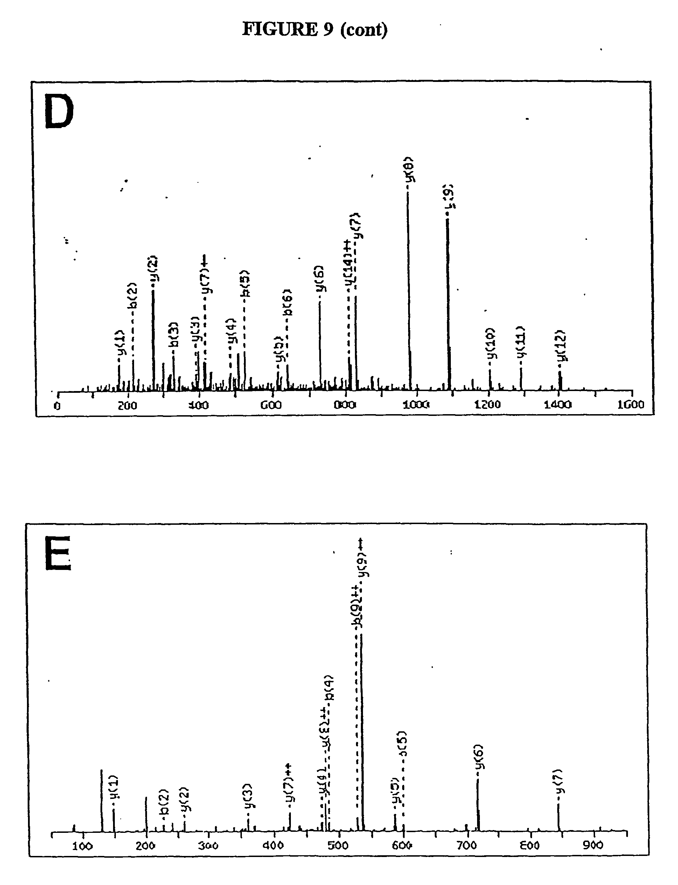

**Figure 9****. Mass Spectrometry analysis of the multi-protein complex**
   **A.** Protein identification strategy. Stained bands from SDS-PAGE (Figure 1G) were excised and digested with trypsin and analysed using Q-TOF (Panel B), LC-MS/MS (Panels C,D,E) followed by online computational analysis.
   **B.** Total Ion Survey Base peak chromatograms were obtained (example from protein band at 90 kDa). The two most intense ions in each MS scan are automatically selected for collision induced fragmentation and analysis in MS/MS mode (LC-MS/MS)(Panel C).
   **C.** LC-MS/MS Base peak chromatogram. The labels at 38.2 min and 42.6 min indicate selected peptides which were used to generate peptide sequence information, two such examples are shown (Panel D, E).
   **D.** Averaged MS/MS spectrum obtained from the peak eluting at 42.6 min of Panel C. A doubly charged ion at mass to charge ratio (m/z) 890.72, corresponding to a peptide of 1617.44 Da, was selected for fragmentation by collision induced dissociation. The fragment ions that originate either from the N-terminus (b type ions) or the C-terminus (y" type ions) correspond to an amino acid sequence that was identified using the Mascot database search program. In this instance the MS/MS spectrum matched the peptide sequence VNDSILFVNEVDVR from PSD-95 residues 113-126 (Accession number Q62108).
   **E.** Example of a novel component of the multi-protein complex identified from the averaged MS/MS spectrum obtained from a peak eluting at 38.2 min of Panel C. The observed doubly charged precursor ion at m/z 538.68 relating to peptide of 1071.65 Da was identified by Mascot search program as the sequence DLKEILTLK that matches dbEST clone AV153731.

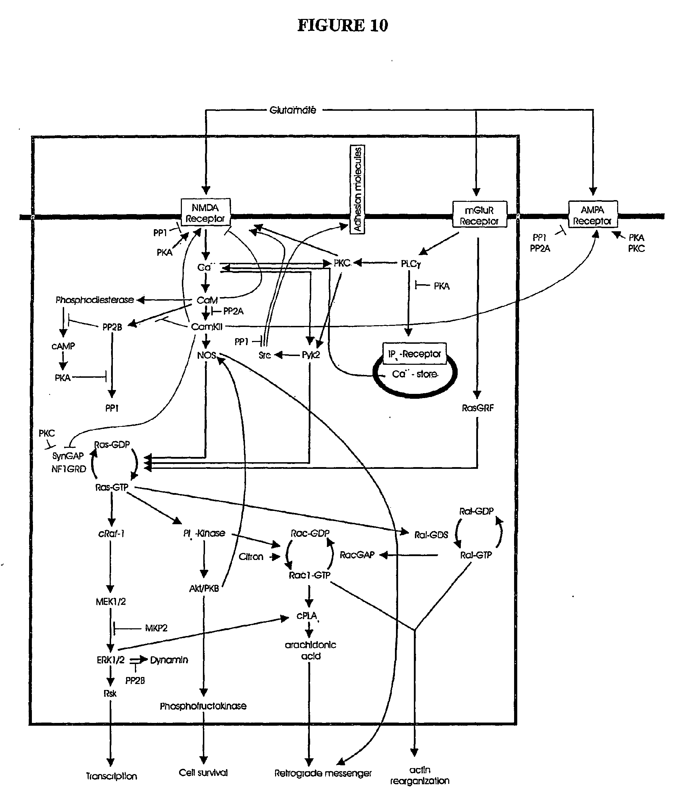

**Figure 10****. Putative signalling pathways involving components of** multi-protein complex.
   Many of the multi-protein complex components are parts of known biochemical pathways. Some components of the pathways are illustrated and suggested routes of regulating AMPA receptors, synaptic structural changes (actin reorganisation and cell adhesion), neuronal survival and transcription are indicated. Adaptor and other structural molecules including PSD-95, Yotiao and AKAPs are not depicted. The postsynaptic membrane is shown as a solid line and the grey box encompasses multi-protein complex components.

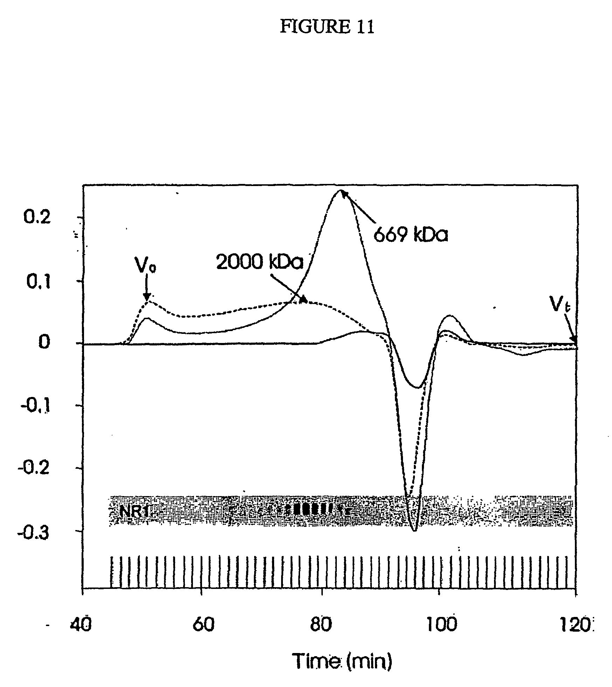

**Figure 11** **Gel filtration analysis of soluble multi-protein complex**
   Complexes were isolated by large scale IP and competed from the resin by peptide competition as described in the Material and Methods section, followed by fractionation by size exclusion chromatography. The graphs depict two calibration runs using Blue Dextran 2000 (dashed line) and thyroglobulin (dotted line) as standards and the separation of the NMDAR sample (solid line). Fractions were taken between the elution volume (v0) and the total column volume (vt) and analysed by Western blotting against NR1 (inset).

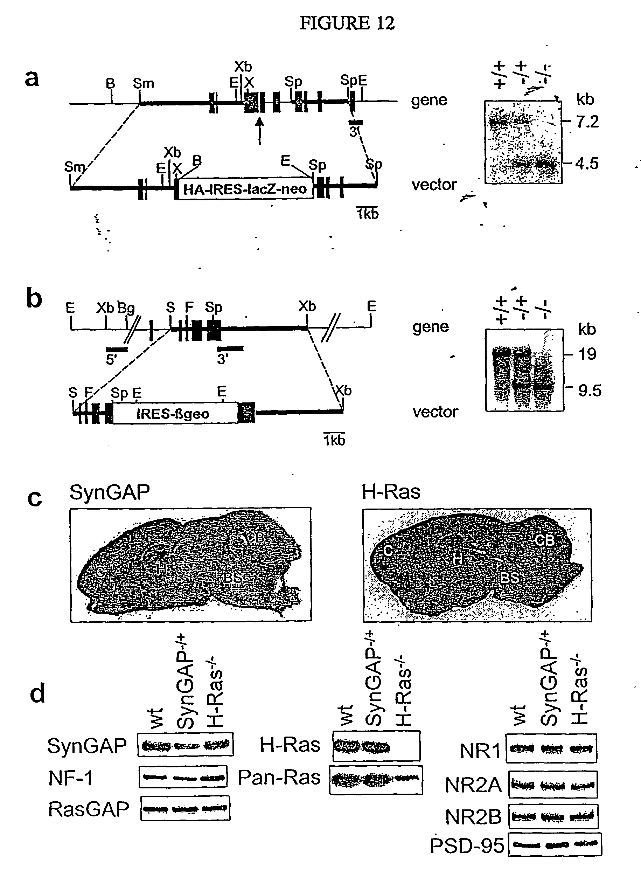

**Figure 12****. SynGAP and H-Ras mutant mice**
   12a. Targeted disruption of SynGAP gene. Top: SynGAP gene with restriction enzyme sites (B, BglII; E, EcoRI; Sm, SmaI; Sp, SpeI; X, XhoI; Xb, XbaI); black boxes, exon; thick horizontal lines, homologous regions used in the targeting vector; Southern blot probes are indicated (3'). Bottom: SynGAP targeting vector. HA, hemagglutinin sequence; IRES, internal ribosomal entry site; 1acZ, β-galactosidase gene; neo, neomycin- resistance gene. The arrow below the SynGAP gene indicates arginine 312 (or 470) (see Example 4 below), which is highly conserved in RasGAP proteins and necessary for GAP activity, and was deleted in the targeting vector. Right of figure: genomic Southern blots of EcoR1-digested DNA from littermates of a heterozygote intercross and probed with the 3' probe. Wild type (+/+), heterozygote (-/+), homozygote (-/-) are indicated.
   12b. Targeted disruption of H-Ras gene. Top: H-Ras gene with restrictions enzyme sites (as above with addition of: Bg, BgII; S, SphI; F, FspI), all other detail as in Fig. 1a. Bottom: targeting vector. B-geo, consist of a β-galactosidase gene and a neomycin- resistance fusion gene. Right of figure: genomic Southern blots of EcoR1-digested DNA from littermates of a heterozygote intercross and probed with the 5' probe. Wild type (+/+), heterozygote (-/+), homozygote (-/-) are indicated.
   12c. Expression patterns of SynGAP and H-Ras using X-gal staining of whole mount sagital brain sections. BS, brain stem; C, cortex; CB, cerebellum, H, hippocampus. Representative SynGAP-/+ and H-Ras-/sections are shown.
   12d. Immunoblots comparing different protein levels of wild type mice, SynGAP-/+ and H-Ras-/- mutants in hippocampus extracts.
   Left panels: Immunoblot analysis of GAP proteins. SynGAP, NF-1 and Ras-GAP were detected in wild type (wt), SynGAP-/+ and H-Ras-/extracts. A reduced amount of SynGAP protein was observed in SynGAP-/ + mutants compared with wild type.
   Middle panels: Immunoblot analysis of Ras proteins. H-Ras was detected in wild type mice and was normal in SynGAP-/+ mutants. Pan-Ras antibodies, which recognize all Ras isoforms revealed lower total Ras levels in H-Ras-/- mutants and normal levels in SynGAP-/+ mutants.
   Right panels: Immunoblot analysis of NMDAR subunits (NR1, NR2A, NR2B) and PSD-95. Equivalent levels were observed in wild type (wt), SynGAP-/+ and H-Ras-/- extracts.

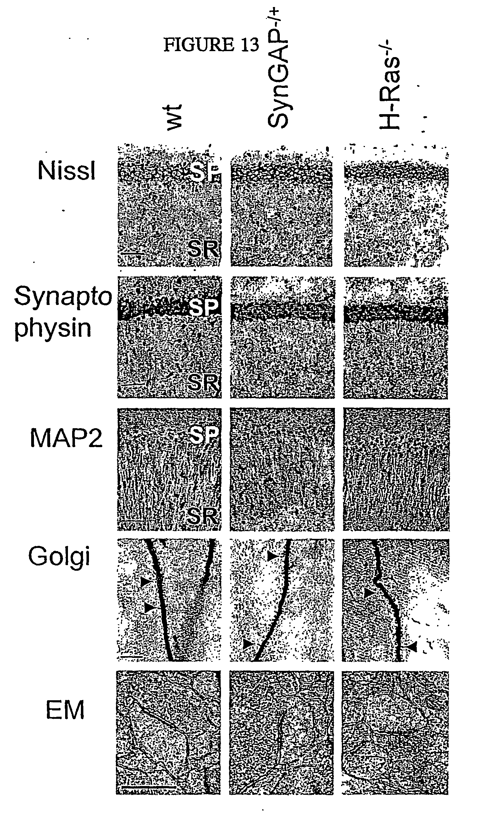

**Figure 13** **Neuroanatomy of hippocampus CA1 region in SynGAP-/+and H-Ras-/- mutant mice.**
   Nissl; cresyl violet stain of CA1 pyramidal cells.
   Synaptophysin; immunohistochemistry for synaptic vesicle marker protein.
   MAP2; immunohistochemistry for dendritic marker protein.
   Golgi; Montaged images of CA1 apical dendrites from Golgi-impregnated pyramidal neurons in the distal region of the stratum radiatum.
   EM; Electron micrograph images of asymmetric axospinous synapses in the stratum radiatum of the CA1 region.
   Scale bars; Nissl, Synaptophysin, MAP2 50µm; Golgi, 10µm; EM, 0.5µm.
   SR, stratum radiatum; Sp. Pyramidal cell body layer; wt, wild type; SynGAP-/ + and H-Ras-/- mutants are indicated.

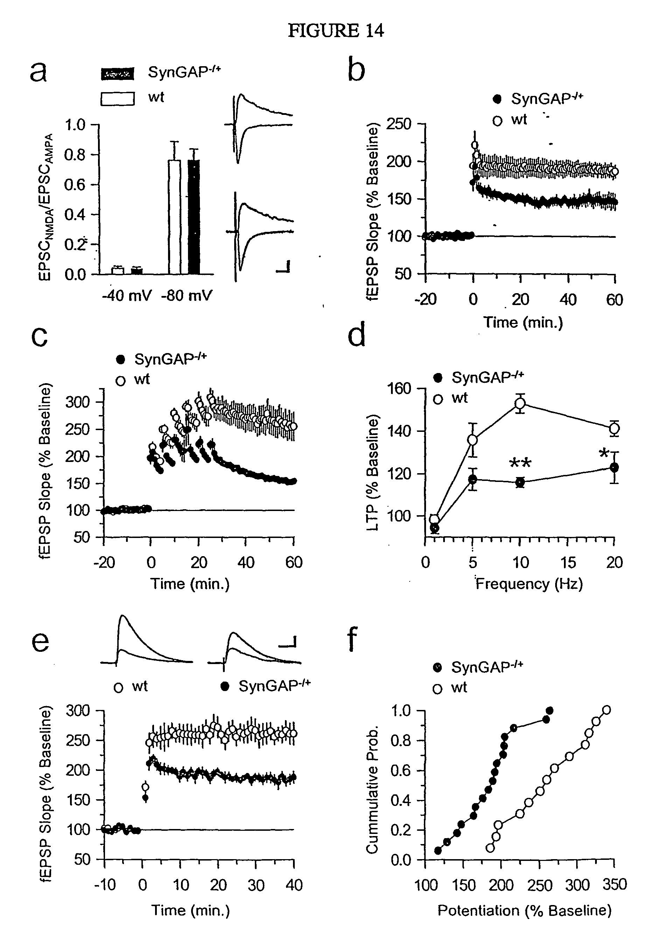

**Figure 14** **NMDAR dependent hippocampal LTP in SynGAP mutant mice**
   LTP is reduced in SynGAP-/+ mutants in the absence of changes in NMDA receptor-mediated synaptic currents.
   14a. The magnitude of the NMDA receptor-mediated component of EPSCs was estimated by the amplitude of the synaptic currents measured 50 milliseconds after the start of the EPSC and expressed relative to the size of the AMPA receptor component estimated by the size of the EPSC measure 5 milliseconds after the start of the EPSC. The size of the NMDA receptor-mediated component of the EPSCs in pyramidal cells from SynGAP-/+ mutant mice (filled bars, N = 5 mice, 15 cells) was not different from that observed in wild-type pyramidal cells (open bars, N = 5 mice, 16 cells) at holding potentials of -80 mV (where the NMDA component is largely blocked by extracellular Mg2+) and +40 mV. The inset shows example EPSCs (average of 3 responses) recorded at -80 and +40 mV in cells from SynGAP-/+ mutants (bottom) and wild-type mice (top). Calibration bars are 50 pA and 25 milliseconds.
   14b. Following a 20 minute period of baseline recording, two trains (1 sec. duration) of 100 Hz stimulation were delivered with an inter-train interval of 10 seconds at time 0. While this protocol induced robust LTP in slices from wild-type animals (open symbols, fEPSPs potentiated to 188 ± 9% of baseline, N = 11 slices from 7 animals), it induced significantly less LTP in slices from SynGAP-/+ mutant mice (filled symbols, fEPSPs potentiated to 150 ± 10% of baseline, N = 11 slices from 7 animals, t(12) = 3.14, p < 0.01 compared to wild-type LTP).
   14c. Six trains of 100 Hz stimulation (each 1 second in duration) were delivered with an inter-train-interval of 5 minutes beginning at time = 0 to induce saturating levels of LTP. In wild-type slices (open symbols, N = 7 slices from 4 animals) fEPSPs were potentiated to 257 ± 25% of baseline but potentiated to only 154 ± 3 % of baseline in slices from SynGAP-/+ mutant mice (filled symbols, N = 11 slices from 5 animals, t(7)=4.71, p < 0.005 compared to wild-type).
   14d. Summary graph showing the amount of LTP present 40-45 minutes after 900 pulse trains of either 1, 5, 10, or 20 Hz stimulation in slices from wild-type (open symbols, N's = 5, 5, 5, and 7 animals respectively for each frequency) and SynGAP-/+ mutant mice (filled symbols, N's = 9, 7, 7, and 7 animals respectively for each frequency). While 1 Hz and 5 Hz trains of synaptic stimulation had similar effects on synaptic transmission in slices from SynGAP-/+ and wild-type mice, 10 Hz and 20 Hz trains of stimulation induced significantly less LTP in slices from SynGAP-/+ mice (**p < 0.001, *p < 0.05).
   14e. LTP induced by low-frequency (2 Hz) presynaptic fiber stimulation paired with postsynaptic depolarization is reduced in CA1 pyramidal cells from SynGAP-/+ mice. EPSPs were paired with postsynaptic depolarization at time = 0. EPSPs recorded between 25 and 30 minutes post-pairing were potentiated to 262 ± 15 % of baseline in cells from wild-type slices (open symbols, N = 13 cells from 7 animals) and to 185 ± 9% of baseline in cells from SynGAP-/+ mice (filled symbols, N = 12 cells from 8 animals, t(13)=4.35, p < 0.001 compared to wild-type). The inset shows EPSPs (average of 3 responses) recorded during baseline and 30 minutes post-pairing in cells from a wild-type animal (left set of traces) and SynGAP-/+ mutant animal (right set of traces). Calibration bars are 5 mV and 25 milliseconds.
   14f. Cumulative probability distribution showing the amount of pairing-induced LTP seen in all cells represented by the average results shown in E (open symbols are cells from wild-type animals, filled symbols are cells from SynGAP-/ + mice).

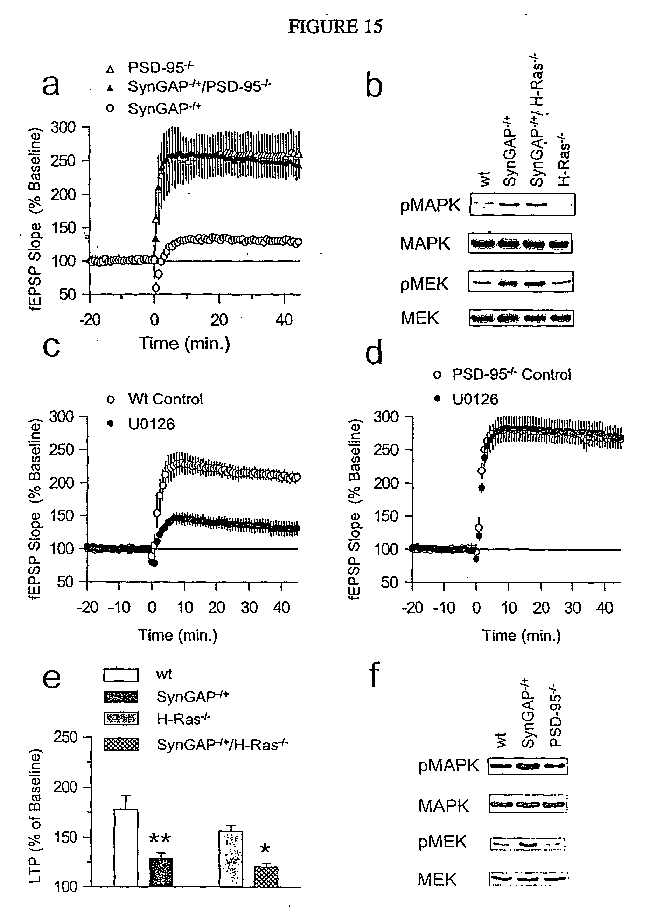

**Figure 15****. PSD-95 couples a SynGAP-MAPK pathway and MAPK-independent pathway.**
   15a. Following a brief train of 5 Hz stimulation (150 pulses, delivered at time = 0) synaptic transmission was potentiated more than 2-fold LTP in slices from PSD-95 (open triangles, fEPSPs potentiated to 258 ± 33 % of baseline, N = 6 slices from 4 animals) and SynGAP-/+/PSD-95 double mutant animals (filled triangles, fEPSPs potentiated to 244 ± 7% of baseline, N = 9 slices from 4 animals). Results from experiments investigating the effects of 5 Hz stimulation on synaptic strength in SynGAP-/+ mutant slices is shown for comparison (open circles, data from experiments summarized in part e).
   15b. MAPK pathway phosphorylation in SynGAP, H-Ras, SynGAP/Ras mutant hippocampus extracts. Immunoblots measuring MEK and MAPK phosphorylation (pMEK, pMAPK) and protein levels (MEK, MAPK) are shown wild type mice (wt), SynGAP-/+, SynGAP-/+/H-Ras-/-and H-Ras-/- mutants. Phosphorylated forms of MEK and MAPK were increased in SynGAP-/+and SynGAP-/+/H-Ras-/- mutants.
   15c. The MEK inhibitor U0126 (20 *M) inhibits 5 Hz stimulation induced LTP in wild type slices. Slices were continuously bathed in ACSF containing U0126 for at least 60 minutes before 5 Hz stimulation (delivered at time = 0). While robust LTP was induced in vehicle control experiments (0.2 % DMSO, open symbols, fEPSPs potentiated to 209 ± 7% of baseline, N = 6 slices), significantly less LTP was induced in U0126-treated slices (filled symbols, fEPSPs were 137 ± 9% of baseline, N = 11 slices, t(15) = 5.4, p < 0.001 compared to controls).
   15d. In slices from PSD-95 mutant mice U0126 had little effect on 5 Hz stimulation-induced LTP. fEPSPs were potentiated to 262 ± 14% of baseline (N = 8 slices) in vehicle control experiments and to 270 ± 12% of baseline (N = 14 slices) in slices continuously exposed to U0126 (t(20) = 0.45, not significantly different from vehicle control experiments).
   15e. Summary of the amount of potentiation seen 45 minutes following a 150 pulse train of 5 Hz stimulation in wild-type, SynGAP-/+, H-Ras-/-, and SynGAP-/+/H-Ras-/- double mutants. Following a brief train of 5 Hz stimulation (150 pulses) synaptic transmission was potentiated to 178 ± 14% of baseline in slices from wild-type animals (N = 12 slices from 6 animals) but only to 129 ± 5% of baseline in slices from SynGAP-/+ mice (N = 10 slices from 5 animals, t(9)=3.03, p < 0.02** compared to wild-type). The amount of LTP induced by this protocol was also significantly reduced in SynGAP-/+/H-Ras-/- double mutants (fEPSPs were 120 ± 4% of baseline, N = 9 slices from 5 animals, t(9)=2.36, p < 0.05*) compared to that seen in H-Ras-/- mutant mice (fEPSPs potentiated to 157 ± 6% of baseline, N = 12 slices from 6 animals).
   15f. MAPK pathway phosphorylation in SynGAP and PSD-95 mutants. Immunoblots measuring MEK and MAPK phosphorylation (pMEK, pMAPK) and protein levels (MEK, MAPK) are shown in wild type mice (wt), SynGAP-/+ and PSD-95-/- mutants. Phosphorylated forms of MEK and MAPK in PSD-95-/- mutants were equivalent to wild type levels in contrast to increased levels in SynGAP-/+ mutants.

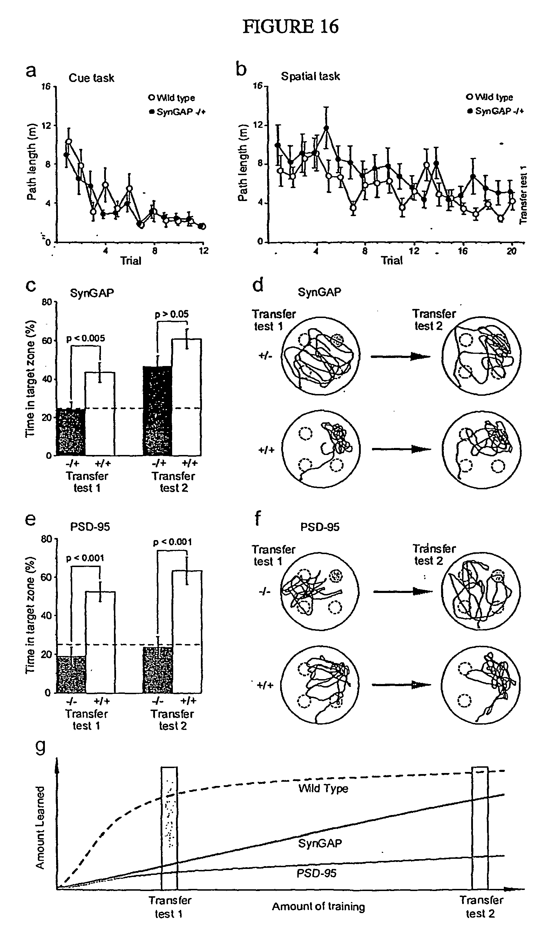

**Figure 16** **Learning and memory in SynGAP mutant mice**
   16a. Path length (mean ± s.e.m.) across 3 days of training to a visible cue (4 trials per day) in SynGAP-/+ (n = 21) and wild type (n = 21) mice. Both groups performed equally well.
   16b. Path length across 5 days of spatial training to a hidden platform. SynGAP mutants ere slightly impaired relative to wild types.
   16c. Time spent in the target zone on transfer tests 1 and 2, expressed as a percentage of the total time spent in all 4 zones. On transfer test 1, SynGAP mutants were significantly impaired relative to wild types, but following training o a performance criterion, mutants searched almost as accurately as wild types on transfer test 2.
   16d. Representative swim paths of individual SynGAP-/+ and wild type mice on transfer tests 1 and 2. Dashed circles indicate the 4 zones, with the target zone filled in grey. Platform locations were counterbalanced, but swim paths have been rotated for display purposes such that the target zone always appears in the NE quadrant of the pool. The wild type mouse searched in the correct location in both transfer tests, whereas the mutant performed at chance on transfer test 1, but searched almost as accurately as the wild type on transfer test 2.
   16e. Time spent in the target zone on transfer tests 1 and 2 for PSD-95-/mutants (n = 12) and wild types (n = 9). Wild types performed well in both transfer tests, but PSD-/- mutants performed at chance throughout.
   16f. Representative swim paths of a PSD-95-/- mutant and a wild type mouse. Note that the mutant fails to search in the target location even after extensive training (transfer test 2).
   16g. A possible framework for interpreting the transfer test data. Wild type mice learn rapidly, soon reaching an asymptote. SynGAP mutants learn slowly, but gradually begin to approach an equivalent asymptote to that reached by wild types. PSD-95 mutants, in contrast, learn little regardless of the amount of training that they receive.

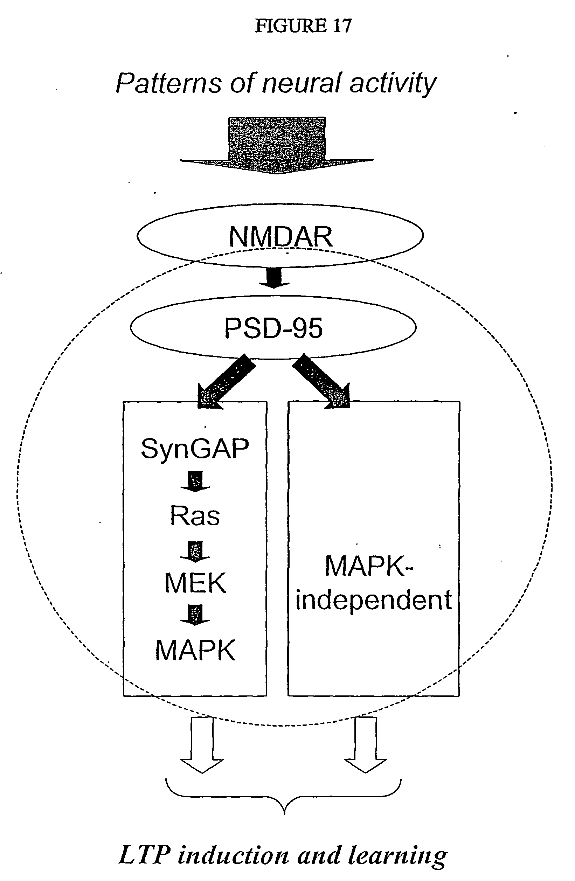

**Figure 17** **Schematic of pathways linking NMDAR, PSD-95, SynGAP and MAPK**
   The functional relationships between pathways of multi-protein complexes (large dotted circle) are described. NMDAR is upstream of PSD-95 which is itself upstream of two pathways (boxed). The SynGAP to MAPK pathway is involved with enhancing LTP and the MAPK-independent pathway restrains LTP. The concerted action of these pathways may control the induction threshold for synaptic plasticity and thus the expression mechanisms of LTP such as modulation of AMPA receptors, which lie further downstream.

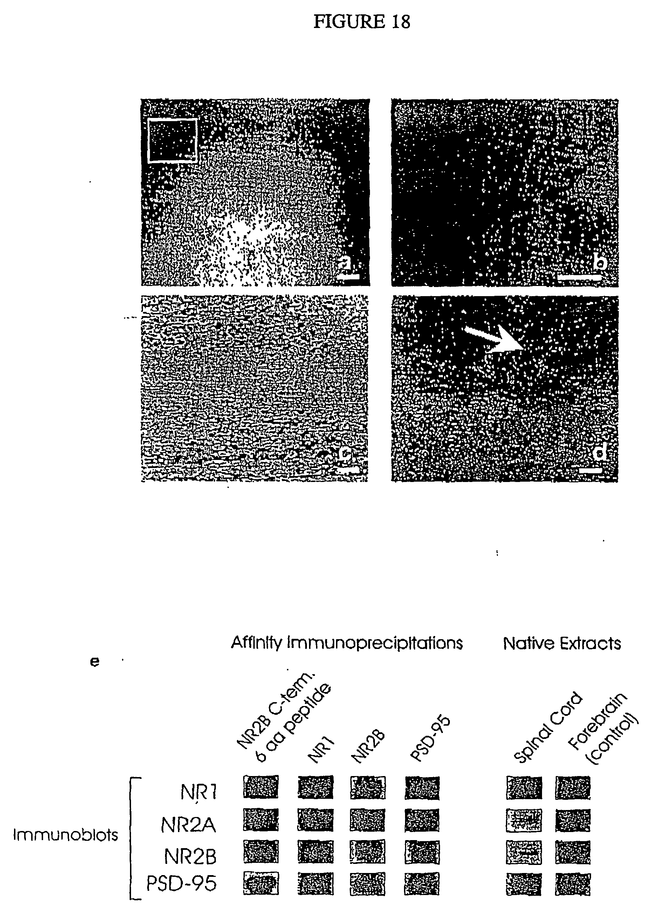

**Figure 18****: Expression of PSD-95 and components of the NMDA receptor complex in spinal cord**
   (A-D) show β-galactosidase expression in lumbar spinal cord (L3-L6) of heterozygous PSD-95 mutant mice. Animals (n=5) were perfused with 4% paraformaldehyde in 0.1M phosphate buffer. 10µm sections were washed with PBS/MgCl₂ and then (0.041 % MgCl₂, 0.01%Na deoxycholate, 0.02% Nonidet-NP40, in 0.1M PBS) on ice. Sections were stained at 22°C in (0.042% potassium ferrocyanide, 0.033% potassium ferricyanide in detergent buffer with 1µg/ml 5-bromo-4-chloro-3-indolyl-β-D-galactoside and incubated at 37°C in the dark for 4-6hrs. (A), Transverse section, showing specific distribution of positive cells in lamina II of the superficial dorsal horn. (B) Inset of (A), showing positive cells at higher magnification (C), Longitudinal sections through dorsal horns, cut parallel to dorsal surface, showing β-galactosidase-positive cells. (D) Longitudinal section showing staining in dorsal horn, but not at the site of dorsal root entry (arrow). Scale bar =10µm. (E) Shows immunoblots for the presence of NMDA receptor complex proteins in affinity immunoprecipitations of L3-L6 spinal cord from wild type mice. Direct immunoblots on spinal cord and forebrain extracts are shown for comparison.

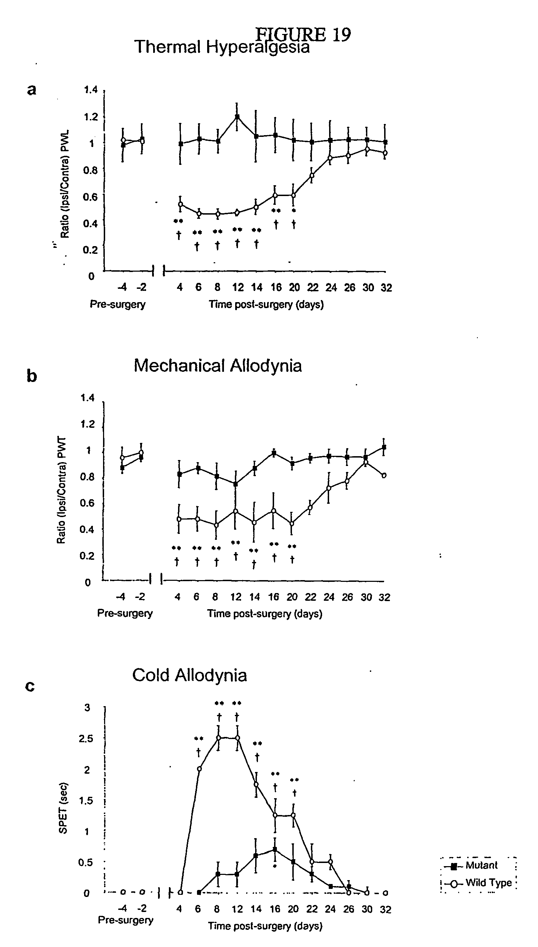

**Figure 19****: Behavioural analysis of wild type and PSD-95 mutant mice with chronic constriction injury (CCI) to the sciatic nerve**
   (a) In wild type mice (open circles), paw withdrawal from a noxious thermal stimulus ipsilateral to CCI performed on the sciatic nerve for wild type mice, show significant differences between pre-operative compared to post-operative values on the same side (†p≤0.05; Kruskal-Wallis one-way ANOVA) and from post-operative, contralateral values (*p≤0.05; **p≤0.001, by Student's t test). Data are displayed as the ratio of ipsilateral to contralateral paw withdrawal latency (PWL). No corresponding differences that would reflect thermal hyperalgesia were seen at all in the mutant mice (filled squares) or on the contralateral side of wild type mice.
   (b) Paw withdrawal to mechanical stimulation for wild type mice (open circles) showed significant differences between pre- and post-operative values on the side ipsilateral to CCI (†p≤0.01; Dunn's Method ANOVA on ranks) and between ipsilateral and contralateral values post-operatively (**p<0.001, Mann-Whitney Rank Sum Test). Data are plotted as the ratio of ipsilateral to contralateral paw withdrawal threshold (PWT). No significant differences were seen on any day of testing for the PSD-95 mutant (filled squares) (or on the contralateral side of wild types), indicating the lack of development of mechanical allodynia.
   (c) The Suspended Paw Elevation Time (SPET) in response to a cold (4° C) stimulus is shown for the ipsilateral side in wild type mice (open circles) and PSD-95 mutant mice (filled squares). SPET scores were always zero on the contralateral side for both wild type and mutant mice. Significant differences are shown between pre- and post-operative values (†, p<0.01, Dunn's Method ANOVA on ranks) and between ipsilateral and contralateral responses (**, p<0.01, Mann-Whitney Rank Sum Test). Eight wild type and nine PSD-95 mutant mice were tested in each case. Mean ± SEM values are shown.

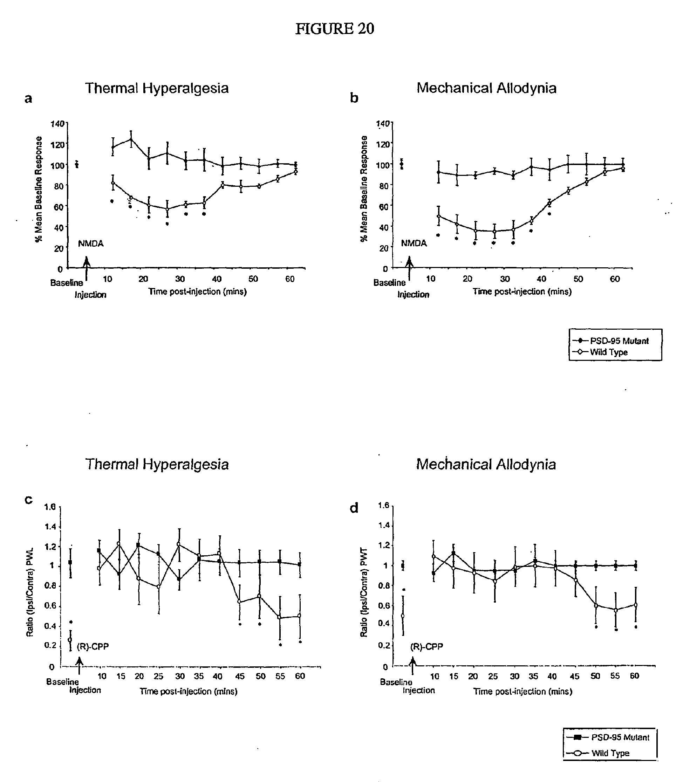

**Figure 20****: Effects of the intrathecal injection of NMDA in naive mice and of the NMDA receptor antagonist (R)-CPP in CCI mice**
   In (a) paw withdrawal latency (PWL) was measured in response to noxious thermal stimulation, before and after the intrathecal administration of NMDA (0.25nmol in 10µl, at arrow) to naive wild type (open diamonds; n=6) and PSD-95 mutant mice (filled diamonds; n=7).
      Statistical significance (* p≤0.01) was determined by Student's t test on raw PWL data.
   In (b), the paw withdrawal threshold (PWT) was measured in responses to mechanical stimulation before and after the intrathecal application of NMDA (at arrow) to wild type (open diamonds) and PSD-95 mutant mice (filled diamonds). Statistical significance was determined using the Mann-Whitney Rank Sum test (*p≤0.01). Data for (a) and (b) are the percentage of the mean pre-injection baseline response plotted as means ± SEM.
   In (c), wild type mice at the peak of thermal hyperalgesia, ipsilateral to CCI, induced 12 days previously, were intrathecally injected with (R)-CPP (arrow) at a dose of 100 pmol in 10 µl to assess reversal of sensitisation (open circles; n=6). (R)-CPP had no effect when administered to similarly prepared PSD-95 mutants (filled squares; n=7). Data are plotted as the mean (± SEM) ratio of ipsilateral:contralateral paw withdrawal latency (PWL) and statistically significant differences in PWL between sides are shown as * (p≤0.01 by Student's t test on raw PWL data).
   In (d), effects of (R)-CPP are shown on the mechanical allodynia that develops ipsilateral to CCI in wild type mice (open circles) compared to PSD-95 mutants (filled squares). Data are plotted as the mean (± SEM) ratio of ipsilateral:contralateral paw withdrawal threshold (PWT) and statistically significant differences in PWT between sides are shown as (* p≤0.01 by Mann-Whitney U-test). In no case was there any significant effect of (R)-CPP on contralateral responses compared to pre-operative values. (R)-CPP had no effect when administered to naive or sham-operated wild type or PSD-95 mutant mice (data not shown).
**Figure 21****: Immunoblots for phospho-Ser 897-NR1 and pan-NR1 in NR1 immunoprecipitates from wild type and PSD-95 mutant mice following CCI surgery**
   Western blot analysis of hemisected spinal cord immunoprecipitates following sciatic CCI induced 12 days previously. Blots from the spinal tissue from either two wild type or two PSD-95 mutant mice, ipsilateral or contralateral to the injury were probed with antibodies for pan-NR1 or phospho-Ser ⁸⁹⁷-NR1.
   Lane A, ipsilateral to injury in wild type, lane B, contralateral to injury in wild type. Lane C, ipsilateral to injury in PSD-95 mutant and D, contralateral to injury in PSD-95 mutant.

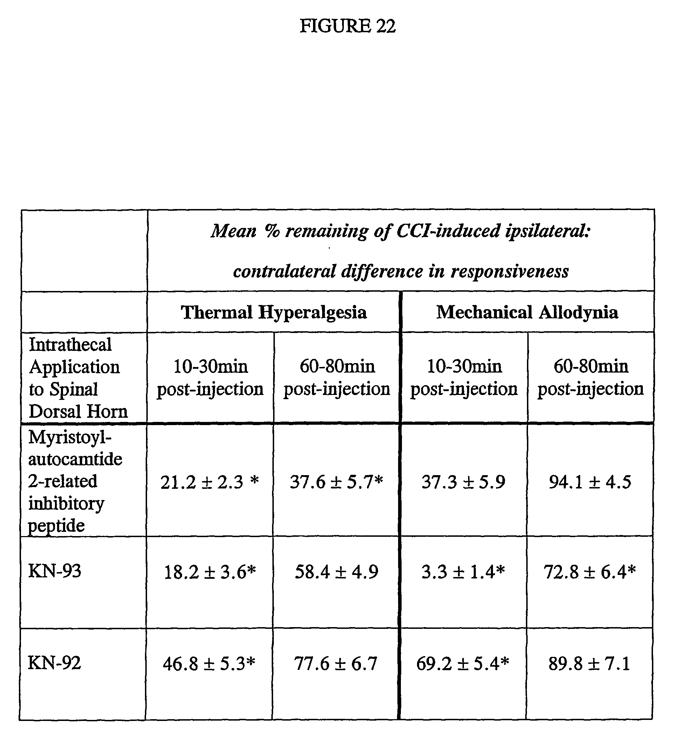

**Figure 22****: Effects of selective CaM kinase II inhibitors, on reflex neuropathic pain behaviours in wild type mice following CCI.**
   At the peak of neuropathic sensitisation following CCI, mice were intrathecally injected with myristoyl-autocamtide 2-related inhibitory peptide (1 n mole in 10 µl), K-93 (120 pmol in 10 µl) or the less active control analogue, KN-92 (120 pmol in 10 µl). n values were 7-9 in each case. Data for both thermal hyperalgesia and mechanical allodynia tests are plotted as the mean percentage reversal of the ipsilateral:contralateral difference in responses over two time windows following injection (10-30 min and 60-80 min). In each case the agents caused reduction of the sensitisation seen ipsilaterally with no significant change in any contralateral responses compared to pre-injection levels. In each case the diminution of ipsilateral sensitisation seen at 10-30 min (i.e. reduction of ipsilateral:contralateral difference) showed partial recovery by 60-80 min. *p≤0.05 by Wilcoxan test comparing pre- and post-injection response values.

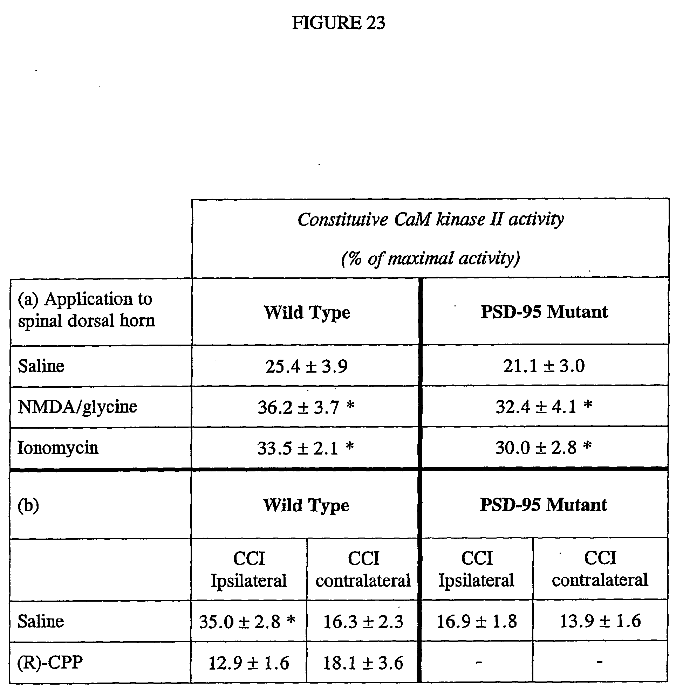

**Figure 23****: Effects of NMDA/glycine and CCI on constitutive activity of CaM kinase II in lumbar spinal cord of wild type and PSD-95 mutant mice.**
   Agents were topically applied for 15 min to the dorsal surface of L3-L6 spinal cord before rapid removal of tissue and homogenisation prior to CaM kinase II immunoprecipitation and kinase activity assay. The mean maximal CaM kinase II activity in immunoprecipitates from naive PSD-95 mutant mice spinal cord was unaltered from that in wild type mice (108.3 ± 13.7 and 97.8 ± 13.1 pmoles ³³P/min/µg original extract protein respectively). Data are expressed as the percentage of maximal CaM kinase II activity and are means ± SEM, n=4. Statistically significant differences are shown as *, p≤0.05 by Mann Whitney U test, compared to corresponding contralateral values in (b).
**Figure 24****:** Ischaemic neuronal damage (%) in the caudate nucleus following bilateral carotid artery occlusion of wildtype and PSD-95 mutant mice.

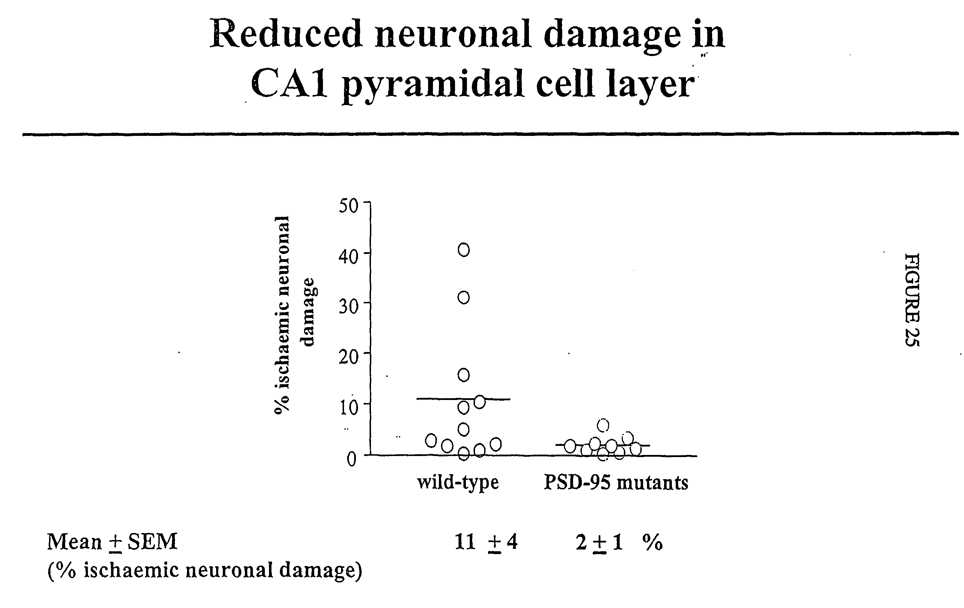

**Figure 25****:** Ischaemic neuronal damage (%) in the CA1 pyramidal cell layer of the hippocampus following bilateral carotid artery occlusion of wildtype and PSD-95 mutant mice.

### EXAMPLES

### EXAMPLE 1: ISOLATION OF A MULTI-PROTEIN COMPLEX COMPRISING AN NMDA RECEPTOR FROM MOUSE BRAIN

### Experimental Procedures

### Materials

Resins used in this study were Affigel-10 resin from BioRad (Hemel Hempstead, UK), PharmaLink resin and avidin agarose from Pierce (Chester, UK), Protein G-Sepharose from Pharmacia (Little Chalfont, UK) and Ni-NTA agarose from Qiagen (Crawley, UK). The multiple-antigen peptide (MAP) MAP-NR1 peptide consisted of the last 20 amino acids of NR1 ((H-RRAIEREEGQLQLCSRHRES)8-MAP) amino acid positions 919-938, the NR1 linear peptide (RRAIEREEGQLQLCSRHRES) 919-938, NR2A (EHLFYWKLR) 837-846, NR2A, B, C (SRGIYSC) 864-870 of NR2A or 925-931 of NR2B, NR2B-C terminus (KLSSIESDV) 1474-1482, NR2B-C terminus (SIESDV) 1477-1482 and NR2B (KAGNLYDISED) 1247-1257. All peptides were from Research Genetics (Huntsville, U.S.A.).

The residue positions for NR1, NR2A and NR2B are numbered according to the numbering system used in accession numbers P35438, P35436 and Q01097, respectively.

Antibodies used for Western blotting were mouse monoclonals 54.1 anti-NR1 from Dr. J.H. Morrison (Mt. Sinai School of Medicine, New York, U.S.A.), anti-PSD-95, anti-NR2B, anti-nNOS, anti-eNOS all from Transduction Laboratories (Lexington, U.S.A.), anti-actin was from Roche Molecular Biochemicals (Lewes, UK), and rabbit polyclonals anti-NR2A from Upstate Biotechnology (Lake Placid, U.S.A.), anti-ChapSyn was a gift from Dr. M. Watanabe (Hokkaido University School of Medicine, Sapporo, Japan) and anti-SynGAP from Dr R.L. Huganir (John Hopkins School of Medicine, Boston, U.S.A.).

Peroxidase-coupled secondary antibodies were from Amersham (Little Chalfont, UK). L-Asparagine, L-Glutamate and D-AP5 were from Fluka (Gillingham, UK), and Arcaine, Ifenprodil, NMDA, Dextromethorphan, Glycine, (+)-HA966 (3-amino-1-hydroxy-2-pyrrolidone) and ConantokinG were all from Sigma (Poole, UK). EZ-Link biotin hydrazide were from Pierce (Chester, UK). All other chemicals were of highest grade available.

### Antibody production and purification

Antibodies specific for the NR1 subunit were generated by injection of the MAP-NR1 peptide into sheep (Scottish Antibody Production Unit, Carluke, UK) resulting in the MAP-NR1 polyclonal IgG or by injection into rabbit (produced in house) to generate antibody 356. The antibody 138 against PSD-95 was obtained from rabbit as described earlier (Migaud, M., et al, 1998, Nature 396, 433-9). The MAP-NR1 antibodies were purified from serum by peptide affinity chromatography using a resin containing the linear NR1 N-terminally coupled peptide with the amino acid sequence as outlined above and the anti-NR1 356 and anti-PSD-95 IgG 138 were enriched by Protein G-Sepharose cleaning steps.

### Biotinylation of MAP-NR1 IgG

20 mg affinity-purified MAP-NR1 IgG was transferred into PBS and mixed with 20 mM cold sodium meta-periodate in PBS. Glycerol (23 µl) was added after an incubation for 30 minutes on ice in the dark. The sample was transferred into PBS after a further 5 minutes incubation on ice and EZ-Link biotin hydrazide was added to a final concentration of 5 mM followed by an incubation at room temperature for 2 hours. The biotinylated material was then dialysed in PBS, concentrated and stored at -20°C.

### Synthesis of ligand-affinity resins

Arcaine (8 mg), L-Asparagine (1 mg), L-Glutamine (2 mg), NMDA (2 mg), D-AP5 (2 mg), Glycine (2mg) and (+)-HA966 (1 mg) were solubilised and coupled to 0.5 ml affigel-10 in methanol for 24 hours at room temperature followed by addition of 150 mM Tris pH8.8 and a further incubation at room temperature for 24 hours to block any residual active side chains. ConantokinG (0.1 mg) was coupled to the same resin under identical conditions but substituting methanol with PBS. Ifenprodil (5 mg), D-AP5 (2 mg), Dextromethorphan (10 mg) and (+)-HA966 (2 mg) were immobilised onto 0.5 ml PharmaLink resin following the manufacturers instructions.

### Synthesis of immunoaffinity resins

The purified 356, 138 and MAP-NR1 antibodies were transferred into PBS by dialysis and coupled to affigel-10 in PBS at a concentration of 2 mg (356 and 138) or 5 mg (MAP-NR1) IgG per 1 ml of resin for 2 days at 4°C with continuous mixing. Unreacted side-chains were blocked by incubating the resin with 0.5 M Tris pH 7.5 at 4°C overnight.

### Synthesis of peptide resins

Peptides were solubilised and coupled to affigel-10 in methanol at 5 mg/ml resin for 3 days at 4°C with constant agitation and residual active side chains were blocked with 0.5 M Tris pH8.8 for 1 day at 4°C followed by transfer of the resin to PBS.

### Protein extraction

Whole wild-type mouse forebrains were homogenised on ice in either 1 % deoxycholate buffer (DOC buffer) consisting of 50 mM Tris pH9.0, 1 % sodium deoxycholate, 50 mM sodium fluoride, 20 µM zinc chloride, 1 mM sodium ortho-vanadate, 0.5 mM PMSF, 2 µg/ml Aprotinin and 2 µg/ml Leupeptin or an SDS containing buffer (SDS buffer) composed of 50 mM Tris pH8.0, 1 mM sodium ortho-vanadate, 150 mM NaCl, 1% Nonidet P-40, 0.5% sodium deoxycholate, 0.1 % sodium dodecylsulfate 1 mM EDTA, 0.5 mM PMSF, 2 µg/ml Aprotinin and 2 µg/ml Leupeptin at 0.38 g wet weight per 7 ml cold buffer. The extracts were clarified after an incubation of 1 hour on ice by centrifugation at 13.000xg for 30 min at 4°C followed by a 5 µm filtration step for small scale preparations or by centrifugation at 50.000xg for 30 min at 4°C for larger extractions.

### Immunoprecipitation

Small-scale experiments were done by mixing 0.2 ml extract with equal amount of the same extraction buffer and adding antibody as outlined in the Figures. 20 µl Protein G-Sepharose were added after an incubation of 2 hours at 4°C followed by a further incubation for 2 hours to overnight at 4°C with constant agitation. The resins were then washed with four cycles of 0.5 ml extraction buffer each and subjected to SDS-PAGE analysis. Large scale experiments were performed by pre-cleaning 50 ml mouse brain DOC extract with 0.5 ml Protein G-Sepharose for 2 hours at 4°C under constant agitation. The resin was then removed and 0.5 mg affinity-purified MAP-NR1 IgG was added to the supernatant. 0.5 ml Protein G-Sepharose was added after an incubation of 4 hours at 4°C with constant agitation followed by a further incubation period overnight at 4°C with constant agitation. The resin was then transferred into a chromatography column and washed with 100 ml DOC buffer to baseline. The protein was separated from the resin by boiling in 0.75 ml 4% SDS-gel sample buffer for 30 minutes and 20 µl aliquots were used for gel analysis and Western blotting.

### Immunoaffinity chromatography

Large scale experiments were done using 50 ml brain DOC extract and 0.5 ml affigel-10 immobilised anti-NR1 356 IgG, MAP-NR1 IgG or anti-PSD-95 138 antibody. Unbound material was removed after an overnight incubation at 4°C with constant agitation by column chromatography using approximately 100 ml DOC buffer until a stable baseline was obtained. The resin was then boiled in 0.75 ml 4% SDS-gel sample buffer for 30 minutes and 20 µl aliquots of the supernatant was used for SDS-PAGE and Western blotting analysis. Alternatively the proteins were eluted from the resin with 6M urea or a pH shift to pH12 with 50 % DOC buffer.

### Ligand affinity chromatography

25 µl of the derivatised ligand affinity resins, as described above, were added to 0.2 ml mouse brain DOC extracts and incubated for 2 hours at 4°C with constant agitation. The resins were then washed five times with 0.5 ml DOC buffer each and resuspended in 25 µl of SDS-PAGE sample buffer, followed by SDS-PAGE analysis and Western blotting.

### Peptide affinity chromatography

Initial small-scale experiments were done using 0.5 ml brain DOC extract and 25 µl affige1-10 immobilised peptides. The resins were washed four times with 0.5 ml cold DOC buffer after an incubation period of four hours at 4°C with constant agitation followed by SDS-PAGE analysis. Large scale samples were prepared by adding 0.5 ml NR2B 1477-1482 peptide derivatised affigel-10 with 50 ml DOC extract followed by an incubation overnight at 4°C with constant agitation. The resin was then transferred into a chromatography column and washed to baseline with approximately 100 ml cold DOC buffer. Bound proteins were released from the resin by boiling for 30 minutes in 4% SDS-containing buffer and 20 µl aliquots were subsequently used for SDS-PAGE analysis and Western blotting (see Example 2).

### Western blotting

Protein samples were subjected to reducing SDS-PAGE and transferred to PVDF membrane (BioRad) at 4°C for 90 minutes at 75 V in 10% (v/v) Methanol, 10 mM CAPS pH 11.0. Dilution of primary antibodies was between 1:100 and 1:1000 depending on the quality of the IgGs. Detection of signals was done using peroxidase-linked secondary IgGs and enhanced chemiluminescence.

### Results

### Solubilisation

The NMDA receptor as part of the post-synaptic density is well known for its extraction difficulty, which has to involve high quantities of detergent as a prerequisite. An initial study was carried out to determine the behaviour of the NMDA receptor and attached molecules in two buffer systems as shown in Figure 1. Extraction of mouse brain using DOC or SDS buffer resulted in a slightly higher amount of NR1 immunoreactivity in the extracts using SDS containing buffer, which is also evident in the immunoprecipitated material. However, there is a clear reduction of NR2A and PSD-95 attached to this NR1-precipitated material in the SDS buffer based approach compared to the DOC extraction despite the amounts of NR2A and PSD-95 which were similar in whole extracts (results not shown). An alternative method based on 1.5% (w/v) CHAPS as the sole detergent did not result in quantitative extraction of a multi-protein complex comprising an NMDA receptor (results not shown) though it was reported to solubilise glutamate receptors with NMDA affinity from rat brain synaptosomes (Kumar et al, 1991, Nature 354, 70-73). It was also tested whether membrane preparations of mouse brain as starting material will result in a different NR1-affinity pattern and they yielded a very similar result as obtained using whole brain extracts (data not shown).

### Immunoprecipitation

Successful isolations of multi-protein complexes all included the use of specific antibodies against various epitopes of the channel, though these studies were done on low levels of protein amount and analysed by Western blotting only. We therefore tried to enrich the isolated material for analysis by gel staining which could give an indication of the complexity of such extracted material. The IgGs chosen for this approach were directed against the C-terminus of NR1 (antibodies 356 and MAP-NR1) and against PSD-95 (138) as shown in Figure 2. All IgGs were capable of binding and isolating the NMDA receptor and PSD-95 as visualised by Western blotting, though gel analysis did show that the levels of purified material were too low. The gel in Figure 2 shows that by using the antibody 356 no bands were visible apart from the IgG heavy chain, whereas the MAP-NR1 antibody revealed several diffuse bands at 90 and 140 kDa and some stronger bands in the region of 42 kDa. Two bands at around 90-100 kDa were clearly visible using the anti-PSD-95 IgG 138, of which one is presumably PSD-95. The same lane also shows some weaker bands between the IgG heavy chain and 200 kDa, which quite likely could be the NMDA receptor and associated molecules.

The most promising IgG was the MAP-NR1, based on the Western blotting results, which was therefore purified and used for titration experiments as shown in Figure 3, panel A. Small scale immunoprecipitations were done in the absence (0 µg) and the presence of MAP-NR1 IgG (0.1 - 30 µg). This allowed us to distinguish between contaminants and the isolated multi-protein complex, since only the latter increased in band strength upon higher amounts of MAP-NR1 used. Furthermore it was of interest to find an optimal ratio of extract to antibody for later scale-up preparations. The control lane shows the experiment of IgG alone without the extract added in order to visualise any bands derived from the IgG preparation or the resin. Comparing the lane with 30 µg IgG with a lane using less than 1 µg antibody shows that a number of protein bands are enriched, ranging from 45 kDa at the bottom of the gel up to 200 kDa. It is clearly visible in the acrylamide gel analysis that antibodies and other molecules present in the starting material with an affinity for Protein G-Sepharose gave rise to background bands (lane 0 µg MAP-NR1 IgG), which can make a distinction between specific multi-protein complex components and unspecific proteins difficult. Therefore another approach of using a modified IgG was tried as shown in Figure 3B. The MAP-NR1 IgG was biotinylated and the multi-protein complex isolated with avidin-agarose. Again, some unspecific bands could be observed in all lanes, namely a double band at around 75 kDa and a strong band at 120 kDa. The control lane shows that none of these molecules are present in the IgG preparation or are from the avidin-agarose. However it is obvious that the biotinylated MAP-NR1 bound a much less complex protein pool compared to the unmodified IgG. At this stage it is not clear if this is due to an isolation of a subpopulation of NMDA receptors or the introduction of steric constraints based on biotinylation like steric hinderance. The latter should not be an issue since the biotin-tag was attached to the glycosylated Fc-region, which should allow a similar free molecular movement like the Protein-G bound MAP-NR1. However it is clearly visible on the Coomassie stained gels that the overall amount of IgG heavy chain is reduced for the biotin-labelled MAP-NR1 compared to the unmodified IgG. It can not be excluded that some of the material was not released from the avidin-agarose even after a boiling step, and maybe some multi-protein complex components were also not released.

### Immunoaffinity

As a next step we tested if it was possible to use the antibodies mentioned above by directly immobilising them to a solid support and to try to elute the isolated material either by a simple pH shift from pH9.0 to pH12 or under denaturing conditions using 6 M urea as shown in Figure 4. Elution of bound proteins by urea from the anti-NR1 affigel (356 - urea) shows a very complex protein band pattern, which, analysed by Western blotting, demonstrates the presence of both NR1 and NR2A as well as PSD-95. A similar result is obtained using bound anti-PSD-95 IgG (138 - urea), but here the SDS-PAGE band pattern is less pronounced. Not surprisingly there is a higher quantity of PSD-95 present in this preparation compared to the 356-resin, though the amount of NR2A is slightly reduced. It is a possibility that the band visible at around 90 kDa in the 138 - urea sample is PSD-95. Furthermore it is evident from the gels that a considerable amount of IgG is also released from the resin which can be seen by the major band at 50 kDa. This break-off of the antibody is reduced by pH12 elution, though the protein band pattern on the gels is also changed for the 356-affigel, but only marginally for the 138-resin. The Western blotting signals were also almost identical, though slightly reduced for the anti-PSD-affigel. The 356-resin eluted by pH12 was completely altered in respect of PSD-95, which was absent in this protein sample. However, both NR1 and NR2B could be eluted in this manner.

### Ligand affinity

Ligand affinity based methods were used originally to isolate the NMDA receptor prior to the cloning strategies, which revealed that the isolated proteins belonged to a different class of Glutamate receptors, although displaying a similar affinity for drugs as the NMDA receptor (E.K. Michaelis, 1994, In: *Cirrhosis, Hyperammonemia, and Hepatic Encephalopathy*, S. Grisolia and V. Felipo (eds.), Plenum Press, New York, 119-128). However it was not examined if the NMDA receptor was co-purified in their preparations due to the lack of subunit specific antibodies at the time. We investigated if it was possible to isolate the NMDA receptors and attached molecules using various immobilised ligands directed at different known interaction sites on the channel as shown in Figure 5.

Drugs tested in this study which are known to interact with the polyamine site of the NMDA receptor were Arcaine, Ifenprodil and the peptide ConantokinG. A substantial amount of protein did interact with immobilised Arcaine, including NR1, NR2B and PSD-95, and the strongest bands visible on the gel were 35, 40 and 50 kDa in size, which are all currently unknown. Ifenprodil coupled to PharmaLink resin was also capable of binding the multi-protein complex and many other molecules, though to a slightly lower degree than the Arcaine resin. This is also reflected in the signal intensities of the Western blotting results of these two experiments. However, the ConantokinG resin did not result in any band visible on the gel, and Western analysis showed only trace amounts of NR1 and NR2B and no PSD-95.

We also tested if ligands directed against the Glutamate-binding site could be used for the isolation of the multi-protein complex. L-Glutamate as well as NMDA resins showed weak binding to only two molecules at 40 and 50 kDa, and the Western blotting results showed that only marginal quantities of the multi-protein complex were retained on these resins. The L-Asparagine affigel-10 support and PharmaLink coupled D-AP5 both bound to an array of protein species, with the major bands again at 40 and 50 kDa. Western blot analysis of NR1 and PSD-95 were very similar in signal strength, whereas the NR2B signal was very strong for L-Asparagine-isolated samples compared to a faint signal for D-AP5. The affigel-10 bound drug D-AP5 was ineffective in the isolation of any protein.

The open channel blocker Dextromethorphan was efficient in binding several proteins as seen on the Coomassie-stained gel. Western blotting of this sample revealed that only marginal amounts of both NR1 and NR2B were present, but surprisingly a comparatively large amount of PSD-95.

Glycine is an essential amino acid which is necessary for proper NMDA receptor channel function. However, immobilised Glycine bound only very weakly to a 50 kDa protein, and the three molecules probed for in Western analysis were all absent. This is in contrast with the Glycine-site modulator HA-966 coupled to either PharmaLink or affigel-10 resins. In both instances the results were identical, both did show a slightly more complex pattern on SDS-PAGE, and were found positive for NMDA receptor channel components and the attached PSD-95. It is noteworthy to notice that the NR1 was specifically enriched compared to other ligand-based methods.

NMDA receptor function is known to be modulated by Zn²⁺, and sequence analysis of known multi-protein complex components showed the presence of Histidine-repeats. Therefore we investigated if we can retain the channel by using chelated Ni²⁺ (lane Ni-NTA). The Coomassie stained gel showed essentially only two bands at 40 and 50 kDa, but surprisingly the Western blot analysis revealed the presence of NR1, NR2B and PSD-95 arguing for an intact NMDA receptor channel presence.

However, it was evident from these ligand-based purification methods that the main proteins retained on most resins are the 40 and 50 kDa protein species, both of which are currently unknown. It is a possibility that these proteins are components of the identified and characterised Glutamate receptor as it is known that those proteins possess affinities towards drugs which also bind and modulate the NMDA receptor (Kumar, K.N., Tilakaratne, N., Johnson, P.S., Allen, A.E. & Michaelis E.K., 1991, Nature 354, 70-73; Ikin, A.F., Kloog, Y. & Sokolovsky, M., 1990, Biochemistry 29, 2290-2295).

### Peptide affinity

Antibody-based isolation strategies have the disadvantage of containing usually high quantities of added IgG in the final samples, which could interfere with the analysis in many ways. Therefore we were looking for other ways to isolate and enrich the multi-protein complex or subcomponents of it. One way would be to use portions or domains of proteins like a PDZ-domain which can bind to C-terminal sequences like in the case of the NR2 - PSD-95 interaction, or to use peptides which can bind to target domains or be used as substrates. Figure 6 shows the ability of several peptides of the NMDA receptor channel subunits to acts as matrices for binding partners. We chose the NR1 C-terminal peptide which we used previously to generate and purify the MAP-NR1 IgG since it was unclear whether this region is also used for binding sites of other molecules which could interfere in the IgG-based multi-protein complex purification protocol. The other peptides NR2A (837-846), NR2A, B, C and NR2B (1247-1257) were selected due to the tyrosine-residues which might act as tethering points for tyrosine kinases. The NR2B C-terminal peptides NR2B (1474-1482) and (1477-1482) comprise the known binding sequence for PDZ-domain containing proteins like PSD-95. All the peptide-resins showed an interaction with proteins of 45 and 55 kDa plus some additional minor bands above 60 kDa. This pattern is not observed using Tris-blocked affigel-10, which means that these molecules are interacting with the peptide-resins either specifically due to the selected peptides or in an unspecific way due to charge-relations of the resins not obtained by Tris-blocked affigel. None of the peptide resins show any particular enrichment apart from the NR2B (1477-1482) peptide-affigel, which revealed several bands at 75 and 100 kDa. Surprisingly the immobilised NR2B C-terminal peptide NR2B (1474-1482) did not show any binding pattern similar to the slightly shorter one (NR2B 1477-1482), though the reason is as yet unknown. This enriched 100 kDa band can be accounted for the binding of the synapse-associated protein (SAP) molecules PSD-95, ChapSyn110, SAP97 and SAP102, which are all well described to bind to the C-termini of NR2, and which have all very similar molecular masses and therefore migrate very close together on SDS-PAGE. However, the band observed at 75 kDa can not be assigned to any protein at this stage and it remains to be identified. Nevertheless we could identify another way of isolating proteins which could be part of the larger network comprising the NMDA receptor channel and other biomolecules using an NR2B C-terminal peptide immobilised on a solid support.

### Comparative analysis of three isolation methods

Figure 7 shows large-scale isolations of the multi-protein complex by immunoprecipitation using the MAP-NR1 antibody, immunoaffinity chromatography with affigel-10 immobilised MAP-NR1 IgG and NR2B (1477-182) peptide affinity chromatography of mouse brain extracts, analysed both by Coomassie stained SDS-PAGE and Western blotting. Both IgG approaches show huge quantities of IgG heavy (50 kDa) and light chain (25 kDa) on the stained gel, which will interfere with the interpretation of proteins migrating in those areas. This is demonstrated by the absence of the band at 55 kDa in the peptide-based strategy, which is either not present or obscured by the heavy chain of the antibody in the other two methods. Additionally the immobilised IgG matrix shows also a strong band at 80 kDa, which is slightly weaker in the immunoprecipitated sample and even fainter in the peptide-based method. This band, or a fraction of it, could be assigned by Western blot analysis as immunoglobulin, IgH or cross-linked IgG, in both the antibody-based strategies, but not in the case for the peptide-resin.

In order to identify whether selected molecules are present in these three samples, we proceeded in analysing them using Western blotting as shown in the lower section of Figure 7 and as described in detail in Example 2.

### Discussion

The isolation of the NMDA receptor was achieved in the past using immunoprecipitation methods, though only on low levels which hampered further characterisation and in-depth analysis. Standard purification approaches like ion-exchange chromatography are not feasible due to the incorporation of charged detergents like 1% deoxycholate, which is an absolute requirement for a quantitative solubilisation of the multi-protein complex. Partial substitution of deoxycholate by SDS does achieve a higher solubilisation degree, though partially disrupting the attachment of NMDA receptor binding partners and at least to some extent a different solubilisation pattern for NR1/NR2 complexes. An alternative choice of 1% Triton X-100 as the main detergent would be suitable for chromatographic means, however it was reported that this method does not solubilise NR2 containing receptors (Chazot, P.L. & Stephenson, F.A., 1997, J. Neurochem. 68, 507-516). It is also a possibility that the isolated components tend to aggregate in the SDS or Triton X-100 containing buffer system and therefore either epitopes could not be accessible for antibody recognition or precipitation of parts of the multi-protein complex resulted in an accidentally depleted subfraction of the whole multi-protein complex pool. This is actually supported by analysing the solubility of isolated material by elution from columns. Obviously at least PSD-95 displays an interesting behaviour based on pH shifts, where part of this molecule population is retained on the resin at pH12. It is not known which subpopulation undergoes such a hydrophobicity shift but it can be concluded that the presence of the NMDA receptor must be a key element. It is however not known how the choice of detergent can influence the extractability and solubility of the multi-protein complex based on elevated temperatures since all experiments in this study were done at 4°C.

Ligand-affinity based methods such as immobilised drugs or amino acids, which were used in the past for the isolation of glutamate-binding proteins including the NMDA-like receptor (Kumar, K.N., Tilakaratne, N., Johnson, P.S., Allen, A.E. & Michaelis E.K., 1991, Nature 354, 70-73), could also be shown in this study to be capable of binding the multi-protein complex. Surprisingly the resins used in the past by other investigators, like immobilised glutamate, showed low affinity for the multi-protein complex. Unfortunately most of the promising drug-matrices suitable for isolation purposes, like Arcaine-affigel and HA-966 PharmaLink or HA-966 affigel, can be expected to bind to other ionotropic glutamate receptors as well as other proteins since these drugs are known to have a higher affinity for NMDA channels, but can also bind and modulate channels as Kainate and AMPA receptors.

We also investigated if peptides could be used for the isolation of the multi-protein complex. The NR1 C-terminal peptide showed no evident binding pattern and therefore it can be assumed that this site is not used as a binding domain for other proteins and the isolation of the multi-protein complex by MAP-NR1 IgG methods will not perturb NR1-attached molecules. Other peptides from the internal sequences of NR2A, B and C did not yield any observable enriched protein pattern based on SDS-PAGE analysis. This could also be interpreted that perhaps only molecules present in low abundance display an affinity for these peptides and could therefore not be visualised. However, it is somewhat surprising that the NR2B (1477-1482) peptide resin was capable of not only purifying PSD-95 and other members of this family, but also the NMDA receptor itself. This could be interpreted that within the postsynaptic density there are more NR2 anchorage points available than actually used by the receptor, and that there might exist a link between the SAP molecules. This view is supported by the finding that SAP proteins including PSD-95 and ChapSyn110 can multimerise with each other through a N-terminal disulfide-bridge. To our surprise we could not detect any enrichment of brain proteins using a 3 amino acid extended version of the successful NR2B (1477-1482) peptide. This could point out that the longer peptide adopts a conformation that is not capable of binding to a PDZ-domain and might have to undergo a modification step in order to become accessible. Such a modification could be a phosphorylation on one of the two serine residues within the sequence. Currently it is not known if *in vivo* NR2B is phosphorylated in this region in order to become accessible to PDZ-domain proteins.

A comparative analysis of the most successful methods of immunoprecipitation, immunoaffinity and peptide affinity strategies presented in this study showed that the multi-protein complex is composed of a large number of proteins. It is remarkable how all three methods gave similar results by Western blot analysis, though the gel band pattern differed, mainly because two of the strategies included IgGs which are interfering with gel analysis and band recognition. An alternative of using Protein-G based immunoprecipitation could be demonstrated in this study by using biotinylated antibody and avidin agarose precipitations.

### EXAMPLE 2: CHARACTERISATION OF MULTI-PROTEIN COMPLEXES

### Materials and Methods

### Antibodies

Affinity purified NR1 specific sheep polyclonal antibodies (MAP-NR1) were generated using a multiple-antigen peptide (MAP) of the last 20 amino acids of NR1 ((H-RRAIEREEGQLQLCSRHRES)8-MAP) (Scottish Antibody Production Unit, Carluke, UK). Other antibodies were obtained from sources described in Table 3.

### Purification of the NMDA receptor complex

The receptor complex was isolated from mouse forebrain extracts using either covalently-coupled MAP-NR1 immunoaffinity resins, immunoprecipitation with the same antibody, or peptide-affinity chromatography with a hexapeptide of the NMDA-R2B C-terminus (SIESDV), as described in Example 1. In brief, samples were homogenized in 1% (w/v) deoxycholate containing buffer at pH 9.0, spun for 30 minutes at 50.000xg at 4°C, followed either by incubation with MAP-NR1 antibody and subsequent Protein G-Sepharose precipitation, or by immunoaffinity chromatography using MAP-NR1 antibody substituted affigel-10 (BioRad)(5 mg antibody/ml resin), or the NR2B peptide resin (5 mg peptide/ml affigel-10 resin). The resins were washed after an overnight incubation at 4°C with 100 to 1000 column volumes of extraction buffer at 4°C, and proteins were separated from the resin by boiling in 4% SDS for 30 minutes.

### Western blotting

### (i) Transfer of protein

- Buffer composition:: 10 mM CAPS pH11.0 @ 4°C
10% (v/v) Methanol

- Soak PVDF membrane in Methanol for 1 minute, then transfer into buffer
- Incubate 2 filter papers, membrane and 2 foam pads in buffer for 10 man
- Incubate the gel in buffer for approximately 5 min
- Assemble gel sandwich into cassette in a tray containing enough buffer to cover the assembled sandwich in the following order (bottom to top):
   Black side of cassette
   Foam pad
   Filter paper
   Gel
   PVDF membrane
   Filter paper
   Foam pad
   Clear side of cassette
- Ensure that there are no air bubbles trapped between the layers of the sandwich
- Clamp the sandwich and insert into the gel transfer apparatus filled with buffer
- Transfer proteins for 90 min at 4°C at 75 V with gentle stirring to avoid trapping of bubbles
- Disassemble sandwich and transfer PVDF membrane protein face up into a container containing PBS

### (ii) Western blotting detection

- Buffer: PBS containing 0.05% (v/v) Tween 20

- Block PVDF membrane with buffer containing 5% non-fat dried milk powder overnight at 4°C under constant agitation
- Wash membrane three times with buffer for 5 min each
- Incubate membrane with buffer containing diluted primary antibody (1:100 to 1:5000, needs to be estimated separately for each antibody) for 2 h at room temperature with constant agitation
- Wash membrane three times with buffer for 5 to 10 min each
- Incubate membrane with buffer containing diluted secondary antibody (1:1000 to 1:5000, needs to be estimated separately for each antibody) for 2 h at room temperature with constant agitation
- Wash membrane for 5, 5 and 30 minutes with buffer
- Incubate membrane with ECL detection reagents (Amersham Pharmacia) and expose to x-ray film for 1 sec up to 5 min

Samples were subjected to SDS-PAGE and transferred to PVDF membrane at 4°C for 90 minutes at 75 V in 10% (v/v) Methanol, 10mM CAPS pH 11.0. Dilution of primary antibodies was between 1:100 and 1:1000 depending on the quality of the IgG. Signals were detected using peroxidase-linked secondary IgGs and enhanced chemiluminescence.

### Mass Spectrometry sample preparation

Multi-protein complex samples were separated by SDS-PAGE, stained by Coomassie blue or silver staining (Shevchenko, A., Wilm, M., Vorm, O. & Mann, M., 1996, Anal Chem 68, 850-8), and individual protein bands between 60-300kD were excised, reduced, alkylated and digested with trypsin (Wilm, M. et al., 1996, Nature 379, 466-9). The resultant peptide mixtures were analysed first by Delayed Extraction Matrix Assisted Laser Desorption Ionisation (DE-MALDI) Time of Flight (TOF) mass spectrometry to produce peptide mass fingerprints (Jensen, O. N., Podtelejnikov, A. V. & Mann, M, 1997, Anal Chem 69, 4741-50) and subsequently by on-line Liquid Chromatography Tandem Mass spectrometry (LC/MS/MS) to generate peptide sequence information (Link, A. J. et al., 1999, Nat Biotechnol 17, 676-82).

### Analysis by DE-MALDI

The MALDI mass spectrum was obtained using a TofSpec SE instrument (Micromass, Manchester, UK) fitted with a 337 nm nitrogen laser. Individual mass spectra were calibrated via a lock mass routine using a trypsin autolysis peptide at m/z 2163.0570. Monoisotopic peptide masses were assigned and used to search an in house non-redundant protein sequence database (nrpep) using the PepSea program (Protana, Denmark). No restriction was placed on the species of origin of the protein, on its isoelectric point, and a protein mass range from 0 to 300 kDa was allowed. In order for the protein(s) to be unambiguously identified, at least 5 peptides, representing a minimum of 15 % sequence coverage, were matched within a mass accuracy of 50ppm (Jensen, O. N., Podtelejnikov, A. V. & Mann, M, 1997, Anal Chem 69, 4741-50).

### Online LC-MS/MS analysis

Chromatographic separations of the peptide mixture were performed on a 180 µm PepMap column using an Ultimate LC system (LC Packings, The Netherlands) delivering a gradient to formic acid (0.05%) and acetonitrile. The eluting peptides were ionised by electrospray ionisation on a Q-TOF hybrid mass spectrometer (Micromass, U.K.) fitted with a Z-spray source. The instrument is set to operate in automated function switching mode, whereby precursor ions are selected based on intensity for peptide sequencing by collision induced fragmentation tandem MS. The MS/MS analyses were conducted using collision energy profiles that were chosen based on the m/z of the precursor under analysis; a total of nine MS/MS scans were acquired for each precursor. Several hundred MS/MS spectra were generated per run allowing the analysis of complex mixtures without any prior interpretation. The mass spectral data was processed into peak lists containing m/z value, charge state of the parent ion, fragment ion masses and intensities, and correlated with proteins and nucleic acid sequence databases using Mascot software (Matrix Science, U.K.). Proteins were identified based on matching the MS/MS data with mass values calculated for selected ion series of a peptide. The searches on a non-redundant protein database, and dbEST, a nucleotide database containing Expressed Sequence Tags from a number of organisms that is compiled by NCBI/EBI (National Centre for Biotechnology Information/European Bioinformatics Institute, UK), were performed without applying any constraints on molecular weight or species of origin. Most proteins were identified with several peptide matches although a few proteins were assigned on the basis of a single peptide provided near complete peptide sequence had been obtained.

### Results and Discussion

### Table 3. Summary of molecular composition of the multi-protein complex.

Immunoblotting screen of multi-protein complex and known binding partners within multi-protein complexes.
Columns: Protein, classes of proteins are boxed and specific molecular names are indicated with identifying numbers (1-106); Mr[kDa], relative molecular mass; Ex, mouse brain extracts; IA, complexes isolated by MAP-NR1 immunoaffinity; IP, complexes isolated by MAP-NR1 immunoprecipitation; Pep, complexes isolated by NR2B peptide affinity; Ab, antibody source; Binding partner, identified interactions from published in vitro studies.
Specific brain proteins where assayed in three preparations of complexes (IA, IP, Pep) using immunoblotting and detectable signals were scored as strong (+ + +), medium (+ +) and weak (+) signal and undetectable signals (-). Some proteins could not be analysed in IA or IP because of comigration of Ig (ch, comigration heavy chain Ig; cl, comigration light chain Ig).
For each protein found in the complexes the reported associated proteins are indicated (binding partner) by reference to the numbering scheme in the first column.
Antibodies source (column Ab): a, J.H. Morrison; b, Upstate Biotech; c, Chemicon; d, Pharmingen; e, R. Huganir; f, Transduction Labs; g, M. Watanabe; h, Alomone Labs; i, H. Kreienkamp; j, K. Inokuchi; k, E. Ziff; 1, J. Scott; m, J. Henley; n, Promega; o, P. Cohen; p, Oncogene Sci; q, New England Biolabs; r, Santa Cruz; s, Calbiochem; t, D. Kuhl; u, D. Coleman; v, Sigma; w, Roche Molecular Biochemicals; x, J. Parsons.

### Table 4. Summary of Mass Spectrometry analysis of multi-protein complex proteins.

Visualised protein bands between 60-300kD were prepared and analysed as described (see above and Figure 9). Columns: Protein name; Mr[kDa], relative molecular mass; Accession number; MS events, Matching peptides sequenced by MS/MS.

**TABLE 3**

| Protein | | Mr[kDa] | Ex | IA | IP | Pep | Ab | binding partner |
|---|---|---|---|---|---|---|---|---|
| **Glutamate receptors** | | | | | | | | |
| 1 | NR1 | 120 | +++ | +++ | +++ | +++ | a b | 2, 3, 18, 35, 68, 95, 96, 101 |
| 2 | NR2A | 180 | +++ | +++ | +++ | +++ | | 1, 3, 10, 11, 12, 35, 73, 36 |
| 3 | NR2B | 180 | +++ | +++ | +++ | ++-+ | c | 1, 2, 10, 11, 12, 35, 43, 95, 73, 96, 101 |
| 4 | GluR1 | 108 | +++ | - | - | - | d | |
| 5 | GluR2 | 102 | +++ | - | - | - | d | |
| 6 | GluR2/3 | 102 | +++ | - | - | - | e | |
| 7 | GluR4 | 108 | +++ | - | - | - | d | |
| 8 | GluR 6 + 7 | 117 | +++ | + | + | + | b | 10, 12 |
| 9 | mGluR1α | 200 | ++ | ++ | ++ | + | d | 15, 68, 73, 101 |

| **Scaffolding and adaptors** | | | | | | | | |
|---|---|---|---|---|---|---|---|---|
| 10 | PSD-95 | 95 | +++ | +++ | +++ | +++ | f | 2, 3, 8, 10, 11, 12, 13, 65, 59, 75 |
| 11 | Chapsyn-110/PSD-93 | 110 | +++ | +++ | +++ | +++ | g | 2, 3, 10, 69 |
| 12 | Sap102 | 115 | + | - | - | +++ | h | 2, 3, 8, 10, 13, 65, 68 |
| 13 | GKAP / SAPAP | 95-140 | +++ | +++ | ++ | +++ | e | 10, 12, 14 13, 15, 102 |
| 14 | Shank | 200 | + | ++ | ++ | ++ | i | 9, 15, 14 |
| 15 | Homer | 28/45 | +++ | ++ | ++ | ++ | j | |
| 16 | GRIP | 120 | +++ | - | - | - | e | |
| 17 | ABP (GRIP2) | 95-130 | + | - | - | - | k | |
| 18 | Yotiao | 200 | ++ | ++ | ++ | ++ | l | 1, 25, 37 |
| 19 | AKAP150 | 150 | +++ | +++ | +++ | +++ | l | 25, 26-34. 39, 68 |
| 20 | NSF | 83 | +++ | ++ | ++ | +++ | m | 5, 20 |

| **PKA** | | | | | | | | 18, 19, 92 |
|---|---|---|---|---|---|---|---|---|
| 21 | PKA catalytic subunit | 40 | +++ | ++ | ++ | ++ | f | |
| 22 | PKA-R1α and β | 48 | +++ | ch | ch | - | f | |
| 23 | PKA-R1a-α | 49 | +++ | ch | ch | - | f | |
| 24 | PKA-R2α | 51 | ++ | ch | ch | - | f | |
| 25 | PKA-R2β | 53 | +++ | ++ | ++ | ++ | f | |

| **PKC** | | | | | | | | 19, 105 |
|---|---|---|---|---|---|---|---|---|
| 26 | PKC α | 82 | +++ | - | - | - | f | |
| 27 | PKC β | 80 | +++ | ++ | ++ | ++ | f | |
| 28 | PKC γ | 80 | +++ | +++ | +++ | +++ | f | |
| 29 | PKC δ | 78 | ++ | - | - | - | f | |
| 30 | PKC ε | 90 | +++ | ++ | ++ | ++ | f | |
| 31 | PKC η | 82 | ++ | - | - | - | f | |
| 32 | PKC θ | 79 | ++ | - | - | - | f | |
| 33 | PKC τ | 74 | ++ | - | - | - | f | |
| 34 | PKC λ | 74 | ++ | - | - | - | f | |

| **CaM Kinase** | | | | | | | | 1, 2, 3, 65, 68, 69 |
|---|---|---|---|---|---|---|---|---|
| 35 | CaM Kinase II β | 60 | +++ | +++ | +++ | +++ | f | |
| 36 | phospho-CaM Kinase II | 60 | +++ | ++ | ++ | ++ | n | |

| **Phosphatases** | | | | | | | | |
|---|---|---|---|---|---|---|---|---|
| 37 | PP1 | 36 | +++ | +++ | +++ | +++ | f | 18 |
| 38 | PP2A | 36 | +++ | +++ | +++ | + | f | |
| 39 | PP2B (calcineurin) | 61 | +++ | + | + | + | f | 19 |
| 40 | PP5 | 50 | +++ | ++ | ++ | ++ | o | |
| 41 | PTP1B | 50 | + | ch | - | - | b | |
| 42 | PTP1C | 68 | +++ | - | - | - | b | |
| 43 | PTP1D | 72 | +++ | ++ | ++ | ++ + | f | 3 |

| **Tyrosine Kinases** | | | | | | | | |
|---|---|---|---|---|---|---|---|---|
| 44 | Src | 60 | +++ | + | + | + | p | 47, 59, 60, 90, 101 |
| 45 | Fyn | 59 | ++ | - | - | - | f | |
| 46 | FAK | 125 | ++ | - | - | - | f | |
| 47 | PYK2 | 116 | +++ | + | + | + | f | 44 |

| **MAP Kinase pathway** | | | | | | | | |
|---|---|---|---|---|---|---|---|---|
| 48 | ERK (pan ERK) | 42/44 | +++ | ++ | ++ | ++ | f | |
| 49 | ERK1 | 42/44 | +++ | ++ | ++ | ++ | f | |
| 50 | ERK2 | 42 | +++ | ++ | ++ | ++ | f | 57 |
| 51 | ERK3 | 62 | ++ | - | - | - | f | |
| 52 | phospho-ERK1/2 | 42/44 | ++ | - | - | - | q | |
| 53 | MEK1 | 45 | +++ | ++ | ++ | + | f | 59 |
| 54 | MEK2 | 46 | +++ | ++ | ++ | + | f | 59 |
| 55 | MKP2 | 43 | + | ++ | ++ | + | f | |
| 56 | JNKK1/MKK4 | 44 | + | - | - | - | f | |
| 57 | Rsk | 90 | +++ | ++ | ++ | ++ | f | 50 |
| 58 | Rsk-2 | 90 | +++ | ++ | +++ | + | f | 50 |
| 59 | c-Raf1 | 74 | +++ | ++ | ++ | + | f | 44, 53-54, 60 |

| **Small G-proteins and modulators** | | | | | | | | |
|---|---|---|---|---|---|---|---|---|
| 60 | H-Ras | 21 | +++ | cl | cl | + | f | 44, 59, 65, 66, 71 |
| 61 | Rac1 | 21 | +++ | ++ | + | + | f | 75, 101, 102 |
| 62 | Rap1 | 21 | + | - | - | - | f | |
| 63 | Rap2 | 21 | +++ | ++ | ++ | + | f | |
| 64 | RalA | 24 | +++ | cl | cl | + | f | |
| 65 | SynGAP | 150 | +++ | ++ | ++ | ++ | e | 10, 12, 35, 60 |
| 66 | NF1 | 250 | +++ | + | + | + | r | 60, 101 |
| 67 | p120GAP | 120 | ++ | - | - | - | f | |

| **Other signalling molecules** | | | | | | | | |
|---|---|---|---|---|---|---|---|---|
| 68 | Calmodulin | 15 | +++ | +++ | +++ | +++ | b | 1, 9, 12, 19, 35, 69, 92 |
| 69 | nNOS | 155 | ++ | + | + | + | f | 10, 11, 35, 68 |
| 70 | eNOS | 140 | ++ | - | - | - | f | |
| 71 | PI3 Kinase | 85 | +++ | + | + | + | f | 60, 101 |
| 72 | Calpain | 30 | + | - | - | - | s | |
| 73 | PLCγ | 150 | ++ | + | + | + | r | 2, 3, 9, 94, 101 |
| 74 | cPLA2 | 110 | ++ | ++ | + | + | r | |
| 75 | Citron | 183 | ++ | ++ | ++ | + | r | 10, 61 |
| 76 | VAV | 95 | ++ | - | - | - | f | |
| 77 | arg3.1 | 55 | + | ++ | ++ | +++ | t | |
| 78 | CREB/CREM | 46/26 | - | ch | ch | - | f | |
| 79 | NCK | 47 | ++ | - | - | - | f | |
| 80 | SHC | 52/66 | ++ | - | - | - | f | |

| **Cell adhesion and cytoskeletal proteins** | | | | | | | | |
|---|---|---|---|---|---|---|---|---|
| 81 | N-Cadherin | 150 | ++ | + | + | ++ | u | 81, 87, 88, 90 |
| 82 | E-Cadherin | 120 | ++ | - | - | - | f | |
| 83 | P-Cadherin | 120 | ++ | - | - | - | f | |
| 84 | Cadherin-5 | 130 | +++ | - | - | - | f | |
| 85 | Desmoglein | 165 | +++ | ++ | ++ | + | f | 85, 88 |
| 86 | α-Catenin | 102 | +++ | - | - | - | f | 87, 88, 95 |
| 87 | β-Catenin | 92 | +++ | ++ | ++ | + | f | 81, 86 |
| 88 | γ-Catenin | 82 | +++ | - | - | - | f | 85, 86 |
| 89 | L1 | 200 | ++ | ++ | ++ | + | f | 89 |
| 90 | p120cas | 120 | +++ | +++ | ++ | + | f | 44, 81 |
| 91 | Paxillin | 68 | +++ | - | - | - | f | |
| 92 | MAP2B | 280 | +++ | ++ | + | + | f | 25, 68, 101 |
| 93 | Vinculin | 117 | +++ | - | - | - | v | |
| 94 | Actin | 45 | +++ | +++ | +++ | +++ | w | 73, 95, 96, 97, 102 |
| 95 | α-actinin 2 | 110 | ++ | +++ | ++ | ++ | v | 1, 3, 86, 94 |
| 96 | Spectrin | 240/280 | +++ | +++ | ++ | ++ | v | 1, 2, 3, 94, 96 |
| 97 | Myosin | 205 | ++ | ++ | ++ | + | v | 94 |
| 98 | tau | 50-68 | + | - | - | - | f | |
| 99 | Tensin | 215 | + | - | - | - | f | |
| 100 | Ezrin | 80 | +++ | - | - | - | f | |
| 101 | Tubulin | 50 | +++ | +++ | +++ | +++ | v | 1, 3, 9, 61, 66, 71, 73 90 91 102 |
| 102 | Cortactin | 80/85 | +++ | + | + | ++ | x | 14, 44, 61, 94, 103 |
| 103 | CortBP-1 | 180/200 | +++ | + | ++ | +++ | x | 102 |
| 104 | Clathrin Heavy Chain | 180 | +++ | ++ | ++ | +++ | f | 106 |
| 105 | Dynamin | 100 | +++ | ++ | ++ | +++ | f | 26-34 |
| 106 | Hsp-70 | 70 | +++ | ++ | ++ | +++ | f | 101, 104 |

**TABLE 4**

| Protein name | Mr [kDa] | Accession number | MS events |
|---|---|---|---|
| Bassoon | 418 | O88778 | 11 |
| Myosin B heavy chain (non muscle type) | 229 | P35580 | 27 |
| p53 binding protein-1 | 214 | Q12888 | 2 |
| Tight junction protein ZO-1 | 195 | P39447 | 13 |
| Clathrin heavy chain | 192 | P11442 | 1 |
| NR2B | 166 | Q01097 | 85 |
| NR2A | 165 | P35436 | 36 |
| SynGAP | 143 | AAC08071 | 9 |
| NR1 | 105 | P35438 | 208 |
| α-actinin2 | 103 | Q62744 | 25 |
| Sap97 | 100 | Q12959 | 2 |
| Est525.7 hypothetical 97.8 kDa protein | 98 | CAB43675 | 3 |
| Dynamin | 96 | Q61358 | 4 |
| Chapsyn-110 | 95 | Q63622 | 108 |
| Sap102 | 93 | P70175 | 9 |
| Phosphofructokinase | 86 | P12382 | 7 |
| NSF vesicular fusion protein | 83 | P46460 | 1 |
| PSD-95 | 80 | Q62108 | 112 |
| Sarcolemmal associated protein-3 | 74 | Q28623 | 3 |
| GKAP/Sapap | 74 | P97841 | 4 |
| HSP70-like HS71 protein | 71 | U73744 | 24 |
| Kinesin light chain 2 | 67 | 088448 | 2 |
| PP2A regulatory R1α chain | 65 | P30153 | 1 |
| CaM Kinase II β | 60 | P28652 | 1 |
| PP2B (Calcineurin) α chain | 59 | P20652 | 3 |
| α-Internexin | 56 | P46660 | 2 |
| CaM Kinase II α | 54 | P11798 | 4 |
| RNA binding protein FUS/TLS | 53 | P35637 | 2 |
| Tubulin alpha-4 chain | 50 | P05215 | 3 |
| Est700.75 | | AI428173 | 1 |
| Est736.26 | | AI047568 | 2 |
| Est536.68 | | AU050964 | 1 |
| Est621.15 | | AV153731 | 1 |
| Est571.14 | | AA982950 | 1 |
| Est762.20 | | AA592427 | 1 |

A prevailing cellular model of learning involves modification of synapse strength, induced by patterns of neuronal activity during training, which encodes information in neural networks (Bliss, T. V. & Collingridge, G. L., 1993, Nature 361, 31-9; Bear, M. F. & Malenka, R. C., 1994, Curr Opin Neurobiol 4, 389-99). Understanding the molecular mechanisms used by synapses to detect altered patterns of firing, and how signalling cascades then change strength of synaptic transmission, may provide basic insights into this model and illuminate pathological mechanisms that occur in human learning impairments. Considerable attention has focussed on the NMDA subtype of glutamate receptor (Hollmann, M. & Heinemann, S., 1994, Annu Rev Neurosci 17, 31-108) since its blockade in the hippocampus impairs both synaptic plasticity and learning (Bliss, T. V. & Collingridge, G. L. , 1993, Nature 361, 31-9). This receptor-channel, which allows calcium influx into the postsynaptic spine, has been shown to regulate kinases, phosphatases and other enzymes which then regulate AMPA (α-amino-3-hydroxy-5-methyl-4-isoxasole-4-propionic acid) receptors, spine cytoskeletal changes, translational, transcriptional and other events. Moreover, in a physiological setting these signals must be subtly integrated as shown by electrophysiological studies in CA3-CA1 synapses of the hippocampus, where low frequency of synaptic stimulation results in an NMDA receptor dependent long term depression (LTD) and higher frequencies a potentiation (LTP) in synaptic strength (Bear, M. F. & Malenka, R. C., 1994, Curr Opin Neurobiol 4, 389-99). In addition to this apparent diversity of NMDA receptor signalling roles, the observations that many molecules including adhesion proteins, multiple second messengers and structural proteins are involved in synaptic plasticity has been puzzling (Sanes, J. R. & Lichtman, J. W., 1999, Nat Neurosci 2, 597-604).

Assembly of receptors with signal transduction proteins into large multiprotein complexes has emerged as a general mechanism of cellular signalling (Pawson, T. & Scott, J. D., 1997, Science 278, 2075-80). The NMDA receptor binds postsynaptic proteins (Kim, J. H. & Huganir, R. L., 1999, Curr Opin Cell Biol 11, 248-54) including Post Synaptic Density 95 (PSD-95), which was shown to be necessary for synaptic plasticity in mutant mice (Migaud, M. et al., 1998, Nature 396, 433-9). To further explore this model, we have isolated NMDA receptor-PSD-95 complexes from mouse brain and analysed their properties using immunoblotting, mass spectrometry and proteomic techniques which are powerful tools for analysis of large protein complexes (Jensen, O. N., Larsen, M. R. & Roepstorff, P., 1998, Proteins Suppl, 74-89; Mendelsohn, A. R. & Brent, R., 1999, Science 284, 1948-50; Neubauer, G. et al., 1998, Nat Genet20, 46-50). This biochemical approach allows the detection of protein interactions that require posttranslational modifications such as phosphorylation and lipid modification, e.g. in PSD-95 (Craven, S. E., EI-Husseini, A. E. & Bredt, D. S., 1999, Neuron 22, 497-509), and ternary or weak interactions undetected by 2-hybrid screens (Mendelsohn, A. R. & Brent, R., 1999, Science 284, 1948-50), which have been extensively used to identify NMDA receptor and PSD-95 binding partners (Kim, J. H. & Huganir, R. L., 1999, Curr Opin Cell Biol 11, 248-54; Hsueh, Y. P. & Sheng, M., 1998, Prog Brain Res 116, 123-31). Moreover, a biochemical analysis from brain is required to analyse mice carrying mutations affecting the complexes as well as other *in vivo* analyses.

Three methods including immunoaffinity chromatography and immunoprecipitation with an antibody directed against the NMDA R1 subunit and peptide affinity based on the structure of the NMDA R2B subunit C-terminus that binds to the NMDA receptor binding protein PSD-95, produced clearest results as shown in Figure 8. SDS-PAGE analysis of individual fractions showed the purified material was very complex (Figure 8A and B, lanes IA, IP and Pep) compared to unspecific interaction of extracted proteins with Protein G-Sepharose used in immunoprecipitations (lane WT/G) or affigel-10 used in antibody and peptide affinity purification. The integrity of the complexes and specificity of protein interactions were examined. NR subunits (NR1, NR2A, NR2B) and their reported interacting proteins (Kim, J. H. & Huganir, R. L., 1999, Curr Opin Cell Biol 11, 248-54; Hsueh, Y. P. & Sheng, M., 1998, Prog Brain Res 116, 123-31) (PSD-95, Chapsyn-110/PSD-93, calmodulin, α-actinin, calcium/calmodulin kinase II (CamKII), phospholipaseCγ) were readily detected (Figure 8C and Table 3) and PSD-95 binding proteins including nNOS, SynGAP, SAPAP/GKAP and Citron were also found (Figure 8D). We next examined AMPA subunits (GluR1-4) and their cognate adapter protein GRIP, which were not detected in the multi-protein complex despite their abundance in the extract (Figure 8E). Kainate receptor subunits (GluR6/7) were detected, consistent with previous reports that PSD-95 binds these subunits (Garcia, E. P. et al, 1998, Neuron 21, 727-39). The metabotropic (mGluR1α) receptors and their cognate binding partner Homer/Vesl-1, were found in the multi-protein complex (Figure 8F) consistent with a recent report using 2-hybrid screening showing Homer binds SHANK which can bind GKAP to PSD-95 (Tu, J. C. et al., 1999, Neuron 23, 583-92). Therefore the NR and mGluR receptors are associated in distinct complexes to AMPA receptors.

We next identified novel components of the multi-protein complex using two strategies: a western blotting screen for candidate proteins involved with NR signalling and scaffolding (Table 3) and protein identification using Mass Spectrometry (Figure 9 and Table 4). The protein bands indicated in Figure 8G were analysed by MALDI- peptide mass fingerprinting and online LC-MS/MS (Figure 9).

The NR and mGluR receptors have been implicated in the induction of synaptic plasticity through activation of second messenger pathways (Figure 10). We examined a wide range of different kinases and phosphatases in the multi-protein complex and found several specific serine-threonine kinase and phosphatase family members. Protein Kinase A (PKA) catalytic subunit and regulatory subunit R2β (R1, R1α and R2α were not detected), Protein Kinase C (PKC) isoforms β, γ, ε were found however α, δ, η, θ, 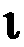 , λ isoforms were not detectable. The α and β subunits of (CamKII) were associated, as previously reported (Leonard, A. S., Lim, I. A., Hemsworth, D. E., Horne, M. C. & Hell, J. W., 1999, Proc Natl Acad Sci U S A 96, 3239-44), and also found to be in the phosphorylated active form. The presence of these kinases, which are known to be involved with the induction of synaptic plasticity, suggests that counterpart phosphatases may also be present in the multi-protein complex. We found that PP1 and PP2A were strongly associated and PP2B (calcineurin) and PP5 were also associated. PKA and PP1 were recently found to be linked to the NR1 subunit via an adapter protein (Yotiao) and can modulate NR currents *in vitro* (Westphal, R. S. et al., 1999, Science 285, 93-6). We found Yotiao and another PKA adaptor, AKAP150, suggesting that PKA may be linked to the multi-protein complex at multiple locations, perhaps serving in distinct pathways.

Tyrosine phosphorylation is also involved with NMDA receptor (Wang, Y. T. & Salter, M. W., 1994, Nature 369, 233-5) and synaptic plasticity (O'Dell, T. J., Kandel, E. R. & Grant, S. G., 1991, Nature 353, 558-60). Investigation of cytoplasmic tyrosine kinases revealed Src (but not Fyn) and the calcium activated kinase Pyk2 (but not the Pyk2 homologue Focal Adhesion Kinase, FAK) associated with the multi-protein complex. Although tyrosine phosphatase inhibitors influence NR channels (Wang, Y. T. & Salter, M. W., 1994, Nature 369, 233-5), the identity of relevant tyrosine phosphatases has remained unclear. We detected PTP1D, which was described recently to associate with NR2B (Lin, S. Y. et al., 1999, Brain Res Mol Brain Res 70, 18-25), but neither the related PTP1B or PTP1C in the multi-protein complex, implicating this tyrosine phosphatase in multi-protein complex regulation.

How NR mediated kinase and phosphatase signal integration occurs is unclear. In the present studies, we found both H-Ras and Rap2 small G-proteins and their GTPase Activating Proteins NF1 and SynGAP (which binds PSD-95), but not p120GAP in the multi-protein complex. Rap1, which may couple PKA to c-Raf1 was not detectable in the multi-protein complex. Ras mediates its effects by differential activation of several downstream effector pathways (Gille, H. & Downward, J., 1999, J Biol Chem 274, 22033-40) including the c-Raf1-MEK-ERK/MAPK pathway, PI3Kinase and RalA. It is now recognised that the different MAPK pathways, are organised into modules comprised of the key enzymes producing that cascade, tethered within a cell to produce signal specificity (Schaeffer, H. J. & Weber, M. J., 1999, Mol Cell Biol 19, 2435-44). This was also the case for the multi-protein complex since ERK1 and ERK2 and their upstream activating kinases MEK1, MEK2 and c-Raf1 were found in contrast to JNKK1/MKK4 and ERK3. Moreover, the ERK phosphatase (MKP2), which inactivates ERK was also identified. Together with the observation that the other Ras effectors, PI3K and Ral were detected, these data are consistent with glutamate receptor signals driving Ras which couples to distinct downstream pathways within the multi-protein complex.

The role of ERK/MAPK pathways in synaptic plasticity and learning has received considerable attention since phosphorylation of ERK accompanies these processes which are also disrupted by inhibitors of MEK (Impey, S., Obrietan, K. & Storm, D. R., 1999, Neuron 23, 11-4). Phosphorylation of ERKs has mainly been implicated in regulating transcription, through phosphorylation of RSK2, which translocates to the nucleus to phosphorylate transcription factors CREB and CREM. Interestingly, phosphoERK was not present in the multi-protein complex although readily detected in the extract consistent with its ability to translocate from the multi-protein complex upon phosphorylation. Moreover, RSK2 was found within the multi-protein complexalthough the transcription factors CREB and CREM, were not detectable in the multi-protein complex. We noticed that several of the multi-protein complex proteins (Homer, NR1, NR2B, PKCγ, ERK2, c-Raf1, HSP70) were encoded by activity dependent genes and we therefore tested Arg3.1/Arc (Link, W. et al., 1995, Proc Natl Acad Sci USA 92, 5734-8), a postsynaptic protein of unknown function that is also rapidly regulated by LTP. Arg3.1 was readily detected in the multi-protein complex suggesting it may participate in signalling and dynamic organisation of the multi-protein complex. Thus, the multi-protein complex contains signalling mechanism that could contribute to transcriptional activation following synaptic activation and then itself be subject to structural changes secondary to altered gene expression. cPLA2 was another potential ERK effector (Lin, L. L. et al., 1993, Cell 72, 269-78) also regulated by Citron which binds PSD-95. cPLA2 mutant mice show resistance to ischaemic neuronal damage (Bonventre, J. V. et al., 1997, Nature 390, 622-5) and cPLA2 generates arachidonic acid, a candidate *trans*-synaptic retrograde signalling molecule.

Changes in synapse structure may be fundamental to the storage of long term memory and roles for cell adhesion molecules and cytoskeletal structural proteins were implicated (Murase, S. & Schuman, E. M., 1999, Curr Opin Cell Biol 11, 549-53). Many of the signalling proteins described above are known regulators of cytoskeleton and cell adhesion. Actin cytoskeleton was previously shown to have a function in NR channel properties (Rosenmund, C. & Westbrook, G. L., 1993, Neuron 10, 805-14), NR mediated LTP (Kim, C. H. & Lisman, J. E., 1999, J Neurosci 19, 4314-24) and NR localisation (Allison, D. W., Gelfand, V. I., Spector, I. & Craig, A. M., 1998, J Neurosci 18, 2423-36), and is dynamically regulated in spines with neural activity (Fischer, M., Kaech, S., Knutti, D. & Matus, A., 1998, Neuron 20, 847-54). We found α-actinin2 and spectrin (Fodrin), which were previously shown to bind NMDA receptor subunits (Wechsler, A. & Teichberg, V. I., 1998, Embo J 17, 3931-9), and actin binding proteins including cortactin, cortactin binding proteins (CortBP1 and SHANK) and MAP2 but not Ezrin, Tensin or Vinculin in the multi-protein complex. The cell adhesion proteins N-Cadherin and Desmoglein, two cadherin family members and their cytoplasmic interacting proteins β-catenin, ZO-1 and p120^{cas} were detected although E-cadherin, P-cadherin, cadherin-5, α-catenin, γ-catenin were negative. L1 adhesion protein, which is required for learning and synaptic plasticity (Luthl, A., Laurent, J. P., Figurov, A., Muller, D. & Schachner, M., 1994, Nature 372, 777-9) was also detected in the multi-protein complex. These adhesion proteins may play a role in structural organisation of the multi-protein complex at the synapse, however the involvement of cadherins (Tang, L., Hung, C. P. & Schuman, E. M., 1998, Neuron 20, 1165-75) and L1 in synaptic plasticity(Luthl, A., Laurent, J. P., Figurov, A., Muller, D. & Schachner, M., 1994, Nature 372, 777-9) make it tempting to speculate that this glutamate receptor-cell adhesion protein complex could provide multiple trans-synaptic signalling pathways, where adhesion mediated signalling is coupled to transmitter signalling mechanisms.

Mass spectrometry (MS) of large scale isolated multi-protein complexes has confirmed and extended the identification of multi-protein complex components beyond yeast 2-hybrid assays and immunoblotting (Tables 3 and 4). Some multi-protein complex components were not detected by MS and this may be accounted for by the low levels of protein in the selected gel fragments. In addition to identification of previously unsuspected known proteins, the MS approach also identified seven peptides that are represented in the dbEST database. Two of these EST clones originate from mouse hippocampus (AU050964 and AV153731) whilst one of the others corresponds to a 97kDa Hypothetical protein (CAB43675). Although it is clear that some protein complexes contain dozens of proteins (e.g. see Jensen, O. N., Larsen, M. R. & Roepstorff, P., 1998, Proteins Suppl, 74-89; Mendelsohn, A. R. & Brent, R., 1999, Science 284, 1948-50; Neubauer, G. et al., 1998, Nat Genet 20, 46-50), the large number of multi-protein complex proteins identified in this study does not imply all proteins exist in a single homogeneous complex. Heterogeneity could result from different complexes in the starting material, as may be found at different synapses. Moreover the large size of the complex may reflect dynamic assembly or transient protein-protein interactions of signalling complexes, which is a feature of many signalling pathways involving the proteins in the multi-protein complex (Pawson, T. & Scott, J. D., 1997, Science 278, 2075-80).

The structure of the multi-protein complex isolated from the mouse brain implies that subsets of neurotransmitter receptors, cell adhesion proteins, adapters, second messengers and cytoskeletal proteins are organised together into a physical unit comprising signalling pathways (Figure 10). The simplest general function for the multi-protein complex may be in the 'induction phase' of synaptic plasticity in contrast to the 'expression phase' as described in electrophysiological experiments. Induction involves activation of the NMDA receptor and mGluR, Ca²⁺ influx and second messenger signalling and lasts less than one hour, whereupon inhibition of NMDA receptor or second messengers does not alter the new level of synaptic strength, which is mediated or expressed by AMPA receptors. This model of the multi-protein complex as an induction device is strongly supported by targeted mouse mutations, transgenic expression and pharmacological inhibition of multi-protein complex proteins which alter the induction of synaptic plasticity: targeted mouse mutations; NR1, NR2A, mGluR1, PSD-95, PKA catalytic and regulatory subunits, PKCγ, CamKII, nNOS, NF1, H-Ras; transgenic mice expressing NR2B, CamKII, Calcineurin, PKA inhibitors, L1; and pharmacological inhibitors of NR, mGluR, PKA, PKC, CamKII, MEK, tyrosine kinases, PP1, PP2A, PP2B, Cadherins, L1, actin polymerisation, Calmodulin, nNOS, PI3K and cPLA2. The multi-protein complex is also well suited to induce bidirectional (LTP and LTD) synaptic plasticity since many calcium sensitive proteins with kinases and phosphatases are beneath the NMDA receptor calcium pore in a microdomain. The integration of signalling events within the complex, such as kinase and phosphatase activation and Ras regulation, could provide the graded output necessary for setting the thresholds for LTP and LTD as well as regulating the activation of Late-LTP involving translational and transcriptional activation.

### EXAMPLE 3: DETERMINATION OF THE SIZE OF THE MULTI-PROTEIN COMPLEX BY SIZE EXCLUSION CHROMATOGRAPHY

### Materials and Methods

### Purification of multi-protein complex

The multi-protein complex was purified from mouse forebrain by immunoaffinity chromatography, as described in Example 1.

### Size exclusion chromatography

A large scale anti-NR1 IP sample using 50 ml mouse brain extract and 0.5 mg of the MAP-NR1 sheep IgG was incubated with 10 mg MAP-NR1 peptide for lh at 4°C in a total volume of 1.5ml in order to compete bound material from the resin. The supernatant was then removed and the resin washed with a further 1 ml of DOC buffer. Both supernatants were combined and subjected to gel filtration over a 120 ml Toyopearl HW-65F resin in a XK16/70 column connected to a FPLC (both AmershamPharmacia, Amersham, UK) at 4°C at 1 ml/min in DOC buffer. Samples were taken at 1.5 min intervals and the absorption of effluent monitored at 280 nm. Samples were subjected to analysis by Western blotting against NR1. The column was calibrated using standards from the HMW gel filtration kit.

### Results

Results of gel filtration analysis of the multi-protein complex extracted from mouse forebrain are shown in Figure 11.

The absolute size of the multi-protein complexes within which NMDA receptors are located has important implications for understanding the function of the receptor at synapses. Using the isolation methods described in Example 1, we determined that the composition of the multi-protein complexes comprising NMDAR receptors includes about 75 proteins. This large number of components is consistent with either a large 'super complex' or multiple smaller heterogeneous complexes. We chose to use gel filtration to address this issue and to obtain an estimate of the size of the complexes. Complexes were isolated using the NR1 affinity procedure, eluted using peptide competition, then subject to gel filtration where collected fractions were Western blotted using antibodies to NR1. The results showed that aggregation did not occur, since this would yield NR1 immunoreactive material at void volume (v0). Furthermore, the absence of monomeric NR1 subunits in the region just prior to the total column volume (vt) supports the view that complexes were collected. The Gaussian distribution of NR1 containing samples across the 2000 kDa mark is a further indication that the multi-protein complexes are of similar size.

### Discussion

Although the stoichiometry of the NMDA receptor channel is unclear, the possibility that it is a tetramer or pentamer of NR1 and NR2 subunits would predict the size of the channel ranges from 600-900 kDa. In contrast, the multi-protein complex isolated in Example 1 was determined to have a molecular weight of about 2000 kDa. Thus the complex appears to be a large signalling entity within which the NMDA receptor is a subcomponent.

This demonstration of 2000 kDa complexes comprising the NMDA receptor and associated proteins and the detailed composition of the complex into neurotransmitter receptors, cell adhesion proteins, adaptor molecules, signalling proteins and cytoskeletal proteins is important for determining how the postsynaptic terminal functions. Although it is known that specific neurotransmitter receptors mediate specific signalling events and thereby cellular functions, the growing body of two-hybrid interaction data along with more traditional PSD protein identification studies has lead to the view that the PSD is comprised of a large scaffold or network of interacting proteins. The present study shows that the postsynaptic neurotransmitters are in physically isolatable complexes, the NMDA receptor being located within a very large the multi-protein complex which is distinct to the complexes of other important PSD neurotransmitter receptors, such as AMPA complexes. Thus, the postsynaptic architecture must be comprised of large multiprotein complexes that are distinct subcomponents of PSDs, which is a more nonspecific entity or organelle observed using electron microscopy.

The demonstration that PSD-95 is integral to the function of the NMDA receptor in synaptic plasticity and learning (Migaud et al., 1998, *Nature* **396**:433-439) indicates that the specific complexes are functional entities in a physiological setting. These multi-protein complexes contain a high number of molecules that when perturbed pharmacologically or genetically lead to impairments in the synapses ability to detect and convert patterns of firing into long-term changes in synaptic strength and for learning in the animal. These properties of pattern detection leading to synaptic reinforcement, described by Hebb (1949), are well explained by the physiological role of these 2000 kDa complexes, which we refer to as "Hebbosomes".

### EXAMPLE 4: INVESTIGATION OF THE ROLE OF THE MULTI-PROTEIN COMPLEX IN SIGNALLING, SYNAPTIC PLASTICITY AND LEARNING

### Materials and Methods

### Gene Targeting

An internal ribosomal entry site upstream of a β-galactosidase reporter gene was introduced to monitor the cellular expression pattern (Migaud et al., *supra*). Targeting constructs were electroporated into embryonic stem (ES) cells and southern blot analysis indicated that homologous recombination had occurred in 18 out of 180 clones (10%) for SynGAP and 58 out of 72 (81 %) for H-Ras and germline transmission established (Fig. 1a,b). A 565-base pair cDNA fragment which encodes part of the SynGAP PH and C2 domains was generated from mouse brain RNA extract by RT-PCR. This cDNA was used as a probe to screen a 129/Ola mouse genomic library and overlapping λ clones covering 22 kb of the SynGAP locus were isolated. To construct a SynGAP targeting vector, an 8.1 kb SmaI-XhoI fragment and a 3.8 kb SpeI-SpeI fragment were used as 5' and 3' homology arms respectively. A coding sequence for hemagglutinin (HA) epitope tag was inserted in frame at an XhoI site in the C2 domain (Kim, J. H., et al, Neuron 20, 683-91 (1998), Chen, H. J., et al, Neuron 20, 895-904 (1998)). at the 3' end of the 5' homology arm, and followed by stop codons and IRES-lacZ-polyA•MC1neo-polyA cassette (Morris, R. G., et al, Nature 319, 774-6, 1986). The resultant vector deletes exons encoding the C2 and GAP domains. The Diphtheria toxin A gene (MC1-DT-A) was used as a negative selection marker. The targeting vector was linearised and electroporated into E14TG2a ES cells. Neomycin (neo) resistant clones were screened for homologous recombination by southern blot analysis using a 700 bp 3' flanking probe (EcoRI or XbaI digest) and 354 bp cDNA internal 5' probe for (BglII digest). The targeting vector for the H-Ras1 locus comprised 1.9 kb and 5.5 kb bands of 5' and 3' DNA flanking a cassette (TAG3-IRES-βgeo-polyA) containing the positive selectable marker neo. The 5' end of this cassette contained stop codons in all reading frames to terminate translation of H-Ras at the SpeI site in exon 4, followed by an internal ribosomal entry site (IRES) which allows a β-geo reporter gene to be expressed under the control of the H-Ras1 promoter. The targeting construct was linearised and electroporated into HM1 ES cells. Neomycin resistant clones were screened for homologous recombination by southern blot analysis using a 1.1 kb fragment (5' probe) flanking the homology region and a 1.2 kb genomic DNA (3' probe). Chimaeric mice were produced by injecting targeted ES cells into C57BL/6 blastocysts and heterozygous mutants were generated as previously described (Papaioannou V. and Johnson R., 1999, Production of chimeras by blastocyst and morula injection of targeted ES cells. In Gene Targeting A Practical Approach 2nd ed (ed. Joyner A.L.), 4, pp. 133-175, Oxford University Press, IRL Press, New York). All comparisons of SynGAP^{-/+} and wild type mice were performed using littermates on an F2 MF1 genetic background. Animals were treated in accordance with UK Animal Scientific Procedures Act (1986) and NIH guidelines.

### Biochemistry

The hippocampus was homogenised in 50 mM Tris-HCl, 1% sodium deoxycholate, 50 mM NaF, 20 mM ZnCl₂, 1 mM sodium orthovanadate, 0.5 mg/ml PMSF, Protease Inhibitors Complete^{™} (Roche Molecular Biochemicals). Proteins were separated by SDS-PAGE (25 µg/lane) and standard procedures were used for western blotting. Antibodies: SynGAP, NR2A (Upstate Biotechnology); NF1, SynGAP, HA (Santa Cruz Biotechnology); p120RasGAP, H-Ras, NR1, NR2B, PSD-95, ERK, MEK (Transduction Laboratories); panRas (Oncogene); phosphoMAPK, phosphoMEK (New England BioLabs).

### Morphology

Mice were perfusion fixed via the heart with heparinised saline followed by 2 % paraformaldehyde, 2.5% glutaraldehyde in 0.1M sodium phosphate buffer (pH 7.4). The brain was removed and placed in fixative for a further 2 h. 70 µm coronal sections were taken through the anterior part of the hippocampus using a vibrating microtome (Vibratome, Lancer). From every three serial sections one was taken for EM processing and the remainder for section Golgi impregnation. Sections were dehydrated and mounted in resin before viewing in the light microscope. Consistent regions of dorsal CA1 were re-sectioned and viewed in an electron microscope (Philips CM12) (Morrison, B. M., et al., J Comp Neurol 395, 523-34, 1998; Bolam J.P., 1992, Preparation of central nervous system tissue for light and electron microscopy. In Experimental Neuroanatomy A Practical Approach (eds. Rickwood D., Hames B.D.), 1, pp. 1-29.Oxford University Press, IRL Press, New York.). The stratum radiatum was examined and the first twenty clearly defined asymmetrical axospinous synapses were photographed. X-gal Staining: Fresh brain was dissected into 5mm sagital slices fixed in 1 % formaldehyde, 0.01% glutaraldehyde in 0.1M sodium phosphate buffer (pH 7.4) for 1hr at 4°C then stained overnight for X-gal (5-bromo-4-chloro-3-indolyl-β-galactoside) (Gibco BRL) (Migaud et al, *supra*). Immunofluorescent Staining: Brain tissue was fixed in 10% Buffered Formalin for 6hrs dehydrated and then wax embedded. 8µm coronal sections were cut on a microtome. Standard procedures were followed for immunofluorescent staining, mounting and coverslipping (Morrison et al, *supra*). Primary antibodies were: anti-synaptophysin mAb (1/100; Boehringer); MAP2B mAb (1/100; Transduction Labs). Secondary antibodies were: Cy3 conjugated antibody (1/200; Jackson Lab).

### Electrophysiology

Hippocampal slices (400 µm thick) were prepared using standard techniques and maintained in a interface-type recording chambers perfused at a constant rate (1 to 3 mL/min.) with a warmed (30 °C), oxygenated (95 % O₂/5% CO₂) artificial cerebrospinal fluid (ACSF) consisting of 124 mM NaCl, 4.4 mM KCl, 25 mM Na₂HCO₃, 1 mM NaH₂PO₄, 1.2 mM MgSO₄, 2 mM CaCl₂, and 10 mM glucose. Low resistance glass microelectrodes filled with ACSF were placed into stratum radiatum of the hippocampal CA1 region to record field excitatory postsynaptic potentials (fEPSPs) evoked by presynaptic stimulation pulses delivered once every 50 seconds to the Schaffer collateral/commissural fibers via a bipolar nichrome wire stimulating electrode. At the beginning of each experiment the intensity of presynaptic fiber stimulation was adjusted to evoke fEPSPs that were approximately half of the maximal fEPSP amplitude that could be evoked by strong intensity stimulation. High-frequency stimulation-induced LTP was elicited using two trains of 100 Hz stimulation (1 sec. in duration) delivered with an inter-train interval of 10 seconds. To elicit saturating levels of LTP we delivered 6, one-second-long trains of 100 Hz stimulation with an inter-train interval of 5 minutes. For statistical comparisons (unpaired t-tests, two-tailed, N = number of animals) we compared the average amount of potentiation present 55 to 60 minutes after the first high-frequency stimulation train or 40 to 45 minutes after low-frequency trains of stimulation. Whole-cell current clamp recordings were used to study the induction of LTP by low-frequency presynaptic fiber stimulation paired with postsynaptic depolarization. In these experiments slices with the CA3 region removed were bathed in a modified ACSF containing elevated levels of CaCl₂ and MgSO₄ (4 mM each), lower levels of KCl (2.2 mM), and 100 µM picrotoxin. EPSPs evoked by 0.05 Hz presynaptic fiber stimulation were recorded from individual CA1 pyramidal cells using low resistance (2 to 5 Mohms) patch-clamp electrodes filled with a solution containing 122.5 mM Cs-gluconate, 17.5 mM CsCl, 10 mM TEA-Cl, 0.2 mM EGTA, 10 mM HEPES, 2 mM Mg-ATP and 0.3 mM GTP (pH = 7.2). Constant current injections were used to hyperpolarize cells to between -80 and -85 mV and a 50 millisecond long 0.1 nA pulse of hyperpolarizing current was injected 150 milliseconds after each evoked EPSP to monitor input and access resistance throughout the experiment. The intensity of presynaptic fiber stimulation was set to evoke EPSPs between 5 and 10 mV in amplitude. After a 10 minute period of baseline recording, LTP was induced by depolarizing the postsynaptic cell to near 0 mV using current injected through the recording electrode and pairing this depolarization with 100 presynaptic fiber stimulation pulses delivered at 2 Hz. Unpaired t-tests (two-tailed) were used to compare the average amount of LTP induced 25 to 30 minutes post-pairing in cells from wild-type and mutant animals. Whole-cell voltage-clamp recordings were used to examine the NMDA receptor-mediated component of excitatory postsynaptic currents (EPSCs) across a range of postsynaptic membrane potentials. In these experiments slices were bath in the modified ACSF described above and CA1 pyramidal cells were voltage-clamped using patch-clamp electrodes (2 to 4 Mohms, access resistance ranged from 14 to 22 Mohms) filled with either the Cs gluconate-based electrode-filling solution or with a solution containing 120 mM CsMeSO₃, 20 mM CsCl, 8 mM NaCl, 0.2 mM EGTA, 10 mM HEPES, 4 mM Mg-ATP, and 0.3 mM GTP (pH = 7.2). Similar results were obtained with both internal solutions and the results were combined. The AMPA and NMDA receptor-mediated components of the EPSCs were estimated from the amplitude of the EPSCs measured 5 and 50 milliseconds after the onset of the EPSC, respectively. All experiments and initial data analysis were performed blind except for experiments done on 3 H-Ras^{-/-} mutant mice. The results from these experiments did not differ from additional experiments on these animals done that were done in a blind fashion and the results were therefore combined. All values are reported as mean ± SEM.

### Behaviour

We used an open-field watermaze (2 m in diameter, opaque water, 25 ± 1°C, automated swim path monitoring). In the cue task, mice were trained to a randomly located platform position marked with a cylindrical cue (4 trials per day for 3 days; 30-cm platform diameter; curtains drawn around the pool to occlude extra-maze cues; maximum trial duration was 90 s; inter-trial interval (ITI), 10 min). For spatial training, we used a hidden platform with the extra maze cues visible (4 trials per day for 5 days, 30-cm platform diameter; platform/pool area was 1/44; 30 s was spent on the platform at the end of each trial; maximum trial duration 90 s; ITI 10 min). Transfer test 1 was conducted 10 min after the previous training trial, mice were placed in the pool for 60 s (platform absent; the start position was opposite to whatever training quadrant had been used for each individual animal). Two measures of transfer test performance were calculated: 1) percentage time spent in the target quadrant of the pool, and 2) time spent in a zone corresponding to the area of the absent platform (radius = 15 cm), expressed as a percentage of the total time spent in all 4 possible zones (see Fig. 16d,f). Training to criterion involved using a smaller hidden platform, (20-cm diameter; platform area/pool area was 1/100) again with the extra maze cues visible, until the animals had completed 2 consecutive days with each trial taking < 20 s, or until 32 trials had been completed. On reaching criterion, and 10 min after the previous training trial, individual mice were placed in the pool for 60 s (platform absent, transfer test 2). This training protocol was identical to that used for PSD-95^{-/-} mice (Migaud et al, *supra*), thus enabling a comparison of the performance of PSD-95^{-/-} and SynGAP^{-/+} mutant mice. Two replications completed testing up to this point (SynGAP^{-/+} total n = 21; wild type total n = 21). Only the initial replication underwent retention testing as follows (SynGAP^{-/+} n = 12; wild type n = 12). Two further transfer tests, or retention tests, were conducted 7 days and 45 days after the mice had reached criterion, and had completed the second transfer test. Again, mice were placed in the pool for 60 s (platform absent, transfer test 3 and 4 respectively).

### Results

The murine genes encoding SynGAP and H-Ras were cloned and characterized, knockout targeting vectors constructed (Fig. 12 a,b) and mutant mice generated. Male and female SynGAP^{-/+} mice appeared normal, displayed no sign of seizures, tremor, ataxia or other neurological abnormality, and they were fertile. Genotypes of 3 week old pups from SynGAp^{-/+} intercrosses showed no surviving homozygote SynGAP^{-/-} animals. Of 543 pups born, 325 reached weaning of which 128 were wild type and 197 SynGAP^{-/+} (χ² = 8.417, P<0.001). 80 newborn pups were genotyped and showed normal mendelian transmission of the mutation (23 wt, 39 SynGAP^{-/+}, 18 SynGAP^{-/-}; χ² = 0.714, P>0.05) indicating that SynGAP is essential for postnatal viability. All SynGAP^{-/-} pups were observed to breath and feed and showed no gross anatomical abnormality although they died within 48 hours. In contrast, H-Ras^{-/+} intercrosses produced H-Ras^{-/-} offspring at the expected mendelian ratio (323 pups at weaning, 93 wt, 152 H-Ras^{-/+} ,79 H-Ras^{-/-}, χ² = 0.293, P>0.05). These offspring were fertile and showed no signs of neurological abnormality.

The neuronal expression pattern of SynGAP is unknown and X-gal staining of SynGAP^{-/+} brains revealed highest expression in neurons of the hippocampus CA1 and dentate gyrus and cortex, and lower expression in striatum (Fig. 12 c), similar to PSD-95 (Migaud et al, *supra*). By contrast, H-Ras was widely expressed in most brain regions (Fig. 12c). Disruption of protein expression was confirmed using immunoblotting of hippocampus extracts (Fig. 12d). SynGAP levels were reduced in SynGAP^{-/+} compared with wild type mice using both amino and carboxyl terminal antibodies. No truncated protein products were detected using these antibodies or with antibodies to the HA tag inserted into the locus. Immunoblotting of H-Ras showed no detectable H-Ras in hippocampus extracts from H-Ras^{-/-} mice. We tested for compensation by the two other mammalian GAP proteins Neurofibromin-1 (NF1) and p120RasGAP in the hippocampus extracts of SynGAP^{-/+} mice and observed no increases above wild type levels (Fig.12d). We used a Pan-Ras antibody that detects all Ras isoforms and saw a reduction in total Ras levels in H-Ras mutant mice. Finally, we examined levels of expression of NR1, NR2A, NR2B, PSD-95 in SynGAP^{-/+} and H-Ras^{-/-} mutant mice and saw no changes from wild type levels (Fig. 12d).

To explore the role of SynGAP and H-Ras in the brain we examined the neuroanatomy at the light and electron microscopic level in mature (6-8 week old) SynGAP^{-/+} and H-Ras^{-/-} mice (Fig. 13). There was no gross detectable abnormality found using Nissl staining in the brain of SynGAP^{-/+} or H-Ras^{-/-} mice. Both SynGAP and H-Ras are highly expressed in the CA1 region of the hippocampus (Fig. 12c). In this region, the intensity and distribution of Nissl staining and the distribution of the synaptic-terminal marker synaptophysin and of the dendritic marker MAP2 were the same in SynGAP^{-/+}, H-Ras^{-/-} and wild type mice. Dendritic architecture was further examined using Golgi staining and electron microscopy, revealing dendritic branching, spine distribution and asymmetric synapse morphology, which were all normal. We prepared primary neuronal cultures from individual E18.5 embryos and found neurones from SynGAP^{-/-} mice extended neurites and formed morphological synapses, as shown by immunostaining with the synaptic vesicle proteins Synaptophysin and dendritic protein MAP2, which were indistinguishable from wild type cultures. Electrophysiological evidence for synaptic transmission between SynGAP^{-/-} neurons was obtained using whole-cell patch clamping to record miniature synaptic currents (mEPSCs) and showed typical kinetics of glutamatergic responses (data not shown). These data indicate that SynGAP was not essential for synapse formation or synaptic transmission in vitro. Together these data show SynGAP and H-Ras were not critical for synapse formation.

### Synaptic plasticity in SynGAP mutant mice

We next examined the role of SynGAP in synaptic transmission and plasticity in the CA1 region of the hippocampus. Under normal experimental conditions, where extracellularly recorded field excitatory postsynaptic potentials (fEPSPs) are predominately mediated by AMPA-type glutamate receptors, we detected no difference in the maximal fEPSP amplitude that could be recorded using strong intensity presynaptic fiber stimulation (max fEPSPs were 8.4 ± 0.7 mV in wild-type slices and 8.3 ± 0.5 mV in slices from SynGAP^{-/+} mice, N = 23 animals for each). Moreover, the ratio of fiber volley amplitude to fEPSP slope measured using presynaptic fiber stimulation intensities that evoked fEPSPs that were one-half the maximal amplitude was similar in slices from SynGAP^{-/+} and wild type mice (ratios were 0.2 ± 0.02 for wild type and 0.2 ± 0.03 for SynGAP^{-/+} mice, N = 15 animals for each). This suggests that the number of presynaptic fibers needed to evoke an equivalent postsynaptic response was similar in wild type and SynGAP^{-/+} mice. We also observed no difference in the NMDA receptor component of excitatory postsynaptic currents (EPSCs) recorded from pyramidal cells in wild type and SynGAP^{-/+} slices (Fig. 14a). Finally, pairs of presynaptic fiber stimulation pulses delivered at inter-pulse intervals of 25, 50, 100 and 200 milliseconds evoked nearly identical amounts of paired-pulse facilitation in slices from wild type and SynGAP^{-/+} mice (data not shown). Thus, we found no evidence for changes in several aspects of normal synaptic physiology, including NMDA receptor channel function, in SynGAP^{-/+} mice.

To examine whether NMDA receptor-dependent forms of synaptic plasticity might be altered in SynGAP^{-/+} mice we first investigated the effects of brief trains of high-frequency synaptic stimulation on synaptic strength at Schaffer collateral/commisural fibers synapses onto pyramidal cells in the hippocampal CA1 region. As shown in figure 14b, 60 minutes after two, one-second-long, trains of 100 Hz stimulation, synaptic transmission was potentiated more than 80 % in slices from wild-type mice (N = 7) but only 50% in slices from SynGAP^{-/+} mice (N = 7, p < 0.01 compared to wild-type). The amount of LTP induced by high-frequency stimulation was also significantly reduced (p < 0.005) in slices from SynGAP^{-/+} mice in experiments where 6 trains of high-frequency stimulation were delivered once every 5 minutes to induce saturating levels of LTP (Fig. 14c). Thus, although high-frequency synaptic stimulation induces significant potentiation in slices from SynGAP^{-/+} mice, the amount of potentiation induced by 100 Hz stimulation is greatly reduced compared to that seen in wild-type slices.

To explore the diminished capacity for LTP in SynGAP^{-/+} mutant slices further we examined the ability of lower frequency trains of synaptic stimulation to induce persistent changes in synaptic strength (Fig. 14d). A 900 pulse train of 1 Hz stimulation that was below threshold for LTP induction in slices from wild-type mice (fEPSPs were 98 ± 3% of baseline 45 minutes post-1 Hz stimulation, N = 5) also had little lasting effect on synaptic strength in slices from SynGAP^{-/+} mice (fEPSPs were 94 ± 3% of baseline, N = 9). Likewise, 900 pulses of synaptic stimulation delivered at 5 Hz induced only a small amount of potentiation in slices from wild-type mice (fEPSPs potentiated to 135 ± 9% of baseline, N = 5) and SynGAP^{-/+} mice (fEPSPs were 117 ± 5% of baseline, N = 9, not significantly different from wild-type, p = 0.07). When delivered at higher frequencies (10 and 20 Hz), 900 pulse trains of synaptic stimulation induced larger LTP in slices from wild-type mice (fEPSPs were potentiated to 153 ± 5%, N = 5, and 141 ± 4% of baseline, N = 7, respectively). In contrast, these stimulation protocols had relatively little effect on synaptic transmission in slices from SynGAP^{-/+} mice (fEPSPs were potentiated to 116 ± 2 %, N = 7, following 10 Hz stimulation, p < 0.001 compared to wild-type, and were 123 ± 7% of baseline, N = 7, following 20 Hz stimulation, p < 0.05 compared to wild-type). The postsynaptic responses evoked during the trains of synaptic stimulation used to induce LTP in these experiments were similar in wild-type and SynGAP^{-/+} slices, suggesting that the LTP deficit observed in SynGAP^{-/+} mice is not due to alterations in presynaptic function (data not shown). To investigate whether changes in postsynaptic excitability and/or inhibitory synaptic transmission contribute to the reduction in LTP observed in SynGAP^{-/+} mutant mice we blocked inhibitory synaptic transmission by including picrotoxin (100 µM) in the extracellular solution and examined the induction of LTP by pairing low frequency (2 Hz) presynaptic fiber stimulation with depolarization of the postsynaptic cell produced by current injected through an intracellular recording electrode (Fig. 14e). While this pairing protocol induced LTP in both wild-type and SynGAP^{-/+} cells, the average amount of potentiation induced in pyramidal cells from SynGAP^{-/+} mice was significantly less (p < 0.001) than that observed in cells from wild-type animals (Fig. 14e,f). This indicates that the LTP deficit observed in SynGAP^{-/+} mice is not due to changes in inhibitory synaptic transmission or excitability of the postsynaptic CA1 pyramidal cells. In addition, since LTP was induced in these experiments by low frequency synaptic stimulation paired with experimentally imposed postsynaptic depolarization, these findings indicate that the LTP deficit observed in SynGAP^{-/+} mice is not due to reduced postsynaptic depolarization resulting form presynaptic changes in synaptic transmission in SynGAP^{-/+} slices that might alter transmitter release during LTP-inducing trains of synaptic stimulation. Together, these data indicate that the physiological function of SynGAP is to couple a pathway to the NMDAR that is necessary to enhance LTP (Fig. 17)

### Genetic dissection of SynGAP and PSD-95 pathways

Since SynGAP and PSD-95 are both required for NMDA dependent plasticity, we can use genetic tools to determine their relationship within pathways. One possibility is a signalling pathway that leads from NMDA receptor to PSD-95 to SynGAP. A second possibility is that SynGAP is not downstream of PSD-95, as in the first scenario, but is instead in a separate parallel pathway downstream of the NMDAR. A genetic test that can distinguish these possibilities is to examine LTP in SynGAP^{-/+}/PSD-95^{-/-} double mutant mice. If they are in distinct parallel pathways, then the predicted magnitude of LTP will be intermediate between the enhanced LTP in PSD-95^{-/-} and reduced LTP in SynGAP^{-/+} mutants. Alternatively, if PSD-95 is upstream of SynGAP then the magnitude of LTP in the double mutant should resemble that seen in PSD-95^{-/-} mutants. In these experiments we induced LTP using a brief train of 5Hz stimulation (150 pulses) that induces a robust, but non-saturating, level of LTP in wild-type slices. As shown in Figure 15a, the amount of potentiation induced by 5 Hz stimulation in slices from SynGAP^{-/+}/PSD-95^{-/-} mutants was identical to that seen in PSD-95^{-/-} mice, supporting the model that there is a signalling pathway from NMDA to PSD-95 to SynGAP.

Although the LTP phenotype in SynGAP^{-/+}/PSD-95^{-/-} implicates a pathway leading from NMDAR to PSD-95 to SynGAP pathway, it also raises an interesting paradox. A single serial pathway would have similar phenotypes for each of the single mutants yet PSD-95^{-/-} and SynGAP^{-/+} mice have opposite LTP phenotypes. This points to the existence of another signalling pathway. While the reduced LTP in SynGAP^{-/+} mice indicates that the PSD-95-associated protein SynGAP is in a pathway that normally enhances LTP, the enhanced LTP observed in PSD-95^{-/-} mutants suggests that PSD-95 is also in a pathway that normally restrains LTP. Because both these pathways are genetically downstream of PSD-95, the simplest explanation would be if SynGAP regulated one distinct pathway and there was some other pathway also regulated by PSD-95 (Fig. 17). To address these possibilities it is necessary to further characterize the molecular events downstream of SynGAP.

### SynGAP regulates a pathway to MAPK

The GTPase function of SynGAP was shown *in vitro* to catalyze the conversion of the activated GTP bound form of Ras to the inactive GDP bound form (Kim et al, *supra*; Chen et al, *supra*). Since the activated GTP bound form of Ras binds effector proteins including Raf, which in turn phosphorylates and activates MEK, which then phosphorylates and activates MAPK (Impey et al, Neuron 24, 11-14, 1999), it was predicted that SynGAP could be a regulator of this MAPK pathway. This model would predict that in SynGAP^{-/+} mutants, which show reduced levels of SynGAP (fig. 12d), that Ras activity would be enhanced and raf, MEK and MAP be activated. To test this hypothesis we immunoblotted hippocampus extracts with antibodies to the phosphorylated active forms of MEK and MAPK (Fig. 15b). In SynGAP^{-/+} extracts the level of the phosphorylated active form of MEK and MAPK were clearly increased although the total level of these proteins was unchanged. A higher level of cRaf protein was also observed consistent with its active recruitment (data not shown). These data provide evidence that SynGAP regulates the activity of the Ras-MAPK pathway, which are components of the multi-protein complex. We can now extend the model described above to conclude that a pathway from SynGAP to MAPK is downstream of PSD-95 (Fig. 17).

### PSD-95 links a SynGAP-MAPK pathway and a MAPK-independent pathway to the NMDAR

The role of the MAPK pathway in LTP is of considerable interest since inhibitors of MEK suppress LTP induction (Selcher, J. C., et al, Learn Mem 6, 478-90 1999; Impey, S., et al., Neuron 23, 11-4 1999; Brenner, S., 1973, Br Med Bull 29, 269-71) while NMDAR activation enhances MAPK phosphorylation (Bading, H. & Greenberg, M. E. Science 253, 912-4, 1991). Our data supports the model that a SynGAP to MAPK pathway is downstream of PSD-95 and there is a second pathway downstream of PSD-95. Since the role of these two pathways appears to be distinct (either enhancing or restraining LTP), this raises the possibility that the second pathway from PSD-95 is MAPK independent. More specifically, we might predict that the enhancement of LTP seen in PSD-95^{-/-} mutants is not sensitive to MEK inhibitors, and that MEK and MAPK phosphorylation might not be increased. As reported previously (Winder, D. G. et al. Neuron 24, 715-26, 1999; Moser, E., et al., J Neurosci 13, 3916-25 1993), the MEK inhibitor U0126 (20 µM) strongly suppressed the induction of LTP by a brief train of 5 Hz stimulation in wild type slices (Fig. 15c), indicating that MAPK activation is normally required for the induction of LTP by this pattern of synaptic stimulation. In contrast, U0126 had no effect on the induction of LTP by 5 Hz stimulation in PSD-95 mutant slices (Fig. 15d), suggesting that the induction of LTP in PSD-95 mutant slices is MAPK-independent. In addition, levels of phosphorylated MEK and MAPK were not elevated above wild type levels in PSD-95 mutant mice (Fig. 15f). These data support the model that PSD-95 is upstream of a MAPK-independent pathway as well as a SynGAP-MAPK pathway (Fig. 17).

### The in vivo role of H-Ras in SynGAP signalling

The availability of H-Ras and SynGAP mutant mice enables us to test if H-Ras is involved in SynGAP signalling in vivo. Based on in vitro experiments that show SynGAP accelerates GTP hydrolysis of H-Ras (Kim et al, *supra*; Chen et al, *supra*), the reduced LTP in SynGAP^{-/+} mice might result from increased H-Ras activity. Similarly, an increased H-Ras activity might be expected to drive the raf, MEK, MAPK activation observed in SynGAP^{-/+} mutant mice. To test these predictions we generated SynGAP^{-/+}/H-Ras^{-/-} double mutant mice and asked if LTP or MEK and MAPK activity was restored to wild type levels. Although the induction of LTP by high-frequency synaptic stimulation is reported to be enhanced in H-Ras^{-/-} mice (Manabe, T. et al. J Neurosci 20, 2504-11, 2000), we found that the amount of LTP induced by a low-frequency stimulation protocol was similar in slices from H-Ras^{-/-} and wild-type mice (Fig 15e). Compared to wild-type mice, significantly less LTP was induced by this 5 Hz stimulation protocol in SynGAP^{-/+} mice (p < 0.02, Fig. 15e). Moreover, 5 Hz stimulation-induced LTP was also significantly reduced (p < 0.05) in SynGAP^{-/+}/H-Ras^{-/-} double mutant mice compared to that seen in H-Ras^{-/-} mutant mice (Fig. 15e). This indicates that H-Ras is not a physiologically significant effector of SynGAP under these conditions. We also tested if H-Ras was required for SynGAP mediated MAPK pathway activation by immunoblotting extracts from H-Ras^{-/-} and SynGAP^{-/+}/H-Ras^{-/-} double mutant mice (Fig. 15b). Levels of MEK and MAPK phosphorylation in H-Ras^{-/-} mice were the same as wild type. In contrast, there was a clear increase in phosphorylated MEK and MAPK levels in SynGAP^{-/+}H-Ras^{-/-} double mutant mice, similar to that observed in SynGAP^{-/+} mutants. Thus, like our results showing that H-Ras is not required for SynGAP regulation of LTP, these results indicate that H-Ras is not an essential effector in SynGAP regulation of MAPK pathway activation.

### SynGAP regulates rate of learning

The genetic dissection of multiple signalling pathways controlling synaptic plasticity within the complex of NMDA receptor associated proteins allows a reexamination of the involvement of this receptor in learning. SynGAP^{-/+} mutants, like PSD-95^{-/-} mutant mice (Migaud et al, *supra*), show no detectable abnormalities in synaptic NMDA receptor currents despite an opposite change in NMDA receptor mediated synaptic plasticity. PSD-95^{-/-} mutants show a severe impairment in the performance of a spatial learning task (Migaud et al, *supra*). The SynGAP^{-/+} mutant mice allow us to dissect the contribution of this protein to one of the PSD-95 associated signalling pathways. To afford a comparison, we deliberately chose an identical watermaze training protocol to that used in the study of PSD-95^{-/-} mutant mice. Both wild type (n = 21) and heterozygous SynGAP^{-/+} mutants (n = 21) were trained in cued and spatial versions of the watermaze, with the experimenter blind to genotype throughout.

In the NMDA receptor-independent cue task, both wild type and SynGAP^{-/+} mutant mice learned to approach a randomly located platform marked by a visible cue with an equivalent (F < 1) and progressive reduction in path length over successive trials (Fig. 16a). In the NMDA receptor-dependent spatial task, both groups showed a decrease in path length over the course of 20 trials (mutants: F(19, 380) = 1.91; p < 0.05; wild types: F(19, 380) = 2.74; p < 0.001), with the swim paths of SynGAP^{-/+} mutants being slightly longer overall than those of wild types (F(1,40) = 6.75; p < 0.05). All mice were then subject to the first transfer test (platform removed). On the conventional measure of time in the training quadrant, which revealed a striking impairment in PSD-95^{-/-} mutants (Fig. 5d in Migaud et al., *supra),* the difference between SynGAP^{-/+} mutants and wild types approached but did not reach significance (t(40) = 1.98; 0.10 > p > 0.05). However, using the more sensitive measure of the proportion of time spent in a zone centred around the target platform relative to time in all 4 possible platform locations (Moser et al, *supra*), the SynGAP^{-/+} mutants showed significantly less focused swimming (Fig. 16c; t(40) = 3.08; p < 0.005). When PSD-95^{-/-} mutants were given overtraining on the spatial task to reach a strict performance criterion, only 2/12 mutant mice were successful compared to 9/9 wild types (#10; χ² = 14.3; p < 0.001). In contrast, 11/21 SynGAP^{-/+} mice (compared to 20/21 wild types) reached this criterion. Although worse than wild types (χ² = 9.98; p < 0.005), significantly more SynGAP^{-/+} mice reached criterion than PSD-95^{-/-} mice (χ² = 5.22; p < 0.05). Consistent with this result, when tested in the second transfer test immediately after reaching criterion, SynGAP^{-/+} mice showed moderately focused searching in the target zone (Fig 16c). This result contrasts with the performance of PSD-95^{-/-} mutants, who remained at chance in both transfer tests (Fig. 16e). Comparison of the two mutant strains revealed that SynGAP^{-/+} mice were significantly less impaired that PSD-95^{-/-} mice (F(1,31) = 5.31; p < 0.05), and a group x transfer test interaction was obtained (F(1,31) = 5.10; p < 0.05): SynGAP^{-/+} and PSD-95^{-/-} mice performed equally badly on transfer test 1, but SynGAP mice performed significantly better than PSD-95^{-/-} mice on transfer test 2 (p < 0.05).

Thus, as also displayed qualitatively in the representative swim paths (Fig 16d), SynGAP^{-/+} mice showed a slower rate of spatial learning characterized by more diffuse searching early in training (transfer test 1), but they were able to locate the platform after further training (transfer test 2); PSD-95^{-/-} mice searched aberrantly throughout all phases of spatial training (Fig. 16e,f; re-analysis of data from Migaud et al). A framework for thinking about these mutants is shown in figure 16g in which SynGAP^{-/+} mutants gradually approach an equivalent asymptote of learning to that reached by control animals, while PSD-95^{-/-} mice do not.

In contrast to their reduced rate of learning, the subset of SynGAP^{-/+} mutants tested (n = 12) showed a normal rate of forgetting relative to wild types (n = 12). Time spent in the target zone (see methods) was similar in both groups on transfer test 3 (7 day retention test; mutants: 52.10 ± 8.15 %; wild types: 60.66 ± 7.43 %) and transfer test 4 (45 day retention test; mutants: 35.34 ± 5.21 %; wild types: 36.70 ± 4.21 %). An ANOVA of performance on transfer tests 2, 3 and 4 (i.e. both immediate and long-term retention tests) revealed no overall group difference between mutants and wild types (F < 1), and no group x transfer test interaction (F < 1), indicating that SynGAP^{-/+} mutants, although initially slower to learn, have unimpaired long-term memory. As expected, significant forgetting was observed over time (F(2,44) = 12.46; p < 0.001, but performance remained above chance even in the 45 day retention test (t(23) = 3.36; p < 0.005). This finding is consistent with the notion that SynGAP contributes to the induction machinery for memory encoding, but not to the persistence of memory traces over time.

### Discussion

In this study, we examine mice carrying mutations in SynGAP, H-Ras and PSD-95, which are components of a 2000kDa intracellular signalling complex linked to the NMDA receptor isolated in Example 1 (Figure 17). We find that SynGAP was essential for NMDAR mediated synaptic plasticity and learning although it was not required for synaptic NMDAR function. SynGAP was a key negative regulator of a pathway from raf, MEK to MAPK, which are also components of the complex. PSD-95, which directly binds the NMDAR, plays a pivotal role in bi-directional plasticity induction by linking two pathways to the NMDAR, one being the SynGAP-MAPK pathway and the second a MAPK-independent pathway (Fig. 17). Although SynGAP mutant mice showed a slower acquisition of learning they showed normal memory retention and rates of forgetting.

### LTP induction controlled by intracomplex signalling pathways

While excitatory synapses in SynGAP^{-/+} mutants and wild-type mice showed similar responses to patterns off synaptic activation that were below threshold for LTP induction, significantly less potentiation was induced in SynGAP^{-/+} mutant slices by all patterns of synaptic stimulation that exceeded the threshold for LTP induction in wild-type slices. This LTP deficit in SynGAP^{-/+} mice occurs in the absence of any obvious changes in basal synaptic transmission and NMDA receptor function and it is present under experimental conditions where potential changes in inhibitory synaptic transmission and/or postsynaptic excitability should have little effect on LTP induction. Together, these results suggest that SynGAP has an important role in coupling NMDA receptor activation to downstream signalling pathways involved in LTP. Previous studies have suggested that SynGAP may act to couple increases in intracellular calcium to activation of the RAS/MAPK pathway (Kim et al, *supra*; Chen et al, *supra*), a pathway known to have an important role in both LTP and learning (reviewed in Impey, S., et al. Neuron 23, 11-4, 1999). Thus, by virtue of its association with PSD-95 and its potential regulation by increases in intracellular calcium (either through its N-terminal C2 domain or changes in activity following phosphorylation by the calcium/calmodulin-dependent kinase CamKII), SynGAP may provide an essential link whereby NMDA receptor activation leads to activation of the MAPK pathway. The genetic analysis of single and double mutant mice supports the view that PSD-95 is upstream of SynGAP, which together with biochemical and pharmacological studies maps out a pathway: NMDAR → PSD-95 → SynGAP → raf → MEK→ MAPK.

We have excluded H-Ras from this pathway, because H-Ras was not required for SynGAP mediated changes in LTP or MEK/MAPK activation. Although the link from SynGAP to raf, MEK and MAPK is currently unknown, it is likely that it is another Ras family member, which are known to be expressed in the hippocampus (Manabe et al, *supra*) and Fig.12d. Interestingly, while the induction of LTP by high-frequency stimulation (100 Hz) is enhanced in H-Ras mutant mice (Manabe et al, *supra)* we found that low-frequency (5 Hz) stimulation-induced LTP is normal in these mice. This suggests that the involvement of H-Ras-dependent signalling pathways in LTP may be highly dependent on the pattern of synaptic stimulation used to induce LTP, a phenomenon reminiscent of the role of protein kinase A in LTP of excitatory synapses in the hippocampal CA1 region (Thomas, M. J., et al Neuron 17, 475-82, 1996); Blitzer, R. D., et al., Neuron 15, 1403-14, 1995; Abel, T. et al, Cell 88, 615-26, 1997).

The normal physiological role of the SynGAP-MAPK pathway appears to be in modulating synaptic plasticity and learning as suggested by the SynGAP mutation and studies with MEK inhibitors. However, studies using pharmacological approaches have found that inhibiting MEK suppresses LTP in wild type slices, while we find that an increase in basal levels of activated MEK and MAPK in SynGAP mutants is also associated with reduced LTP. One possibility is that while basal levels of MAPK pathway activation are increased in SynGAP^{-/+} mice, LTP may be compromised because activation of the MAPK pathway is no longer properly regulated following NMDA receptor activation. Alternatively, chronic up-regulation of the MAPK pathway in the mutant may lead to down-regulation of downstream targets involved in the induction and/or expression of LTP. Clearly, a resolution of this paradox will require a better understanding of the role of MAPK signalling in LTP induction, especially with respect to identification of the downstream targets involved in LTP, as well as a quantitative comparison of MAPK activation following NMDAR activation in mutant and wild-type hippocampus.

Although PSD-95 has been identified as an important adaptor protein that links SynGAP to NMDARs, the dramatically different phenotypes of PSD-95 and SynGAP mutant mice seen in our experiments indicate that PSD-95 most likely serves as a multifunctional adaptor protein that links multiple signalling pathways to the NMDAR (Figure 17). These pathways can be divided into a MAPK-dependent component, where SynGAP/PSD-95 interactions link NMDAR to activation of the MAPK pathway, and a second, SynGAP/MAPK-independent pathway that when disrupted in PSD-95 mutants results in a dramatic facilitation of LTP. Although the signalling molecules that comprise this second pathway have not yet been identified, the enhancement of LTP in PSD-95 mutants suggests that this pathway normally acts to inhibit or suppress the induction of LTP. Based on our finding that the enhanced LTP observed in PSD-95 mutants is not suppressed by MEK inhibitors, mutations in PSD-95 appear to uncouple both of these pathways, resulting in an enhanced, MAPK-independent LTP. This further suggests that the MAPK pathway is not required for the induction of LTP per se but instead may serve a more modulatory role in LTP induction (see also Watabe, A. M., et al., J Neurosci 20, 5924-31, 2000 and Winder, D. G. et al. Neuron 24, 715-26, 1999).

What is the physiological significance of interacting LTP enhancing and LTP suppressing pathways co-localized within a single signalling complex? One likely possibility is that it allows rapid detection of highly localized NMDAR-dependent increase in intracellular calcium and provides the signalling machinery that enables the magnitude and/or duration of the calcium transients to induce long-lasting changes in synaptic strength and other manifestations of neural plasticity. Indeed, the results from our studies of SynGAP and PSD-95 mutant mice show that genetic manipulations of the composition of the NMDA receptor signalling complex that have no effect on synaptic NMDAR currents can, nonetheless, profoundly alter how synapses respond to a particular frequency of synaptic activation. This indicates that some aspects of the frequency-dependent characteristics of LTP arise from the actions and interactions of signalling pathways that reside within the multi-protein complex.

The notion that multiple signalling mechanisms reside within protein complexes linked to receptors is well accepted in cell biology and extends to most receptors. These include growth factor (tyrosine kinase) receptors, T-cell receptors and more recently the TRP calcium channel, which is involved in Drosophila phototransduction. The complex assembled with TRP, known as the 'signalplex' (Montell, C. Annu Rev Cell Dev Biol 15, 231-68, 1999) or 'transducisome' (Tsunoda, S. et al. Nature 388, 243-9, 1997), requires the PDZ containing protein INAD to organize multiple signalling enzymes (PKC and phospholipase C) and the light-activated TRP channels. Disruption of these components leads to altered physiological responses in an analogous manner to those observed in our studies of multi-protein complexes. Recent evidence indicating that cytoplasmic components of signalling complexes can assemble prior to interaction with the ion channel (Tsunoda, S., et al. J Neurosci 21, 150-158, 2001) raises the possibility that the intracellular complex may be the key signalling machinery rather than the receptor, and its specificity to signals from the external environment is obtained by insertion of a specific receptor. This kind of model would accommodate the view that different neurotransmitter receptors can bind to PSD-95 and other multi-protein complex adaptor proteins, and generate an assortment of distinct complexes conferring plasticity responses on different synapses driven by different neurotransmitters. In addition to a role in signal transduction, receptor-complexes participate in regulating turnover of ligand-gated ion channels and modulation of their properties. For example, PKA is recruited to NMDAR to modulate channel properties (Westphal, R. S. et al. Science 285, 93-6, 1999) and disruption of NSF interactions with AMPA receptors alters their surface expression (Nishimune, A. et al. Neuron 21, 87-97, 1998; Song, I. et al. Neuron 21, 393-400, 1998).

### Learning and Hebbian plasticity

Hebb's postulate (see Hebb, D. O. The organization of behavior; a neuropsychological theory, Wiley, New York, 1949) that repeated firing of a neuron by another neuron leads to a stable increase in efficacy of activation between the neurons, and that this process underlies learning, has been supported by many electrophysiological and psychological studies in invertebrates and vertebrates (reviewed in Bailey, C. H., et al., Adv Second Messenger Phosphoprotein Res 29, 529-44, 1994; Martin, S. J., et al., Annu Rev Neurosci 23, 649-711, 2000). In the mammalian hippocampus, a structure required for spatial learning in rodents and humans, pharmacological blockade of NMDAR channels interferes with both learning and LTP Collingridge, G. L., et al. J Physiol (Lond) 334, 33-46, 1983; Morris, R. G., et al., Nature 319, 774-6, 1986). The large number of proteins in the complexes required for normal learning and synaptic plasticity indicates the complexes provide a molecular basis for the Hebbian properties of these synapses. However, comparison of the LTP and spatial learning phenotypes of SynGAP and PSD-95 mutants presents a departure from some aspects of the Hebb model. The SynGAP mutant mice show reduced LTP with an effect on the rate of spatial learning but not the eventual asymptote reached, whereas the PSD-95 mutants show enhanced LTP with severely impaired spatial learning. Thus two interacting proteins bound to the NMDAR, with similar patterns of expression in the hippocampus and central nervous system, appear to dissociate a simple correlation between LTP and learning.

On the one hand, this raises the possibility that some pathways within the complex play a more significant role in plasticity while others are involved primarily in learning, or on the other hand, the complex may control other features of synaptic plasticity and/or metaplasticity that are more relevant to spatial learning than the overall magnitude of LTP. For instance, signalling events controlled by the complex may have an important role in generating the synaptic "rules" (such as frequency sensitivity, EPSP/spike timing, etc.) that govern how specific patterns of synaptic activity are converted into persistent changes in synaptic strength and changes in these parameters may be more relevant to how well information is stored during learning.

Both SynGAP and PSD-95 mutant mice exhibit a deficit in spatial learning. This observation is in keeping with at least 16 other genetic and pharmacological perturbations in components of the multi-protein complex that also result in learning impairments. However, the fact that this complex is critically involved in the encoding of spatial memories does not necessarily imply an involvement in additional memory processes such as retrieval. Owing to the severity of the behavioural impairment observed in PSD-95 mutant mice, it was impossible to assess the role of this protein in separate memory processes. SynGAP mice, on the other hand, although slower to learn than wild types, were eventually able to encode the platform location. In contrast to their acquisition deficit, SynGAP mice exhibited normal long-term retention of spatial information, with equivalent forgetting rates in both mutants and wild types. It is therefore unlikely that SynGAP plays any significant role in the retrieval of spatial memories. This finding is consistent with previous work involving the pharmacological blockade of NMDA receptors. The administration of AP5 in rats has no effect on the retention of a previously learned olfactory discrimination (Staubli, U., et al., Behav Neurosci 103, 54-60, 1989) or watermaze task (Morris, R. G., J Neurosci 9, 3040-57, 1989; Steele, R. J. & Morris, R. G., Hippocampus 9, 118-36, 1999), although new learning is severely impaired in both types of task. Data such as these indicate that the multi-protein complex comprising the NMDA receptor is primarily responsible for detecting the patterns of neural activity that are salient for learning, and then triggering the cellular changes (LTP, LTD, gene transcription etc.) that together change the state of neurons and circuits. The subsequent readout of information - memory retrieval - does not normally require further activation of the complex, although memory reactivation may sometimes involve new learning Nader, K., et al., Nature 406, 722-6 (2000).

In humans and in mice, disruption of the neurofibromin (NF-1) gene results in learning impairments (Ozonoff, S., Am J Med Genet 89, 45-52, 1999; Silva, A. J. et al. Nat Genet 15, 281-4, 1997). NF-1 is also a GAP protein and shares structural similarity to SynGAP. We previously reported that both NF-1 and SynGAP were components of multi-protein complexes and suggested that NF-1 mutations may result in learning deficits through its function in this complex. Comparison of physiological and biochemical properties of NF-1 and SynGAP mutant mice will be necessary to address the possible overlap in functions of these two GAP proteins.

In conclusion, this study shows that distinct signalling pathways linked via PSD-95 to the NMDAR control the physiological properties of synaptic plasticity and learning. Importantly, these pathways are contained within a 2000kD complex of proteins. Our data show this complex acts as a signal integration device and processes signals underlying the cellular basis of learning.

### EXAMPLE 5: INVESTIGATION OF THE ROLE OF THE MULTI-PROTEIN COMPLEX IN CHRONIC PAIN BEHAVIOUR

### Introduction

Glutamate receptors in the spinal cord play a key role in the processing of somatosensory inputs (L. Urban, S. W. Thompson, A. Dray, Trends Neurosci. 17, 432-438, 1994). In particular, NMDA receptors are implicated in the central sensitisation and pain caused by repetitive afferent stimulation or injury to peripheral tissues or nerves (T. J. Coderre, R. Melzack, Journal of Neuroscience 12, 3665-3670, 1992; S. R. Chaplan, A. B. Malmberg, T. L. Yaksh, Journal of Pharmacology and Experimental Therapeutics 280, 829-838, 1997; J.E. Haley, A. Sullivan, A. Dickenson, Brain Research 518, 218-226, 1990; C. J. Woolf, M. Costigan, Proc.Natl.Acad.Sci. U.S.A 96, 7723-7730, 1999). Neuropathic pain is inadequately treated by current analgesics (S. Arner, B. A. Meyerson, Pain 33, 11-23,1988), and involves multiple changes in phenotype and function of afferent and central neurons (T. Hökfelt, X. Zhang, Z. Wiesenfeld-Hallin, Trends Neurosci. 17, 22-30, 1994; T. Dickinson, S. M. Fleetwood-Walker, Trends Pharmacol.Sci. 20, 324-329, 1999; D. J. Mayer, J. Mao, J. Holt, D. D. Price, Proc.Natl.Acad.Sci. U.S.A 96, 7731-7736, 1999), yet as in other chronic pain states, the NMDA receptor appears to be crucial (S. R. Chaplan, A. B. Malmberg, T. L. Yaksh, Journal of Pharmacology and Experimental Therapeutics 280, 829-838, 1997; M. Tal, G. J. Bennett, Neuroscience Letters 151, 107-110, 1993; J. Mao et al., Brain Res. 605, 164-168, 1993; T. R. Tölle, A. Berthele, W. Zieglgänsberger, P. H. Seeburg, W. Wisden, Journal of Neuroscience 13, 5009-5028, 1993). The NMDA receptor consists of two main subunits, NR1 and NR2, which are expressed throughout the dorsal horn of the spinal cord (T. R. Tölle, A. Berthele, W. Zieglgänsberger, P. H. Seeburg, W. Wisden, Journal of Neuroscience 13, 5009-5028, 1993; J. M. Luque, Z. Bleuel, P. Malherbe, J. G. Richards, Neuroscience 63, 629-635, 1994). While the NR1 subunit is the key functional subunit, NR2 subunits provide docking sites for intracellular adapter and regulatory proteins (H.C. Kornau, L.T. Shenker, M.B. Kennedy, H. Seeburg, Science 269, 1737-1740, 1995; M. Niethammer, E. Kim, M. Sheng, Journal of Neuroscience 16, 2157-2163, 1996).

NR2 C-terminus motifs are targeted by PDZ domain adapter proteins of the MAGUK family (H.C. Kornau, L.T. Shenker, M.B. Kennedy, H. Seeburg, Science 269, 1737-1740, 1995; M. Niethammer, E. Kim, M. Sheng, Journal of Neuroscience 16, 2157-2163, 1996; Example 1), the best characterised of which is PSD-95. Specifically, the first two PDZ domains of PSD-95 have been shown to interact with the final 4-6 amino acid residues of NR2 subunit C-termini. The MAGUK proteins are believed to cluster the receptor at the synaptic membrane and to act as a bridge for signalling to downstream pathways (see Example 1; M. Niethammer, E. Kim, M. Sheng, Journal of Neuroscience 16, 2157-2163, 1996; S.E. Craven & D.S. Bredt, Cell 93, 495-498, 1998). Here we have addressed the possibility that neuropathic sensitisation may depend on adapter-mediated mechanisms of NMDA receptor function.

Using transgenic mice expressing a mutant PSD-95 gene truncated beyond the second PDZ domain (M. Migaud et al., Nature 396, 433-439, 1998), we show that PSD-95 is expressed in the spinal dorsal horn and that PSD-95 mutants display almost complete absence of the NMDA receptor-dependent neuropathic pain behaviours seen in wild type controls. Utilising constitutive activity of CaM kinase II to monitor Ca²⁺ elevation responses in dorsal horn, we show that while PSD-95 mutant mice show normal acute responses to NMDA, the enhancement of NMDA-dependent responses seen normally in nerve injured wild type mice is abrogated in mutant animals. Correspondingly, injury-induced phosphorylation of spinal NR1 subunits is strongly attenuated in PSD-95 mutants. MAGUK-mediated interactions leading to modulation of NMDA receptor function by phosphorylation, appear crucial to the mechanisms underlying neuropathic pain.

### Materials and Methods

### Molecular composition of NMDA receptor complexes in spinal cord

Spinal cords from wild type mice were homogenised at 4°C in 50 mM Tris pH 9.0, with 1% Na deoxycholate, 50 mM NaF, 20µM ZnCl₂, 1mM Na orthovanadate, 0.5 mM PMSF, 2 µg/ml aprotinin and 2 µg/ml leupeptin. The extract (0.5 ml) was incubated for 1 hour on ice and clarified by centrifugation at 13, 000 x g for 1 hour at 4°C. As described previously [H.Husi et al., Nature Neuroscience 3, 661 (2000)], MAP-NR1 IgG, PSD-95 IgG, NR2A and NR2B or NR2B 6aa C-terminus were incubated with extract for 4 hours at 4°C. Protein G Sepharose (15 µl) was subsequently added and the samples were kept under constant agitation overnight at 4°C. Samples were washed five times with 0.5 ml cold extraction buffer before solubilisation in Laemmli buffer. Protein samples were subjected to reducing SDS-PAGE and transferred to PVDF membrane at 4°C for 90 minutes at 75 V in 10% (v/v) methanol, 10 mM CAPS pH 11.0. Primary antibodies used to probe blots were as described previously (see Example 1) and signal detection was carried out using peroxidase-linked secondary antibody enhanced chemiluminescence. Direct immunoblots of 100µg spinal cord and 50 µg forebrain extract are shown for comparison.

### Ex vivo CaM kinase II activity assays

Following lumbar L3-6 laminectomy under anaesthesia, mice, which in some cases had previously received chronic constriction injury, were treated by topical application of agents to the dorsal surface of the spinal cord. NMDA (500 µM) with the co-agonist glycine (100 µM), ionomycin (10 µM) or saline vehicle were applied in a volume of 500µl for 15 minutes before rapid removal to cold buffer on ice. Constitutive (autophosphorylated) and Ca²⁺ /CM-elicited activity of CaM II kinase [P.I. Hanson et al., Neuron 3, 59 (1989); P.M. Jones and S.J. Persaud, Am. J. Physiol. 274, E708 (1998)] were measured by the following procedure. Samples were rapidly homogenised on ice in 20 mM Na HEPES pH 7.5 with 5% glycerol, 1mM EGTA, 1mM dithiothreitol, 1mM AEBSF, 2 µg.ml⁻¹ aprotinin, 10 µg.ml⁻¹ leupeptin, 2 µg.ml⁻¹ pepstatin, 50 µg.ml⁻¹ soybean trypsin inhibitor, 25 mM Na β-glycerophosphate, 1mM Na orthovanadate, 1mM Na F, 1 µM calyculin A, 1 µM cypermethrin. Following a 30 min incubation on ice with 0.25% CHAPS and clarification, samples were incubated at 4°C for 2 hours with 3 µg.ml⁻¹ mouse anti-α-CaM kinase II IgG (clone CBα-2; Zymed) and then with excess Protein G-Sepharose for 1 hour. Aliquots of washed immunoprecipitate were incubated for 20 min at 30°C (linear range of assay) with 50 µM autocamtide-2, 50 µM ATP (with [³³P] γ-ATP to 0.25 µCi/tube), 10 mM MgCl₂ and 8 µg.ml⁻¹ purified bovine calmodulin (Sigma), in the presence of 0.5 µM PKC-α19-31 and 0.5 µM PKI₆₋₂₂ amide (to suppress any PKC and PKA-mediated activity respectively) before termination with cold TCA (to 10%), centrifugation and spotting of the supernatant onto P81 phospho-cellulose paper.

Samples were washed extensively in 75 mM H₃PO₄ and dried before scintillation counting. Constitutive and maximal Ca²⁺-evoked activity were measured in the absence and presence of 2 mM CaCl₂ respectively. Zero time and substrate-free blanks were less than 2 % of maximal activity. Parallel immunoblotting experiments were attempted with phospho-specific antisera for Thr²⁸⁶-CaM kinase II (the key autophosphorylation site), but gave insufficient signal:noise ratios for further use.

### Immunoblotting for phospho-NR1

Spinal cord samples were initially homogenised in the buffer as for CaM kinase II assays (without CHAPS) and a membrane fraction prepared. This was solubilised in 1% deoxycholate-containing buffer for 1 hour at 4°C and then the clarified supernatant was incubated for 4 hours at 4°C with 3.5 µg/ml of goat anti-NR1 IgG, (SC-1467, Santa Cruz) before precipitation with Protein G Sepharose CL-4B for 1 hour at 4°C. After washing, bead-attached proteins were solubilised in Laemmli buffer before SDS-PAGE on Immobilin P (Millipore) membranes and detected by peroxidase-linked secondary antibody enhanced chemiluminescence.
Blots were probed with pan-NR1 (Chemicon, 1:100), phospho-Ser⁸⁹⁷-NR1 (Upstate Biotech, 1:100). The phospho-Ser⁸⁹⁷ antibody specifically detects post-translational modification of NR1 at a site shown to be targeted by PKA, although it has not been explicitly shown that this is the only kinase capable of phosphorylating Ser⁸⁹⁷-NR1 [W.G. Tingley et al., J.Biol. Chem., 272, 5157 (1997); X. Zhou et al., J. Neurosci., 20, 6989 (2000)].

### Peripheral Nerve Morphology:

### Myelinated Fibres

1 µm transverse sections of sciatic nerve (tibial branch) stained with toluidine blue were examined using '*Image 1. 44*' software (NIH). The cross sectional area of the tibial nerve was measured at x100 magnification, in 5 randomly generated regions on each nerve section were chosen using a 4x4 grid. For analysis, 30 myelinated fibres closest to the centre of the screen were selected and the axon areas, external areas, and G ratios (axon diameter / external diameter) were measured to quantity myelin thickness.

### Unmyelinated Fibres

80nm transverse sections of the sciatic nerve were mounted onto copper slot grids and examined in a transmission electron microscope. 25 random areas were photographed at 2300x magnification and then printed at a final magnification of x5750, which was used to count all the myelinated and unmyelinated fibres. For analysis, five negatives were chosen at random and scanned using a flat bed scanner for computer analysis using *'Image 1.44'* .The axon diameters/areas of 30 unmyelinated fibres were measured from each of the micrographs, the fibres being selected from clusters consisting solely of unmyelinated fibres. The frequency of myelinated fibres was scored at 1 µm intervals from 1-10 µm and that of unmyelinated fibres at 0.1 µm intervals from 0.1 - 2.7 µm. Totals of 827 and 759 myelinated fibres were scored from tibial nerves of wild type and PSD-95 mutant mice, giving median diameters of 2.4 ± 1.3 and 3.5 ± 1.9 respectively (median ± S.D.). Correspondingly, totals of 436 wild type and 479 PSD unmyelinated fibres, were scored, giving mean diameters of 0.56 ± 0.3 and 0.47 ± 0.5 µm respectively (median ± S.D.). Interval distributions were not significantly different between wild type and PSD-95 mutants (χ² tests) and all profiles were similar between wild type and mutant mice in accordance with previously published findings [G. Guilbaud et al., Pain 53, 147 (1993); C. Sommer et al., J. Neuropath. Exp. Neurol. 54, 635 (1995)].

### Behavioural assessment of neuropathic pain:

### Chronic Constriction Injury (CCI) model to sciatic nerve in mice

Animals were initially anaesthetised with sodium pentobarbital (Sagatal; 0.01ml/kg (1:4 in saline, i.p.; Rhône Mérieux, UK) and this was then supplemented with halothane O₂ (Zeneca, UK). Under aseptic conditions, using a variation of the method of Bennett and Xie for rat [G.J. Bennett and Y.K. Xie, Pain 33, 87 (1988)], the sciatic nerve was exposed at the mid-thigh level proximal to the sciatic trifurcation. Three chromic catgut ligatures (5.0; Ethicon, Edinburgh) separated by 1mm were tied loosely to constrict the tibial branch as viewed under 40x magnification. Vascular supply was not compromised. The overlying muscle and skin were sutured and the animals were placed in a recovery cage.

### Behavioural Tests

Animals were tested for 6 days prior to surgery in order to establish stable baseline levels for each test and then every one or two days post-operatively (PO). The responses were compared to the average of the pre-surgery values and the contralateral control. The animals were examined daily to ensure that no autotomy was present.

### Thermal Hyperalgesia

Thermal hyperalgesia was monitored using Hargreaves' Thermal Stimulator (Linton Instruments, USA). The stimulus was applied to the mid-plantar surface of the hind paw. The withdrawal response was characterised as a brief paw flick. The latency of withdrawal was recorded for the ipsilateral (injured) and the contralateral hindpaws. The means of between 5 and 10 readings were recorded at 3-5 min intervals to ensure no hypersensitivity to the test was established. Mean basal values for pre-operative paw withdrawal latency were typically around 5 sec.

### Mechanical Allodynia

Mechanical allodynia was measured as the withdrawal threshold to calibrated Semmes-Weinstein von Frey filaments (Stoelting, Wood Dale, IL) with a bending force of 0.22-84.96g. The mice were placed in a small rectangular cage. The filaments were applied to the mid-plantar surface of the hindpaw from beneath until the filament started to bend. This stimulus was repeated 8-10 times at 1-2 sec intervals for both the injured and uninjured hindpaws. The mean threshold required to elicit a withdrawal response was recorded. The filaments were applied in ascending order, and a response was characterised as a brief paw flick. The withdrawal threshold was defined from the lower of two consecutive von Frey filaments that elicited a response. Data were expressed as the threshold indentation pressure (i.e. the bending force/the cross-sectional area at the tip of the filament). Mean basal values for pre-operative paw withdrawal threshold were typically around 600 mN.mm⁻².

### Cold Allodynia

In order to test the presence of cold allodynia, mice were placed in a circular glass tank with an elevated metal grid floor. This was covered (^{~}1cm) with a mixture of ice and water (3-4°C), which ensured both the glabrous and hairy skin of the foot was covered with water. The mice were placed in the tank for a 30 sec period, the first 10 sec of which were to allow the animals to acclimatise. During the remaining 20 sec, measurements were made of the amount of time the animal elevated the treated paw above the water level. Behavioural testing was carried out using protocols that ensured the tester was blind to the genotype of the subject.

### Intrathecal Drug Treatment

Baseline measurements were recorded for thermal hyperalgesia (Hargreaves' test) and mechanical allodynia (von Frey filament test) in mice which had undergone the chronic constriction injury and were at the peak of neuropathy. Mice were anaesthetised with halothane and O₂. Drugs were injected intrathecally, in 10µl saline, at the L4 level of the spinal cord using a 25µl microsyringe [J.L. Hylden and G.L. Wilcox, Eur. J. Pharmacol., 67, 313 (1980)]. The experimenter was blind to the content of the injections so as to eliminate bias, and dye injections (Pontamine Sky Blue) were performed to determine the site of injection. 15 minutes following injection, mice were re-tested using the same procedures and measurements were taken to determine the effects of drug on hyperalgesia and allodynia. Animals were tested every 5 minutes until readings returned to baseline levels.

### Results

In heterozygous mutant mice, histochemical staining for β-galactosidase (a reporter gene inserted into the mutant PSD-95 construct) was specifically restricted to lamina I and IIo of the superficial spinal dorsal horn (Fig 18a-c). This expression extended throughout lumbar and thoracic spinal cord, but could not be detected in dorsal root entry zones (Fig 18d), DRG, dorsal roots or sciatic nerve (data not shown). Immunoaffinity separations of spinal multi-protein complexes comprising an NMDA receptor in wild type spinal cord (as described previously for forebrain (Example 1)) confirmed that spinal NR1, NR2A, NR2B and PSD-95-directed immunoprecipitates each contained associated NR1, NR2A NR2B and PSD-95 (Fig 18e).

Light and electron microscopic analyses of tibial sciatic nerve sections showed similar profiles of axon diameter and myelin thickness for myelinated (Aδ and Aβ) and unmyelinated (C) fibres between naive wild type and mutant mice that were in accordance with previous reports. Despite the absence of any detectable defect in afferents in the mutant mice, they showed almost complete lack of development of nociceptive behaviours that normally develop ipsilateral to a chronic constriction injury (CCI) of the sciatic nerve (Fig 19). For both mutant and wild type mice, the baseline values in thermal mechanical nociceptive paw withdrawal tests were consistent with previously reported values for mice (C.S. Gillespie et al., Neuron (2000) 26, 523-531). Locomotion and the ability to produce reflex motor responses under basal conditions appeared normal. Over several days following CCI, wild type mice developed marked ipsilateral, thermal hyperalgesia, mechanical allodynia (pain-like responses to low intensity stimuli) and cold allodynia (Fig 19a-c). All responses from the contralateral hind limb remained unchanged from basal values (with invariant null responses in the cold allodynia test). Neither thermal hyperalgesia nor mechanical allodynia could be detected at all in PSD-95 mutant mice (Fig 19a, b), while cold allodynia was severely attenuated, reaching statistical significance at only one time point (Fig 19c). All CCI-evoked changes had resolved spontaneously within 22-23 days.

Acute intrathecal injection of the selective NMDA receptor antagonist, (R)-CPP, completely reversed the thermal hyperalgesia and mechanical allodynia that developed ipsilateral to CCI in wild type mice with no effect on contralateral responses (Fig 20a,b). Correspondingly, the behavioural changes were bilaterally mimicked in naive wild type mice by intrathecal NMDA (Fig 20c,d). In striking contrast, behavioural responses of PSD-95 mutant mice were unaffected by either (R)-CPP or NMDA (Fig 20). The NMDA receptor-mediated events responsible for the neuropathic sensitisation of spinal sensory reflexes are therefore highly dependent on the presence of the intact PSD-95 protein.

To assess the cellular function of spinal NMDA receptors in these models, we monitored the activation state of CaM kinase II. This enzyme is associated with the postsynaptic density complex (M.B. Kennedy, Trends In Neurosciences 20, 264-268, 1997) (docking to NR2 subunits (F. Gardoni et al., J.Neurochem. 71, 1733-1741, 1998) and is converted to a constitutively active, autophosphorylated form following cellular Ca²⁺ elevation, such as would occur in NMDA receptor activation. A necessary role for CaM kinase II in neuropathic sensitisation was indicated by the reversal of thermal hyperalgesia and mechanical allodynia in wild type mice by the selective CaM kinase II inhibitors, myristoyl-autocamtide 2-related inhibitory peptide and KN93, but to a smaller extent by the less active analogue, KN92 (Figure 22). The proportion of immunoprecipitated CaM kinase II that was constitutively active was measured after topical application of agents to the dorsal spinal cord of wild type or PSD-95 mutant mice (Figure 23). Activation by NMDA (with co-agonist glycine) or ionomycin (Ca²⁺ ionophore) application was equivalent in naïve wild type and mutant mice, indicating that acute NMDA receptor-mediated Ca²⁺ entry was not perturbed in the mutant. Nevertheless, the elevated proportion of constitutive CaM kinase II activity that was seen ipsilateral (but not contralateral) to CCI in wild type mice was absent in PSD-95 mutants. This increment was entirely prevented by application of (R)-CPP. These findings indicate that while PSD-95 is essential for the facilitation of NMDA receptor-mediated Ca²⁺ entry following CCI, it is not necessary for acute Ca²⁺ entry responses to NMDA. Thus, additional adapter protein-mediated processes appear to be essential for the behavioural manifestation of NMDA receptor-mediated neuropathic sensitisation, and Ca²⁺ entry (reflected in blockade by CaM kinase II inhibitors) also appears to be necessary. However, since CaM kinase II responses to CCI, but not acute NMDA, fail in PSD-95 mutants, it seems likely that the critical factor in CCI-induced neuropathic pain is a facilitatory modulation of NMDA receptor Ca²⁺ entry function. The basis for such a modulation could be NMDA receptor phosphorylation by kinases (Tingley et al., J.Biol.Chem. 272, 5157-5166, 1997; Westphal et al., Science 285, 93-96, 1999), and the NMDA responses of spinal neurons are known to be facilitated by PKC and PKA (K. I. Rusin, P. D. Ryu, M. Randic, J.Neurophysiol. 68, 265-286, 1992). Figure 21 shows that phospho-NR1 immunoreactivity in spinal NR1 immunoprecipitates from wild type mice was specifically increased ipsilateral to CCI and that this response was greatly diminished in PSD-95 mutant mice. In the same samples, pan-NR1 immunoreactivity was relatively reduced ipsilateral to CCI, both in wild type and PSD-95 mutant mice. Recent evidence suggests that MAGUK proteins such as PSD-95 may act as linkers to AKAP scaffolding proteins which can position kinases in the proximity of cell surface target proteins such as the NMDA receptor (Colledge et al., Neuron 27, 107-119, 2000). Our findings here with PSD-95 mutant mice, indicate that adapter roles of PSD-95 play a crucial part in the development of neuropathic pain states by enabling mechanisms of NMDA receptor phosphorylation and its enhanced Ca²⁺ entry function.

### EXAMPLE 6: INVESTIGATION OF THE ROLE OF THE MULTI-PROTEIN COMPLEX IN STROKE

### Introduction

Stroke is the leading cause of adult disability and consumes approximately 5% of most developed countries health service budgets. There are no therapies presently available which protects brain tissue from ischaemic damage. Despite stroke resulting in one third of all deaths in the UK there are very few options for prevention and treatment. Because cerebral ischaemia results in a massive release of the excitatory neurotransmitter glutamate, there has been considerable interest by pharmaceutical companies in drugs that block glutamate receptors. Activation of the N-Methyl-D-aspartate (NMDA) subtype of glutamate receptor is known to trigger the pathogenic changes of cerebral ischaemia. Although the NMDA receptor or NMDA gated ion channels appeared to be was an attractive target for intervention, clinical trials have been disappointing, not only because of psychiatric side effects of receptor antagonists, but because the patient presents to hospitals at a time point after receptor activation and during progression of the disease.

Intracellular signalling pathways that act after the NMDA receptor is activated may present more rational targets. Studies of NMDA receptor signalling in synaptic plasticity and learning, show the signal transduction pathways downstream of the NMDA receptor are regulated by PSD-95, a protein that binds NMDA receptor subunits. These plasticity pathways act during the first hour following NMDA receptor stimulation, which is a time window when drugs could be administered to stroke patients. There is no evidence that PSD-95 modifies ischaemic brain damage stroke in animals.

### Methods and Results

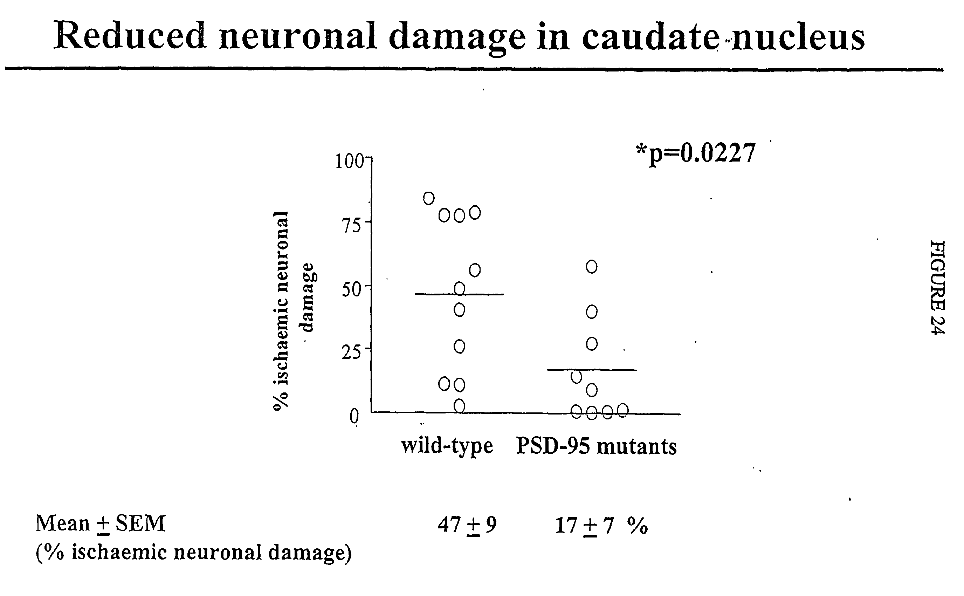

To address these issues we induced transient global ischaemia in PSD-95 mutant mice and examined the extent of brain damage. In a double blind study, bilateral carotid artery occlusion was applied to halothane-anaesthetised PSD-95-/- mutant (n=9) and wild-type littermate control (n=11) mice, which were sacrificed after 72hr and normal and ischaeamic damaged neurons counted using a 10mm² grid (Figure 24). The PSD-95-/mutant mice showed markedly less neuronal damage. In wild-type mice, neuronal damage was observed in 47 ± 9 % of caudate nucleus neurons and only 17 ± 7 % of PSD-95-/- mutant mice (p<0.02). There was also strong protection by the PSD-95 mutation seen in the hippocampus, where control mice showed damage to 11 ± 4 % of neurons and PSD-95-/- mice showed 2 ± 1 % damage (p=0.08) (Figure·25).

### Discussion

The protection against stroke brain damage by the PSD-95 mutation opens new avenues for drug intervention. PSD-95 and the NMDA receptor are components of a 2000kDa multiprotein signalling complex containing many potential drug targets. Pharmacological mimicry of the PSD-95 mutant phenotype using drugs against this complex may result in similarly dramatic reductions in brain damage following stroke or head injury.

## Claims

1. A multi-protein complex comprising the polypeptide subunits NR1, NR2A, NR2B, PSD-95 and at least three components selected from the group of components listed in Table 1 obtainable by peptide affinity chromatographic methods comprising the steps of:
(i) collecting a sample of tissue from the central nervous system of an animal; and
(ii) isolating the complex by contacting the sample with a peptide that binds to PSD-95,
wherein the peptide that binds to PSD-95 is SIESDV or
KRLLDAQRGSFPPWVQQTRV.

2. A complex according to Claim 1 wherein the complex comprises six or more components listed in Table 1.

3. A complex according to Claim 1 or 2 wherein the complex comprises component proteins that can be divided into five categories; neurotransmitter receptor proteins, cell adhesion proteins, adaptor proteins, signalling peptides and cytoskeletal proteins.

4. A complex according to any one of Claims 1 to 3 comprising all of the components listed in Tables 1 and 2.

5. A method of producing a multi-protein complex according to any one of Claims 1 to 4 comprising the steps of:
(i) providing a sample of tissue from the central nervous system of an animal; and
(ii) isolating the complex by contacting the sample with a peptide that binds to PSD-95, wherein the peptide that binds to PSD-95 is SIESDV or KRLLDAQRGSFPPWVQQTRV.

6. A complex according to any one of Claims 1 to 4 or a method according to Claim 5 wherein the sample of tissue is from the brain.

7. A complex or method according to Claim 6 wherein the sample of tissue is from the forebrain.

8. A complex according to any one of Claims 1 to 4, 6 or 7 or a method according to any one of Claims 5 to 7 wherein the sample of tissue is from central nervous system of an adult, juvenile or newborn mammal.

9. A method according to any one of Claims 5 to 8 further comprising the step (prior to step 1) of selecting the age of the mammal from which the tissue is taken.

10. A complex or method according to Claim 6 wherein the sample of tissue is human in origin.

11. A host cell containing one or more genetic constructs that encode a component of a multi-protein complex according to any one of Claims 1 to 4, wherein the component is selected from the group of components listed in Table 1.

12. A host cell according to Claim 11 containing one or more genetic constructs that encode four or more components selected from the group of components listed in Tables 1 and 2.

13. A host cell according to Claim 11 or 12 containing genetic constructs encoding six or more components listed in Tables 1 and 2.

14. A host cell according to any one of Claims 11 or 13 wherein the cell is a mammalian cell.

15. A host cell according to any one of Claims 11 or 14 wherein the cell is a neurone or a neurone-derived cell.

16. A sub-cellular fraction isolated from a host cell according to any one of Claims 11 or 15 that contains within it a multi-protein complex according to any one of Claims 1 to 4 or sub-complex thereof.

17. A subcellular fraction according to Claim 16 wherein the sub-cellular fraction is or comprises a plasma membrane.

18. A method of identifying a candidate compound for treating disorders and conditions associated with dysfunction of NMDA receptors in the central nervous system comprising:
(i) selecting a compound to be tested;
(ii) contacting the test compound with a multi-protein complex according to any one of Claims 1 to 4; and
(iii) determining whether the compound to be tested interacts with the complex;
wherein test compounds that interact with the complex are identified as candidate compounds for alleviating the symptoms of, preventing or slowing the onset of, and/or correcting the deficit(s) associated with disorders and conditions associated with dysfunction of NMDA receptors in the central nervous system.

19. A method of identifying a candidate compound for treating disorders and conditions associated with dysfunction of NMDA receptors in the central nervous system comprising:
(i) selecting a compound to be tested;
(ii) contacting the test compound with a component of a multi-protein complex according to any one of Claims 1 to 4, wherein the component is selected from the components listed in Table 1; and
(iii) determining whether the compound to be tested modulates a property of the component;
wherein test compounds that modulate a property of the component are identified as candidate compounds for alleviating the symptoms of, preventing or slowing the onset of, and/or correcting the deficit(s) associated with disorders and conditions associated with dysfunction of NMDA receptors in the central nervous system.

20. A method according to Claim 19 wherein step (iii) comprises determining whether the compound modulates the ability of the component to interact with one or more other components listed in Tables 1 or 2 or a sub-complex thereof.

21. A method according to any one of Claims 18 to 20 further comprising the step of testing the test compound in one or more screens for efficacy in the treatment of learning impairment, psychiatric disorders and/or neurological disorders.

22. A method of identifying a candidate compound for treating disorders and conditions associated with dysfunction of NMDA receptors in the central nervous system comprising:
(i) selecting a compound known to modulate a property of a component of a multi-protein complex according to any one of Claims 1 to 4, wherein the component is listed in Table 1; and
(ii) testing said compound in a screen for efficacy in the treatment of disorders and conditions associated with dysfunction of NMDA receptors in the central nervous system.

23. Use of a host cell according to any one of Claims 11 to 15 or a subcellular fraction according to Claim 16 or 17 in a method for identifying candidate compound for treating disorders and conditions associated with dysfunction of NMDA receptors in the central nervous system.

24. Use of a compound that modulates a property of one or more of the components of a multi-protein complex according to one of Claims 1 to 4, wherein the components are listed in Table 1 in the preparation of a medicament for the treatment of disorders and conditions associated with dysfunction of NMDA receptors in the central nervous system.

25. The use according to Claim 24 wherein the medicament is for treating learning impairment, psychiatric disorders (such as schizophrenia) and/or neurological disorders (such as stroke and/or epilepsy).

26. A method of diagnosing, prognosing and/or determining the susceptibility of a subject to disorders and conditions associated with dysfunction of NMDA receptors in the central nervous system, or a method of aiding such prognosis, diagnosis and/or determination, comprising the steps of:
(i) providing a sample containing nucleic acid from the subject to be diagnosed; and
(ii) contacting said nucleic acid from the subject with a nucleic acid which hybridises selectively to a nucleic acid encoding a component of a multi-protein complex according to any one of Claims 1 to 4, wherein the components are listed in Table 1, or a mutant allele thereof, or their complement.

27. A method according to any one of Claim 26 wherein the disorder or condition is selected from learning impairment, psychiatric disorders and/or neurological disorders.

28. A method according to Claim 26 or 27 wherein the nucleic acid encoding a component listed in Table 1 is a gene.

29. A method according to any one of Claims 26 to 28 further comprising the step of contacting nucleic acid from the subject with at least one additional nucleic acid which hybridises selectively to a nucleic acid encoding a component selected from the group of components listed in Tables 1 and 2, or a mutant allele thereof, or their complement, wherein each nucleic acid that hybridises to the nucleic acid from the subject encodes a different component.

30. A method according to any one of Claims 26 to 29 wherein the one or more nucleic acid which selectively hybridises to the nucleic acid encoding a component selected from group of components listed in Table 1, or a mutant allele thereof, or their complement further comprises a detectable moiety.

31. A method according to any one of Claims 26 to 30 wherein the one or more nucleic acid which selectively hybridises to the nucleic acid encoding a component selected from group of components listed in Table 1, or a mutant allele thereof, or their complement is single-stranded.

32. A method according to any one of Claims 26 to 31 wherein the one or more nucleic acid which selectively hybridises to the nucleic acid encoding a component selected from group of components listed in Table 1, or a mutant allele thereof, or their complement is less than 1000 base pairs (if said nucleic acid is double stranded) or bases (if said nucleic acid is single stranded), for example less than 100 base pairs or bases.

33. A method according to any one of Claims 26 to 32 further comprising the step of identifying in the nucleic acid from the patient the presence of a sequence variant of a nucleic acid encoding a component listed in Table 1 and comparing said sequence variant to sequences in equivalent nucleic acids in individuals from one or more test populations.

34. A method according to Claim 33 wherein the one or more test populations comprise a population of individuals who do not suffer and have not suffered previously from to disorders and conditions associated with dysfunction of NMDA receptors in the central nervous system.

35. A method of diagnosing, prognosing and/or determining the susceptibility of a subject to disorders and conditions associated with dysfunction of NMDA receptors in the central nervous system, comprising the steps of:
(i) providing a sample containing antibodies from the subject to be tested; and
(ii) determining whether said sample contains antibodies reactive with a multi-protein complex according to any one of Claims 1 to 4.

36. A kit of parts useful for diagnosing, prognosing or determining the susceptibility of a patient to disorders and conditions associated with dysfunction of NMDA receptors in the central nervous system comprising a nucleic acid that selectively hybridises to a nucleic acid encoding a component of a multi-protein complex according to any one of Claims 1 to 4, wherein the components are listed in Table 1, together with enzymes and reagents for promoting nucleic acid-nucleic acid hybridisation and for detecting the hybrids formed thereby.

## Patentansprüche

1. Multiproteinkomplex, der die Polypeptiduntereinheiten NR1, NR2A, NR2B, PSD-95 und wenigstens drei Komponenten, die aus der Gruppe von Komponenten ausgewählt sind, die in der Tabelle 1 aufgeführt sind, umfasst, der über Verfahren einer Peptid-Affinitätschromatografie erhalten werden kann, die die folgenden Schritte umfassen:
i) Gewinnen einer Gewebeprobe aus dem Zentralnervensystem eines Tieres und
ii) Isolieren des Komplexes durch das In-Kontakt-Bringen der Probe mit einem Peptid, das an PSD-95 bindet,
wobei das Peptid, das an PSD-95 bindet, SIESDV oder KRLLDAQRGSFPPWVQQTRV ist.

2. Komplex gemäß Anspruch 1, wobei der Komplex sechs oder mehr der in der Tabelle 1 aufgeführten Komponenten umfasst.

3. Komplex gemäß Anspruch 1 oder 2, wobei der Komplex Proteinkomponenten umfasst, die in fünf Kategorien eingeteilt werden können, nämlich Rezeptorproteine für Neurotransmitter, Zelladhäsionsproteine, Adapterproteine, Signalpeptide und Zytoskelettproteine.

4. Komplex gemäß einem beliebigen der Ansprüche 1 bis 3, der alle der in den Tabellen 1 und 2 aufgeführten Komponenten umfasst.

5. Verfahren zur Erzeugung eines Multiproteinkomplexes gemäß einem beliebigen der Ansprüche 1 bis 4, das die folgenden Schritte umfasst:
i) Bereitstellen einer Gewebeprobe aus dem Zentralnervensystem eines Tieres und
ii) Isolieren des Komplexes durch das In-Kontakt-Bringen der Probe mit einem Peptid, das an PSD-95 bindet, wobei das Peptid, das an PSD-95 bindet, SIESDV oder KRLLDAQRGSFPPWVQQTRV ist.

6. Komplex gemäß einem beliebigen der Ansprüche 1 bis 4 oder Verfahren gemäß Anspruch 5, wobei die Gewebeprobe aus dem Gehirn stammt.

7. Komplex oder Verfahren gemäß Anspruch 6, wobei die Gewebeprobe aus dem Vorderhirn stammt.

8. Komplex gemäß einem beliebigen der Ansprüche 1 bis 4, 6 oder 7 oder Verfahren gemäß einem beliebigen der Ansprüche 5 bis 7, wobei die Gewebeprobe aus dem Zentralnervensystem eines adulten, juvenilen oder neugeborenen Säugetiers stammt.

9. Verfahren gemäß einem beliebigen der Ansprüche 5 bis 8, das ferner (vor dem Schritt 1) den Schritt des Auswählens des Alters des Säugetiers, dem das Gewebe entnommen wird, umfasst.

10. Komplex oder Verfahren gemäß Anspruch 6, wobei die Gewebeprobe humanen Ursprungs ist.

11. Wirtszelle, die ein genetisches Konstrukt oder mehrere genetische Konstrukte enthält, das bzw. die für eine Komponente eines Multiproteinkomplexes gemäß einem beliebigen der Ansprüche 1 bis 4 codiert bzw. codieren, wobei die Komponente aus der Gruppe von Komponenten ausgewählt ist, die in der Tabelle 1 aufgeführt sind.

12. Wirtszelle gemäß Anspruch 11, die ein genetisches Konstrukt oder mehrere genetische Konstrukte enthält, das bzw. die für vier oder mehr Komponenten codiert bzw. codieren, die aus der Gruppe von Komponenten ausgewählt sind, die in den Tabellen 1 und 2 aufgeführt sind.

13. Wirtszelle gemäß Anspruch 11 oder 12, die genetische Konstrukte enthält, die für sechs oder mehr Komponenten codieren, die in den Tabellen 1 und 2 aufgeführt sind.

14. Wirtszelle gemäß einem beliebigen der Ansprüche 11 oder 13, wobei die Zelle eine Säugerzelle ist.

15. Wirtszelle gemäß einem beliebigen der Ansprüche 11 oder 14, wobei die Zelle ein Neuron oder eine von einem Neuron abstammende Zelle ist.

16. Subzelluläre Fraktion, isoliert aus einer Wirtszelle gemäß einem beliebigen der Ansprüche 11 oder 15, die in sich einen Multiproteinkomplex gemäß einem beliebigen der Ansprüche 1 bis 4 oder einen Subkomplex von diesem enthält.

17. Subzelluläre Fraktion gemäß Anspruch 16, wobei die subzelluläre Fraktion eine Plasmamembran ist oder eine solche umfasst.

18. Verfahren zur Identifizierung einer Kandidatenverbindung für die Behandlung von Störungen und Erkrankungen, die mit einer Fehlfunktion von NMDA-Rezeptoren im Zentralnervensystem assoziiert sind, wobei das Verfahren umfasst:
i) Auswählen einer Verbindung, die getestet werden soll,
ii) In-Kontakt-Bringen der Testverbindung mit einem Multiproteinkomplex gemäß einem beliebigen der Ansprüche 1 bis 4 und
iii) Bestimmen, ob die Verbindung, die getestet werden soll, mit dem Komplex interagiert,
wobei Testverbindungen, die mit dem Komplex interagieren, als Kandidatenverbindungen für die Linderung der Symptome, die Verhinderung oder die Verlangsamung des Einsetzens und/oder das Korrigieren des Defizits oder der Defizite, das bzw. die mit Störungen und Erkrankungen assoziiert ist bzw. sind, die mit einer Fehlfunktion von NMDA-Rezeptoren im Zentralnervensystem assoziiert sind, identifiziert werden.

19. Verfahren zur Identifizierung einer Kandidatenverbindung für die Behandlung von Störungen und Erkrankungen, die mit einer Fehlfunktion von NMDA-Rezeptoren im Zentralnervensystem assoziiert sind, wobei das Verfahren umfasst:
i) Auswählen einer Verbindung, die getestet werden soll,
ii) In-Kontakt-Bringen der Testverbindung mit einer Komponente eines Multiproteinkomplexes gemäß einem beliebigen der Ansprüche 1 bis 4, wobei die Komponente aus den in der Tabelle 1 aufgeführten Komponenten ausgewählt ist, und
iii) Bestimmen, ob die Verbindung, die getestet werden soll, eine Eigenschaft der Komponente moduliert,
wobei Testverbindungen, die eine Eigenschaft der Komponente modulieren, als Kandidatenverbindungen für die Linderung der Symptome, die Verhinderung oder die Verlangsamung des Einsetzens und/oder das Korrigieren des Defizits oder der Defizite, das bzw. die mit Störungen und Erkrankungen assoziiert ist bzw. sind, die mit einer Fehlfunktion von NMDA-Rezeptoren im Zentralnervensystem assoziiert sind, identifiziert werden.

20. Verfahren gemäß Anspruch 19, wobei der Schritt iii) das Bestimmen umfasst, ob die Verbindung die Fähigkeit der Komponente moduliert, mit einer anderen oder mehreren anderen der Komponenten, die in den Tabellen 1 und 2 aufgeführt sind, oder einem Subkomplex von diesen zu interagieren.

21. Verfahren gemäß einem beliebigen der Ansprüche 18 bis 20, das ferner den Schritt des Testens der Testverbindung in einem Screen oder mehreren Screens auf die Wirksamkeit bezüglich der Behandlung von Lernstörungen, psychiatrischen Erkrankungen und/oder neurologischen Erkrankungen umfasst.

22. Verfahren zur Identifizierung einer Kandidatenverbindung für die Behandlung von Störungen und Erkrankungen, die mit einer Fehlfunktion von NMDA-Rezeptoren im Zentralnervensystem assoziiert sind, wobei das Verfahren umfasst:
i) Auswählen einer Verbindung, von der bekannt ist, dass sie eine Eigenschaft einer Komponente eines Multiproteinkomplexes gemäß einem beliebigen der Ansprüche 1 bis 4 moduliert, wobei die Komponente in der Tabelle 1 aufgeführt ist, und
ii) Testen der besagten Verbindung in einem Screen auf die Wirksamkeit bezüglich der Behandlung von Störungen und Erkrankungen, die mit einer Fehlfunktion von NMDA-Rezeptoren im Zentralnervensystem assoziiert sind.

23. Verwendung einer Wirtszelle gemäß einem beliebigen der Ansprüche 11 bis 15 oder einer subzellulären Fraktion gemäß Anspruch 16 oder 17 in einem Verfahren zur Identifizierung einer Kandidatenverbindung für die Behandlung von Störungen und Erkrankungen, die mit einer Fehlfunktion von NMDA-Rezeptoren im Zentralnervensystem assoziiert sind.

24. Verwendung einer Verbindung, die eine Eigenschaft einer Komponente oder mehrerer Komponenten eines Multiproteinkomplexes gemäß einem der Ansprüche 1 bis 4 moduliert, wobei die Komponenten in der Tabelle 1 aufgeführt sind, bei der Herstellung eines Medikaments zur Behandlung von Störungen und Erkrankungen, die mit Fehlfunktion von NMDA-Rezeptoren im Zentralnervensystem assoziiert sind.

25. Verwendung gemäß Anspruch 24, wobei das Medikament für die Behandlung von Lernstörungen, psychiatrischen Erkrankungen (wie der Schizophrenie) und/oder neurologischen Erkrankungen (wie des Schlaganfalls und/oder der Epilepsie) gedacht ist.

26. Verfahren zum Diagnostizieren, Prognostizieren und/oder Bestimmen der Anfälligkeit eines Subjekts für Störungen und Erkrankungen, die mit einer Fehlfunktion von NMDA-Rezeptoren im Zentralnervensystem assoziiert sind, oder Verfahren zur Unterstützung einer derartigen Prognose, Diagnose und/oder Bestimmung, das die folgenden Schritte umfasst:
i) Bereitstellen einer nucleinsäurehaltigen Probe des Subjekts, für das eine Diagnose erstellt werden soll, und
ii) In-Kontakt-Bringen der besagten Nucleinsäure des Subjekts mit einer Nucleinsäure, die selektiv mit einer Nucleinsäure, die für eine Komponente eines Multiproteinkomplexes gemäß einem beliebigen der Ansprüche 1 bis 4 codiert,
wobei die Komponenten in der Tabelle 1 aufgeführt sind, oder einem mutierten Allel von ihr oder deren Komplement hybridisiert.

27. Verfahren gemäß Anspruch 26, wobei die Störung oder Erkrankung aus Lernstörungen, psychiatrischen Erkrankungen und/oder neurologischen Erkrankungen ausgewählt ist.

28. Verfahren gemäß Anspruch 26 oder 27, wobei die für eine in der Tabelle 1 aufgeführte Komponente codierende Nucleinsäure ein Gen ist.

29. Verfahren gemäß einem beliebigen der Ansprüche 26 bis 28, das ferner umfasst den Schritt des In-Kontakt-Bringens von Nucleinsäure des Subjekts mit wenigstens einer weiteren Nucleinsäure, die selektiv mit einer Nucleinsäure, die für eine Komponente codiert, die aus der Gruppe der in den Tabellen 1 und 2 aufgeführten Komponenten ausgewählt ist, oder einem mutierten Allel von ihr oder deren Komplement hybridisiert, wobei jede Nucleinsäure, die mit der Nucleinsäure des Subjekts hybridisiert, für eine andere Komponente codiert.

30. Verfahren gemäß einem beliebigen der Ansprüche 26 bis 29, wobei die eine Nucleinsäure oder die mehreren Nucleinsäuren, die selektiv mit der Nucleinsäure, die für eine Komponente codiert, die aus der Gruppe der in der Tabelle 1 aufgeführten Komponenten ausgewählt ist, oder einem mutierten Allel von ihr oder deren Komplement hybridisiert bzw. hybridisieren, ferner eine nachweisbare Gruppe umfasst bzw. umfassen.

31. Verfahren gemäß einem beliebigen der Ansprüche 26 bis 30, wobei die eine Nucleinsäure oder die mehreren Nucleinsäuren, die selektiv mit der Nucleinsäure, die für eine Komponente codiert, die aus der Gruppe der in der Tabelle 1 aufgeführten Komponenten ausgewählt ist, oder einem mutierten Allel von ihr oder deren Komplement hybridisiert bzw. hybridisieren, einzelsträngig ist bzw. sind.

32. Verfahren gemäß einem beliebigen der Ansprüche 26 bis 31, wobei die eine Nucleinsäure oder die mehreren Nucleinsäuren, die selektiv mit der Nucleinsäure, die für eine Komponente codiert, die aus der Gruppe der in der Tabelle 1 aufgeführten Komponenten ausgewählt ist, oder einem mutierten Allel von ihr oder deren Komplement hybridisiert bzw. hybridisieren, kürzer als 1000 Basenpaare (wenn die besagte Nucleinsäure doppelsträngig ist) oder Basen (wenn die besagte Nucleinsäure einzelsträngig ist) ist bzw. sind, zum Beispiel kürzer als 100 Basenpaare oder Basen.

33. Verfahren gemäß einem beliebigen der Ansprüche 26 bis 32, das ferner umfasst den Schritt des Identifizierens des Vorliegens einer Sequenzvariante einer Nucleinsäure, die für eine in der Tabelle 1 aufgeführte Komponente codiert, in der Nucleinsäure des Patienten und des Vergleichens der besagten Sequenzvariante mit Sequenzen in äquivalenten Nucleinsäuren von Individuen aus einer oder mehreren Testpopulation(en).

34. Verfahren gemäß Anspruch 33, wobei die eine oder die mehreren Testpopulation(en) eine Population von Individuen umfasst bzw. umfassen, die nicht unter Störungen und Erkrankungen leiden und früher nicht unter Störungen und Erkrankungen gelitten haben, die mit einer Fehlfunktion von NMDA-Rezeptoren im Zentralnervensystem assoziiert sind.

35. Verfahren zum Diagnostizieren, Prognostizieren und/oder Bestimmen der Anfälligkeit eines Subjekts für Störungen und Erkrankungen, die mit einer Fehlfunktion von NMDA-Rezeptoren im Zentralnervensystem assoziiert sind, das die folgenden Schritte umfasst:
i) Bereitstellen einer Probe, die Antikörper des Subjekts, das getestet werden soll, enthält, und
ii) Bestimmen, ob die besagte Probe Antikörper enthält, die mit einem Multiproteinkomplex gemäß einem beliebigen der Ansprüche 1 bis 4 reagieren.

36. Aus Teilen bestehendes Kit, das nützlich für das Diagnostizieren, Prognostizieren oder Bestimmen der Anfälligkeit eines Patienten für Störungen und Erkrankungen ist, die mit einer Fehlfunktion von NMDA-Rezeptoren im Zentralnervensystem assoziiert sind, wobei das Kit eine Nucleinsäure umfasst, die selektiv mit einer Nucleinsäure hybridisiert, die für eine Komponente eines Multiproteinkomplexes gemäß einem beliebigen der Ansprüche 1 bis 4 codiert, wobei die Komponenten in der Tabelle 1 aufgeführt sind, zusammen mit Enzymen und Reagenzien für die Förderung einer Nucleinsäure-Nucleinsäure-Hybridisierung und den Nachweis der **dadurch** gebildeten Hybride.

## Revendications

1. Complexe multiprotéique comprenant les sous-unités polypeptidiques NR1, NR2A, NR2B, PSD-95 et au moins trois composants choisis dans le groupe de composants énumérés dans le Tableau 1, pouvant être obtenus par des procédés chromatographiques d'affinité peptidique comprenant les étapes consistant à :
(i) collecter un échantillon de tissu provenant du système nerveux central d'un animal ; et
(ii) isoler le complexe en mettant en contact l'échantillon avec un peptide qui se lie à PSD-95,
dans lequel le peptide qui se lie à PSD-95 est SIESDV ou KRLLDAQRGSFPPWVQQTRV.

2. Complexe selon la revendication 1, dans lequel le complexe comprend six composants ou plus, énumérés dans le Tableau 1.

3. Complexe selon la revendication 1 ou 2, dans lequel le complexe comprend des protéines constitutives qui peuvent être divisées en cinq catégories ; les protéines réceptrices de neurotransmetteurs, les protéines d'adhésion cellulaire, les protéines adaptatrices, les peptides de signalisation, et les protéines cytosquelettiques.

4. Complexe selon l'une quelconque des revendications 1 à 3, comprenant tous les composants énumérés dans les Tableaux 1 et 2.

5. Procédé pour la production d'un complexe multiprotéique selon l'une quelconque des revendications 1 à 4 comprenant les étapes consistant à :
(i) fournir un échantillon de tissu provenant du système nerveux central d'un animal ; et
(ii) isoler le complexe en mettant en contact l'échantillon avec un peptide qui se lie à PSD-95, dans lequel le peptide qui se lie à PSD-95 est SIESDV ou KRLLDAQRGSFPPWVQQTRV.

6. Complexe selon l'une quelconque des revendications 1 à 4 ou procédé selon la revendication 5, dans lesquels l'échantillon de tissu provient du cerveau.

7. Complexe ou procédé selon la revendication 6, dans lesquels l'échantillon de tissu provient du cerveau antérieur.

8. Complexe selon l'une quelconque des revendications 1 à 4, 6 ou 7 ou procédé selon l'une quelconque des revendications 5 à 7, dans lesquels l'échantillon de tissu provient du système nerveux central d'un mammifère adulte, juvénile ou nouveau-né.

9. Procédé selon l'une quelconque des revendications 5 à 8, comprenant en outre l'étape (antérieure à l'étape 1) consistant à choisir l'âge du mammifère sur lequel le tissu est prélevé.

10. Complexe ou procédé selon la revendication 6, dans lesquels l'échantillon de tissu est d'origine humaine.

11. Cellule hôte contenant une ou plusieurs constructions génétiques qui codent un composant d'un complexe multiprotéique selon l'une quelconque des revendications 1 à 4, dans laquelle le composant est choisi dans le groupe de composants énumérés dans le Tableau 1.

12. Cellule hôte selon la revendication 11, contenant une ou plusieurs constructions génétiques qui codent quatre composants ou plus choisis dans le groupe de composants énumérés dans les Tableaux 1 et 2.

13. Cellule hôte selon la revendication 11 ou 12, contenant des constructions génétiques codant six composants ou plus énumérés dans les Tableaux 1 ou 2.

14. Cellule hôte selon l'une quelconque des revendications 11 ou 13 dans laquelle la cellule est une cellule de mammifère.

15. Cellule hôte selon l'une quelconque des revendications 11 ou 14 dans laquelle la cellule est un neurone ou une cellule dérivée d'un neurone.

16. Fraction sous-cellulaire isolée à partir d'une cellule hôte selon l'une quelconque des revendications 11 ou 15 qui contient un complexe multiprotéique selon l'une quelconque des revendications 1 à 4 ou un sous-complexe de ce dernier.

17. Fraction sous-cellulaire selon la revendication 16, dans laquelle la fraction sous-cellulaire est ou comprend une membrane plasmique.

18. Procédé pour identifier un composé candidat pour traiter des troubles et des états associés au dysfonctionnement des récepteurs NMDA dans le système nerveux central comprenant les étapes consistant à :
(i) choisir un composé à tester ;
(ii) mettre en contact le composé test avec un complexe multiprotéique selon l'une quelconque des revendications 1 à 4 ; et
(iii) déterminer si le composé à tester interagit avec le complexe ;
dans lequel les composés tests qui interagissent avec le complexe sont identifiés en tant que composés candidats pour soulager les symptômes, empêcher ou ralentir l'apparition de troubles et d'états associés à un dysfonctionnement des récepteurs NMDA dans le système nerveux central, et/ou corriger le (ou les) déficit(s) associé(s) à ces troubles ou états.

19. Procédé pour identifier un composé candidat pour traiter des troubles et des états associés au dysfonctionnement des récepteurs NMDA dans le système nerveux central comprenant les étapes consistant à :
(i) choisir un composé à tester ;
(ii) mettre en contact le composé test avec un composant d'un complexe multiprotéique selon l'une quelconque des revendications 1 à 4, dans lequel le composant est choisi parmi les composants énumérés dans le Tableau 1 ; et
(iii) déterminer si le composé à tester module une propriété du composant ;
dans lequel les composés tests qui modulent une propriété du composant sont identifiés en tant que composés candidats pour soulager les symptômes, empêcher ou ralentir l'apparition de troubles et d'états associés à un dysfonctionnement des récepteurs NMDA dans le système nerveux central, et/ou corriger le (ou les) déficit(s) associé(s) à ces troubles ou états.

20. Procédé selon la revendication 19, dans lequel l'étape (iii) comprend l'étape consistant à déterminer si le composé module la capacité du composant à interagir avec un ou plusieurs autres composants énumérés dans les Tableaux 1 ou 2 ou un sous-complexe de ces derniers.

21. Procédé selon l'une quelconque des revendications 18 à 20, comprenant en outre l'étape consistant à tester le composé test dans un ou plusieurs cribles pour déterminer son efficacité dans le traitement de la déficience en matière d'apprentissage, des troubles psychiatriques et/ou des troubles neurologiques.

22. Procédé pour identifier un composé candidat pour traiter des troubles et des états associés au dysfonctionnement des récepteurs NMDA dans le système nerveux central comprenant les étapes consistant à :
(i) choisir un composé connu pour moduler une propriété d'un composant d'un complexe multiprotéique selon l'une quelconque des revendications 1 à 4, dans lequel le composant est énuméré dans le Tableau 1 ; et
(ii) tester ledit composé dans un crible afin de déterminer son efficacité dans le traitement des troubles et des états associés au dysfonctionnement des récepteurs NMDA dans le système nerveux central.

23. Utilisation d'une cellule hôte selon l'une quelconque des revendications 11 à 15 ou d'une fraction sous-cellulaire selon la revendication 16 ou 17 dans un procédé destiné à identifier un composé candidat pour traiter des troubles et des états associés au dysfonctionnement des récepteurs NMDA dans le système nerveux central.

24. Utilisation d'un composé qui module une propriété d'un ou de plusieurs composants d'un complexe multiprotéique selon l'une des revendications 1 à 4, dans laquelle les composants sont énumérés dans le Tableau 1 pour la préparation d'un médicament destiné au traitement de troubles et d'états associés au dysfonctionnement des récepteurs NMDA dans le système nerveux central.

25. Utilisation selon la revendication 24 dans laquelle le médicament est destiné à traiter la déficience en matière d'apprentissage, les troubles psychiatriques (comme la schizophrénie) et/ou les troubles neurologiques (comme un AVC et/ou l'épilepsie).

26. Procédé pour diagnostiquer, pronostiquer et/ou déterminer la susceptibilité d'un sujet à présenter des troubles et des états associés au dysfonctionnement des récepteurs NMDA dans le système nerveux central, ou procédé pour aider l'exécution d'un tel pronostic, diagnostic et/ou détermination, comprenant les étapes consistant à :
(i) fournir un échantillon contenant un acide nucléique provenant du sujet à diagnostiquer ; et
(ii) mettre en contact ledit acide nucléique provenant du sujet avec un acide nucléique qui s'hybride sélectivement à un acide nucléique codant un composant d'un complexe multiprotéique selon l'une quelconque des revendications 1 à 4, dans lequel les composants sont énumérés dans le Tableau 1, ou un allèle mutant de ces derniers, ou leur complément.

27. Procédé selon la revendication 26, dans lequel le trouble ou état est choisi parmi la déficience en matière d'apprentissage, les troubles psychiatriques et/ou les troubles neurologiques.

28. Procédé selon la revendication 26 ou 27, dans lequel l'acide nucléique codant un composant énuméré dans le Tableau 1 est un gène.

29. Procédé selon l'une quelconque des revendications 26 à 28 comprenant en outre l'étape consistant à mettre en contact l'acide nucléique provenant du sujet avec au moins un acide nucléique supplémentaire qui s'hybride sélectivement à un acide nucléique codant un composant choisi dans le groupe de composants énumérés dans les Tableaux 1 et 2, ou un allèle mutant de ces derniers, ou leur complément, dans lequel chaque acide nucléique qui s'hybride à l'acide nucléique provenant du sujet code un composant différent.

30. Procédé selon l'une quelconque des revendications 26 à 29 dans lequel le ou les acides nucléiques qui s'hybrident sélectivement à l'acide nucléique codant un composant choisi parmi le groupe de composants énumérés dans le tableau 1, ou un allèle mutant de ces derniers, ou leur complément, comprend en outre un groupe caractéristique détectable.

31. Procédé selon l'une quelconque des revendications 26 à 30 dans lequel le ou les acides nucléiques qui s'hybrident sélectivement à l'acide nucléique codant un composant choisi dans le groupe de composants énumérés dans le Tableau 1, ou un allèle mutant de ces derniers, ou leur complément est monocaténaire.

32. Procédé selon l'une quelconque des revendications 26 à 31, dans lequel le ou les acides nucléiques qui s'hybrident sélectivement à l'acide nucléique codant un composant choisi dans le groupe de composants énumérés dans le Tableau 1, ou un allèle mutant de ces derniers, ou leur complément représentent moins de 1 000 paires de bases (si ledit acide nucléique est bicaténaire) ou bases (si ledit acide nucléique est monocaténaire), par exemple moins de 100 paires de bases ou bases.

33. Procédé selon l'une quelconque des revendications 26 à 32 comprenant en outre l'étape consistant à identifier dans l'acide nucléique provenant du patient la présence d'une variante de séquence d'un acide nucléique codant un composant énuméré dans le Tableau 1 et à comparer ladite variante de séquence à des séquences dans des acides nucléiques équivalents chez des individus provenant d'une ou de plusieurs populations tests.

34. Procédé selon la revendication 33, dans lequel la ou les populations tests comprennent une population d'individus qui ne souffrent pas et n'ont pas souffert auparavant de troubles et états associés au dysfonctionnement des récepteurs NMDA dans le système nerveux central.

35. Procédé destiné à diagnostiquer, pronostiquer et/ou déterminer la susceptibilité d'un sujet à présenter des troubles ou états associés au dysfonctionnement des récepteurs NMDA dans le système nerveux central, comprenant les étapes consistant à :
(i) fournir un échantillon contenant des anticorps provenant du sujet à tester ; et
(ii) déterminer si ledit échantillon contient des anticorps réagissant avec un complexe multiprotéique selon l'une quelconque des revendications 1 à 4.

36. Kit d'éléments utile pour diagnostiquer, pronostiquer ou déterminer la susceptibilité d'un patient à présenter des troubles et des états associés au dysfonctionnement des récepteurs NMDA dans le système nerveux central comprenant un acide nucléique qui s'hybride sélectivement à un acide nucléique codant un composant d'un complexe multiprotéique selon l'une quelconque des revendications 1 à 4, dans lequel les composants sont énumérés dans le Tableau 1, avec des enzymes et réactifs pour promouvoir l'hybridation acide nucléique - acide nucléique et pour détecter les hybrides ainsi formés.
